(19) Europäisches Patentamt
European Patent Office
Office européen des brevets

(11) Publication number: **0 486 011 A2**

# (12) EUROPEAN PATENT APPLICATION

(21) Application number: **91119421.5**

(51) Int. Cl.$^5$: **A61K 37/02**

(22) Date of filing: **14.11.91**

(30) Priority: **16.11.90 US 614125**
**14.12.90 GB 9027152**
**24.01.91 GB 9101552**
**02.04.91 GB 9106822**

(43) Date of publication of application:
**20.05.92 Bulletin 92/21**

(84) Designated Contracting States:
**AT BE CH DE DK ES FR GB GR IT LI LU NL SE**

(71) Applicant: **FUJISAWA PHARMACEUTICAL CO., LTD.**
**4-7, Doshomachi 3-chome Chuo-ku**
**Osaka-shi Osaka 541(JP)**

(72) Inventor: **Furuta, Takahisa**
**2-1-5-815, Omoriminami, Ota-ku**
**Tokyo 143(JP)**
Inventor: **Iwamoto, Toshiro**
**16-4-308, Azuma 4-chome**
**Tsukuba-shi, Ibaraki 305(JP)**
Inventor: **Fujie, Akihiko**
**41-1, Sakuragaokacho**
**Tsuchiura-shi, Ibaraki 300(JP)**
Inventor: **Nitta, Kumiko**
**1160-4, Nagakuni**
**Tsuchiura-shi, Ibaraki 300(JP)**
Inventor: **Tsurumi, Yasuhisa**
**21-1-510-601, Takezono 3-chome**
**Tsukuba-shi, Ibaraki 305(JP)**
Inventor: **Shigematsu, Nobuharu**
**5-4-601, Umezono 2-chome**
**Tsukuba-shi, Ibaraki 305(JP)**
Inventor: **Kasahara, Chiyoshi**
**2-2-13, Midorigaoka**
**Ikeda-shi, Osaka 563(JP)**
Inventor: **Hino, Motohiro**
**13-3-1003, Touzaki-cho**
**Tsuchiura-shi, Ibaraki 300(JP)**
Inventor: **Okuhara, Masakuni**
**14-10, Umezono 2-chome**
**Tsukuba-shi, Ibaraki 305(JP)**

(74) Representative: **Türk, Gille, Hrabal**
**Brucknerstrasse 20**
**W-4000 Düsseldorf 13(DE)**

(54) **Pharmaceutical composition against Pneumocystis carinii.**

EP 0 486 011 A2

(57) Use of a polypeptide compound of the formula :

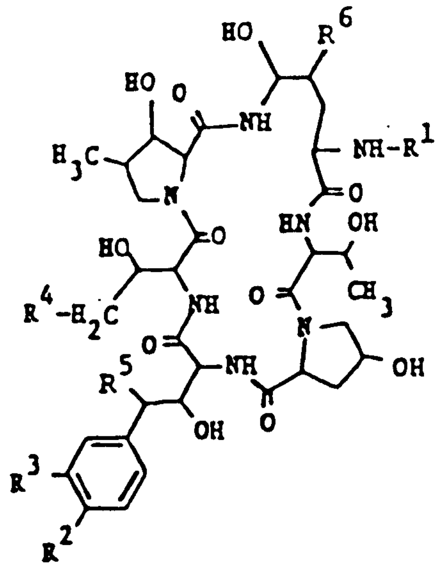

wherein
$R^1$ is hydrogen or acyl group,
$R^2$ is hydroxy or acyloxy,
$R^3$ is hydrogen, hydroxy or hydroxysulfonyloxy,
$R^4$ is hydrogen or carbamoyl, and
$R^5$ and $R^6$ are each hydrogen or hydroxy,
with proviso that
(i) $R^2$ is acyloxy, when $R^3$ is hydrogen, and
(ii) $R^5$ is hydrogen, when $R^6$ is hydrogen,
or a pharmaceutically acceptable salt thereof for the preparation of a medicament for the prevention and/or the treatment of Pneumocystis carinii infection.

The present invention relates to a new use of the polypeptide compound or a pharmaceutically acceptable salt thereof.

More particularly, it relates to the utility of the polypeptide compound for the prevention and/or the treatment of Pneumocystis carinii infection (e.g. Pneumocystis carinii pneumonia, etc) in a human being or an animal.

Accordingly, one object of the present invention is to provide a pharmaceutical composition for the prevention and/or the treatment of Pneumocystis carinii infection (e.g. Pneumocystis carinii pneumonia, etc) in a human being or an animal comprising, as an active ingredient, the polypeptide compound or a pharmaceutically acceptable salt thereof.

Another object of the present invention is to provide a method for the prevention and/or the treatment of Pneumocystis carinii infection (e.g. Pneumocystis carinii pneumonia, etc) in a human being or an animal, which comprises administering the polypeptide compound to a human being or an animal.

A further object of the present invention is to provide a use of the polypeptide compound for the manufacture of a medicament for the prevention and/or the treatment of Pneumocystis carinii infection (e.g. Pneumocystis carinii pneumonia, etc) in a human being or an animal.

The polypeptide compound used in the present invention is novel and can be represented by the following general formula [I] :

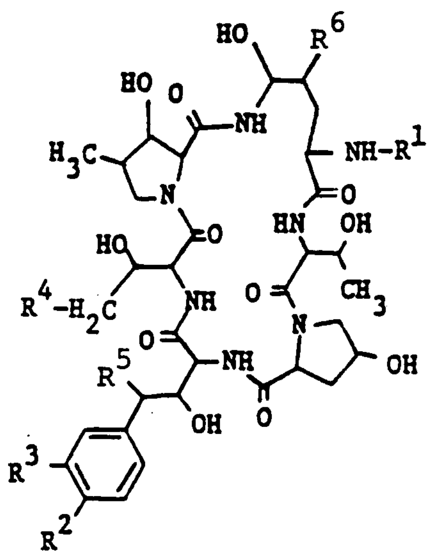

[I]

wherein

$R^1$ is hydrogen or acyl group,

$R^2$ is hydroxy or acyloxy,

$R^3$ is hydrogen, hydroxy or hydroxysulfonyloxy,

$R^4$ is hydrogen or carbamoyl, and

$R^5$ and $R^6$ are each hydrogen or hydroxy,

with proviso that

(i) $R^2$ is acyloxy, when $R^3$ is hydrogen, and

(ii) $R^5$ is hydrogen, when $R^6$ is hydrogen.

The inventors of the present invention have found said polypeptide compound [I] and a pharmaceutically acceptable salt thereof are useful for the prevention and/or the treatment of Pneumocystis carinii infection (e.g. Pneumocystis carinii pneumonia, etc) in a human being or an animal and have completed the present invention.

The pharmaceutical composition of the present invention can be used in the form of a pharmaceutical preparation, for example, in solid, semisolid or liquid form, which contains the polypeptide compound [I] or a pharmaceutically acceptable salt thereof, as an active ingredient in admixture with an organic or inorganic carrier or excipient suitable for rectal, pulmonary (nasal or buccal inhalation), nasal, ocular, external (topical), oral or parenteral (including subcutaneous, intravenous and intramuscular) administrations or insufflation.

The active ingredient may be compounded, for example, with the usual non-toxic, pharmaceutically acceptable carriers for tablets, pellets, troches, capsules, suppositories, creams, ointments, aerosols, powders for insufflation, solutions, emulsions, suspensions, and any other form suitable for use. And, if necessary, in addition, auxiliary, stabilizing, thickening and coloring agents and perfumes may be used.

The polypeptide compound [I] or a pharmaceutically acceptable salt thereof is/are included in the pharmaceutical composition in an amount sufficient to produce the desired effect upon the process or condition of Pneumocystis carinii infection.

The pharmaceutical composition of the present invention can be manufactured by the conventional method in this field of the art. If necessary, the technique generally used in this field of the art for improving the bioavailability of a drug can be applied to the pharmaceutical composition of the present invention.

For applying the composition to a human being or an animal, it is preferable to apply it by intravenous, intramuscular, pulmonary, or oral administration, or insufflation.

While the dosage of therapeutically effective amount of the polypeptide compound [I] varies from and also depends upon the age and condition of each individual patient to be treated, in the case of intravenous administration, a daily dose of 0.0l - 100 mg of the polypeptide compound [I] per kg weight of a human being or an animal, in the case of intramuscular administration, a daily dose of 0.l - 100 mg of the polypeptide compound [I] per kg weight of a human being or an animal, in case of oral administration, a daily dose of 0.5 - 100 mg of the polypeptide compound [I] per kg weight of a human being or an animal is generally given for the prevention and/or the treatment of Pneumocystis carinii infection (e.g. Pneumocystis carinii pneumonia, etc) in a human being or an animal.

Especially, the followings are to be noted.

For administration by inhalation, the compounds of the present invention are conveniently delivered in the form of an aerosol spray presentation from pressurized packs or nebulisers. The compounds may also be delivered as powders which may be formulated and the powder compositions may be inhaled with the aid of an insufflation powder inhaler device. The preferred delivery system for inhalation is a metered dose inhalation aerosol, which may be formulated as a suspension or solution of compound in suitable propellants such as fluorocarbons or hydrocarbons.

Because of desirability to directly treat lung and bronchi, aerosol administration is a preferred method of administration. Insufflation is also a desirable method, especially where infection may have spread to ears and other body cavities.

Alternatively, parenteral administration may be employed using drip intravenous administration.

In order to show the usefulness of the polypeptide compound [I] or a pharmaceutically acceptable salt thereof used in the present invention for the prevention and/or the treatment of Pneumocystis carinii infection (e.g. Pneumocystis carinii pneumonia, etc) in a human being or an animal, the pharmacological test data of the representative compounds thereof are shown in the following.

Test 1

Eight Hooded nude rats (male 3, female 5) were intranasally infected with $10^4$ Pneumocystis carinii cysts derived from rat and subcutaneously injected with 20 mg cortisone once a week for 8 weeks. At the start of the treatment with FR131535 substance, three of 8 rats were sacrificed and the lungs were removed, homogenized with a glass homogenizer in phosphate buffered saline, and processed for quantitation as described below. The remaining rats were divided into two groups, and the rats of one group were intraperitoneally injected once daily with 2 mg of FR131535 substance in 0.5 ml of saline and the rats of the other group were intraperitoneally injected once daily only with 0.5 ml of saline as a negative control.

The total number of cysts per rat lung was determined by quantitating the number of cysts of homogenized lung tissue on slides fixed with ether/sulfuric acid and stained with toluidine blue 0.

The test results were as follows.

| Treatment with | | Rat | Number of cysts per lung ($log_{10}$) |
|---|---|---|---|
| before treatment | | 1<br>2<br>3 | 6.56<br>6.53<br>6.30 |
| FR131535 substance | 10 mg[a] (5[b])<br>12 mg[a] (6[b])<br>26 mg[a] (13[b]) | 4<br>5<br>6 | 4.56<br>3.89<br>4.78 |
| saline | | 7<br>8 | 7.15<br>7.94 |

[a] : Total amount of FR131535 substance given to rat
[b] : the day after the start of treatment when the number of cysts in lung was determined

Test 2

1. Test Method

The female BALB/C nu/nu mice, 5 weeks old, were intranasally infected with $10^4$ cysts per head under anesthesia and housed in the sterilized vinyl isolators, fed in a completely sterilized condition.

Experiment I. Prophylactic effect

52 days after the infection (at this time, we judged Pneumocystis carinii pneumonia has not yet occurred judging from the conditions of the mice), 34 of the infected mice were randomly selected and used for the prophylactic experiment.

Five mice were sacrificed for examining a physical condition at the starting point.

A half of lungs was removed and was weighed and preserved at -80 °C for examining the number of cysts in lung. The number of cysts was determined by microscopically counting the number of cysts of homogenized lung tissue on slide fixed with ether/sulfuric acid and stained with toluidine blue 0 (hereinafter this procedure was referred to as "Protocol").

The remaining 29 mice were divided into three groups. 9 mice (Group 1) were subcutaneously injected with saline once a day except on Saturday and Sunday. Each 10 mice of Group 2 and Group 3 were similarly treated with 10 mg/kg of FR 131535 substance (Group 2) and FR 901379 substance (Group 3), respectively.

Half of mice of each group were sacrificed 18 days after the start of the administration (Point A) and examined by "Protocol", and the remainder was similarly examined 56 days after the start of the administration (Point B).

Experiment II. Curative effect

The mice not used in Experiment I began to show typical symptom of Pneumocystis carinii pneumonia, mainly wasting and cyanosis about three months after the infection.

101 days after the infection, the mice were divided into two groups by the degree of the symptom (light symptom : 17 mice, heavy symptom : 14 mice). Each group was divided to four groups.

One group (5 mice) out of four of each group was sacrificed for examining the status of mice at the start of therapy by "Protocol".

Administration of the drugs was carried out in the same manner as Experiment I once a day except on Saturday and Sunday [heavy symptom : 3 mice for saline (Group 4), 3 mice for FR 131535 substance (Group 5) and 3 mice for FR 901379 substance (Group 6); light symptom : 4 mice for saline (Group 7), 4 mice for FR 131535 substance (Group 8) and 4 mice for FR 901379 substance (Group 9)].

Mice were treated for two weeks. The mice were sacrificed at the end of the therapy for the examination of the number of the total cysts in the same manner as "Protocol".

2. Test Results

Experiment I

| at Point A | |
|---|---|
| Test Group | Total Cysts (mean $log_{10}$) |
| Group 1 | 5.35 ± 0.26 |
| Group 2 | 1.76 ± 0.03*** |
| Group 3 | 1.81 ± 0.06*** |

*** : P<0.001

| at Point B | |
|---|---|
| Test Group | Total Cysts (mean $log_{10}$) |
| Group 1 | 6.34 ± 0.14 |
| Group 2 | 2.31 ± 0.26*** |
| Group 3 | 2.04 ± 0.25*** |

*** : P<0.001

Experiment II

| Test Group | Total Cysts (mean $\log_{10}$) |
|---|---|
| heavy symptom | |
| Group 4 | $6.20 \pm 0.08$ |
| Group 5 | $2.06 \pm 0.06^{***}$ |
| Group 6 | $3.10 \pm 0.513^{***}$ |
| light symptom | |
| Group 7 | $6.21 \pm 0.12$ |
| Group 8 | $3.85 \pm 0.21^{***}$ |
| Group 9 | $3.23 \pm 0.67^{***}$ |

*** : $P < 0.001$

From the above test results, it turned out that the polypeptide compound [I] or a pharmaceutically acceptable salt thereof used in the present invention was very useful for the prevention and/or the treatment of Pneumocystis carinii pneumonia.

In the following, the polypeptide compound [I] or a pharmaceutically acceptable salt thereof used in the present invention is explained in detail.

The polypeptide compound or a salt thereof can be prepared by the processes as illustrated in the following schemes.

Process 1

a strain belonging to the Coleophoma which is capable of producing the compound [Ia] or a salt thereof

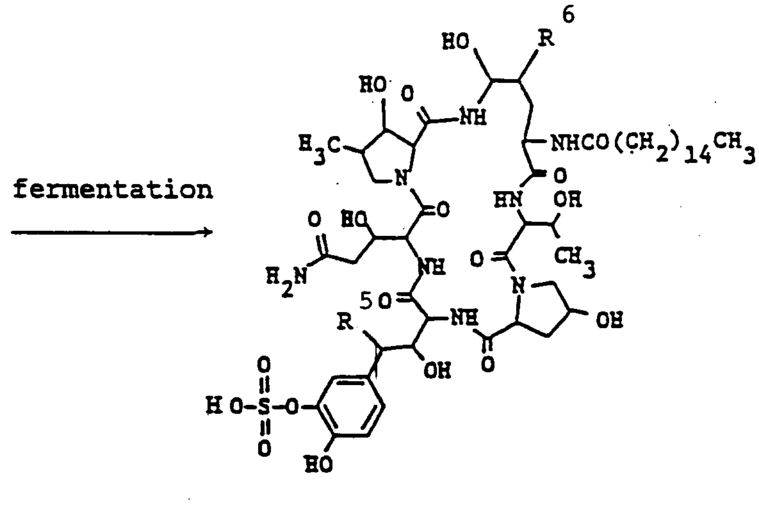


[Ia]
or a salt thereof

Process 2

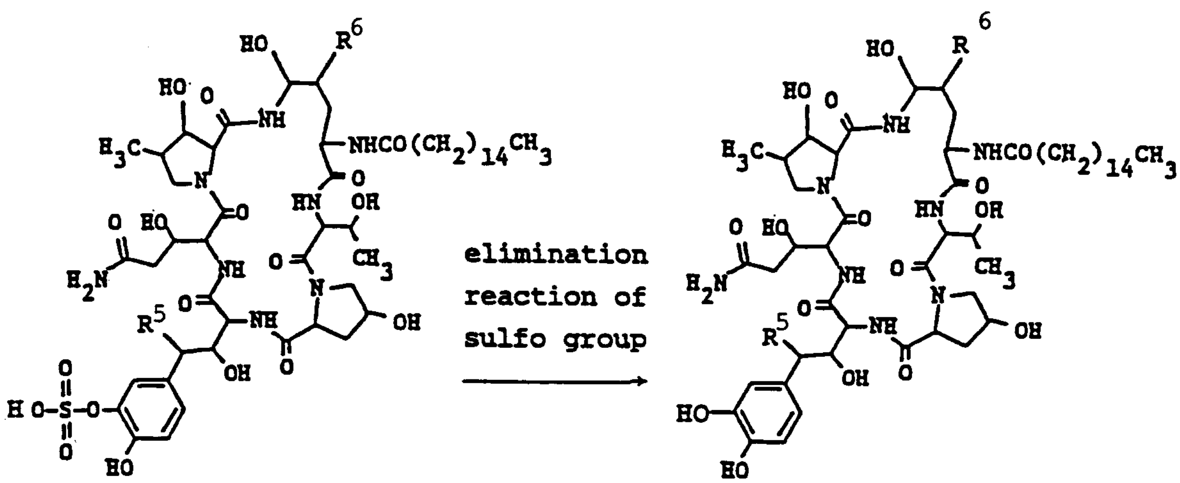


[Ia]
or a salt thereof

[Ib]
or a salt thereof

Process 3

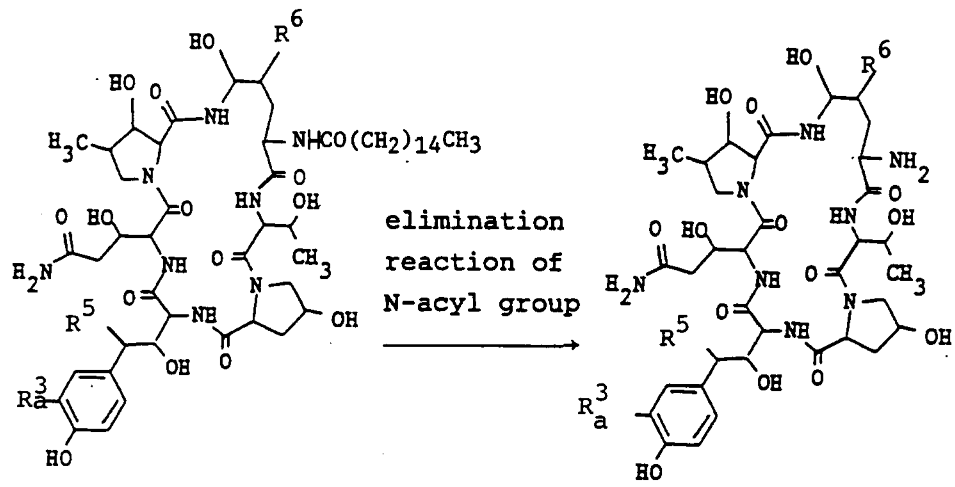


$[I_c]$ or a salt thereof

$[I_d]$ or a salt thereof

Process 4

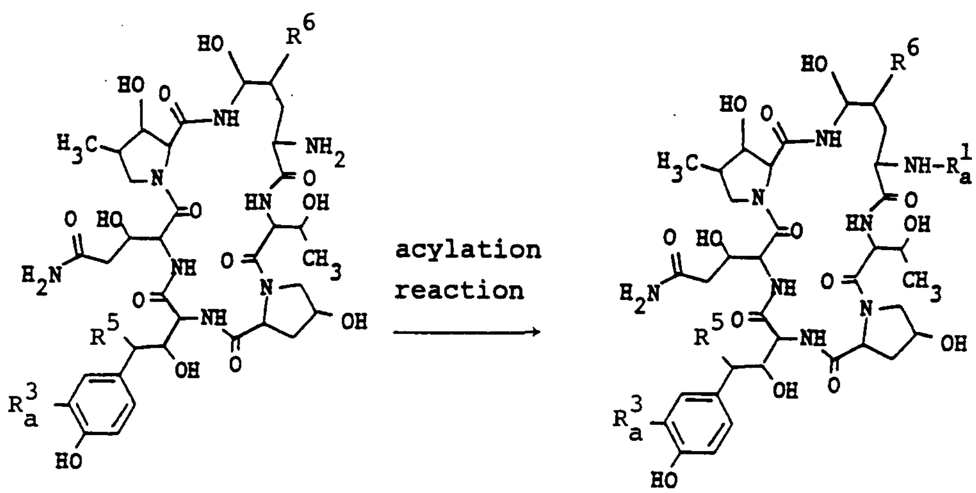


$[I_d]$ or a salt thereof

$[I_e]$ or a salt thereof

Process 5

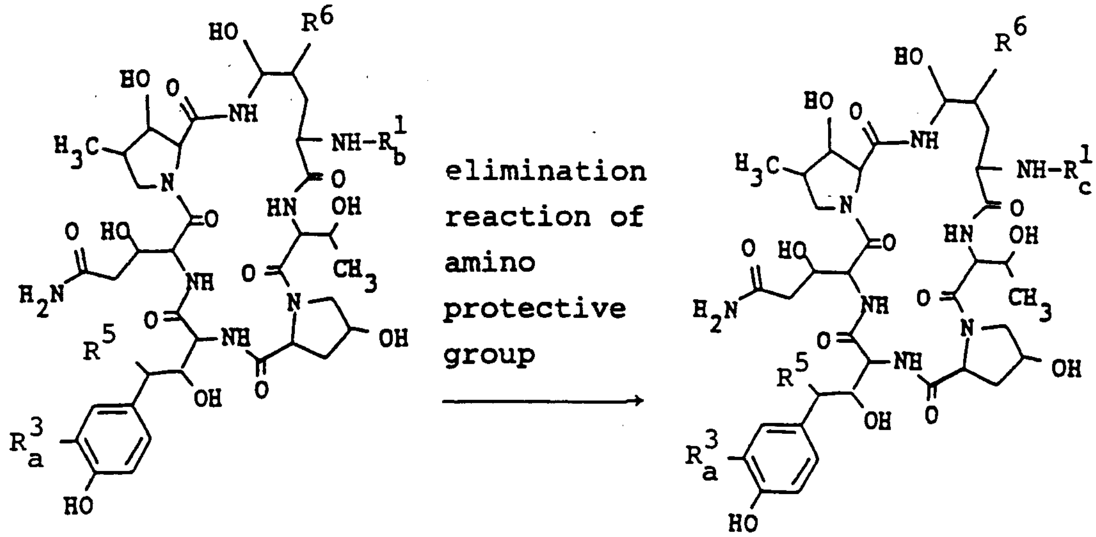


$[I_f]$ or a salt thereof

$[I_g]$ or a salt thereof

Process 6

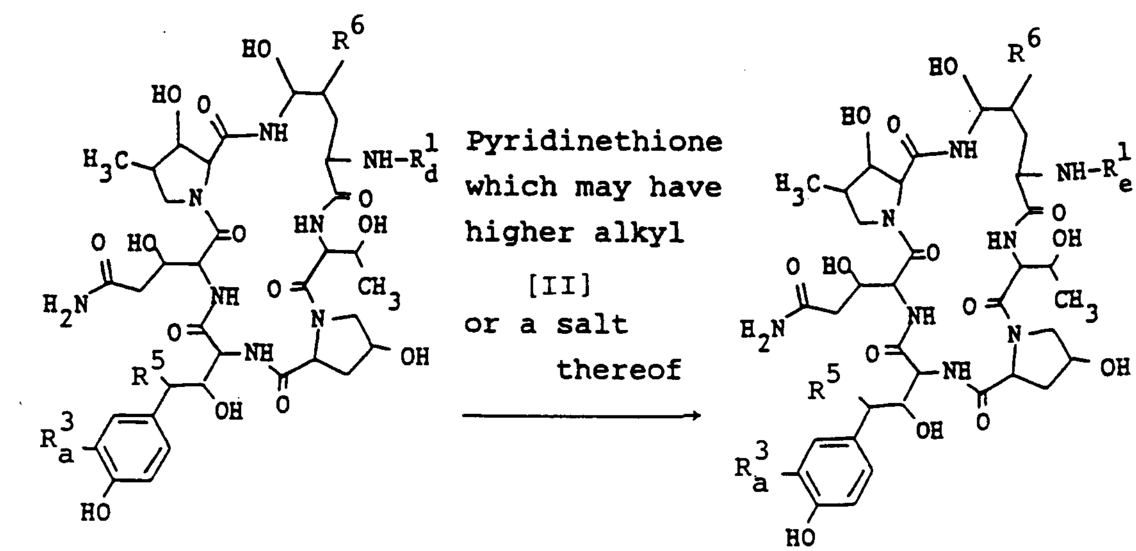


$[I_h]$ or a salt thereof

$[I_i]$ or a salt thereof

## Process 7

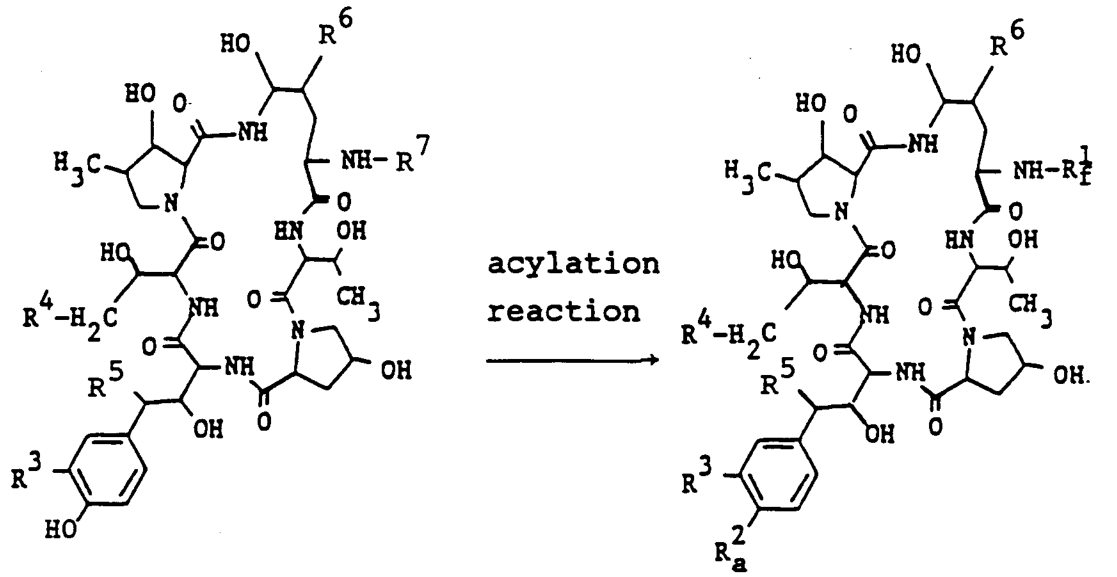


[III]
or a salt thereof

[$I_j$]
or a salt thereof

wherein

| | |
|---|---|
| $R^3$, $R^4$, $R^5$ and $R^6$ are | each as defined above, |
| $R^1_a$ is | acyl group, |
| $R^1_b$ is | ar(lower)alkanoyl which has higher alkoxy and protected amino, |
| $R^1_c$ is | ar(lower)alkanoyl which has higher alkoxy and amino, |
| $R^1_d$ is | halo(lower)alkanoyl, |
| $R^1_e$ is | pyridylthio(lower)alkanoyl which may have higher alkyl, |
| $R^1_f$ is | acyl group, |
| $R^2_a$ is | acyloxy, |
| $R^7$ is | acyl groups , and |
| $R^3_a$ is | hydroxy or hydroxysulfonyloxy. |

Some of the starting compound [III]are novel and can be prepared according to the aforesaid Process 3 to 6.

Suitable pharmaceutically acceptable salt of the object compound [I] is conventional non-toxic mono or di salts and include a metal salt such as an alkali metal halt [e.g. sodium salt, potassium salt, etc.] and an alkaline earth metal salt [e.g. calcium salt, magnesium salt, etc.], an ammonium salt, an organic base salt [e.g. trimethylamine salt, triethylamine salt, pyridine salt, picoline salt, dicyclohexylamine salt, N,N-dibenzylethylenediamine salt, etc.] an organic acid addition salt [e.g. formate, acetate, trifluroacetate, maleate, tartrate, methanesulfonate, benzenesulfonate, toluenesulfonate, etc.], an inorganic acid addition salt [e.g. hydrochloride, hydrobromide, hydroiodide, sulfate, phosphate, etc.], a salt with an amino acid [e.g. arginine salt, aspartic acid salt, glutamic acid salt, etc.], and the like.

In the above and subsequent description of this specification, suitable examples of the various definitions are explained in detail as follows :

The term "lower" is intended to mean l to 6 carbon atom(s), unless otherwise indicated.

The term "higher" is intended to mean 7 to 20 carbon atoms, unless otherwise indicated.

Suitable "acyl group" may be aliphatic acyl, aromatic acyl, heterocyclic acyl, arylaliphatic acyl and heterocyclic-aliphatic acyl derived from carboxylic acid, carbonic acid, carbamic acid, sulfonic acid, and the like.

Suitable example of the "acyl group" thus explained may be :

lower alkanoyl [e.g. formyl, acetyl, propionyl, butyryl, isobutyryl, valeryl, hexanoyl, pivaloyl, etc.] which may have one or more (preferably 1 to 3) suitable substituent(s) such as halogen (e.g. fluoro, chloro, bromo, iodo); aryl (e.g. phenyl, naphthyl, anthryl, etc.) which may have one or more (preferably l to 3) suitable substituent(s) like hydroxy, higher alkoxy as explained below, aforesaid aryl, or the like; lower alkoxy as explained below; amino; protected amino, preferably, acylamino such as lower alkoxycarbonylamino (e.g. methoxycarbonylamino, ethoxycarbonylamino, propoxycarbonylamino, butoxycarbonylamino, t-butoxycarbonylamino, pentyloxycarbonylamino, hexyloxycarbonylamino, etc.); or the like; di(lower)alkylamino (e.g. dimethylamino, N-methylethylamino, diethylamino, N-propylbutylamino, dipentylamino, dihexylamino, etc.); lower alkoxyimino (e.g. methoxyimino, ethoxyimino, propoxyimino, butoxyimino, t-butoxyimino, pentyloxyimino, hexyloxyimino, etc.); ar(lower)alkoxyimino such as phenyl(lower)alkoxyimino (e.g. benzyloxyimino, phenethyloxyimino, benzhydryloxyimino, etc.) which may have one or more (preferably l to 3) suitable substituent(s) like higher alkoxy as explained below, or the like; heterocyclicthio, preferably, pyridylthio, which may have one or more (preferably l to 3) suitable substituent(s) like higher alkyl (e.g. heptyl, octyl, 2-ethylhexyl, nonyl, decyl, 3,7-dimethyloctyl, undecyl, dodecyl, tridecyl, tetradecyl, pentadecyl, 3-methyl-l0-ethyldodecyl, hexadecyl, heptadecyl, octadecyl, nonadecyl, icosyl, etc.), or the like; heterocyclic group (e.g. thienyl, imidazolyl, pyrazolyl, furyl, tetrazolyl, thiazolyl, thiadiazolyl, etc.) which may have one or more (preferably l to 3) suitable substituent(s) like amino, aforesaid protected amino, aforesaid higher alkyl, or the like; or the like;

higher alkanoyl [e.g. heptanoyl, octanoyl, nonanoyl, decanoyl, undecanoyl, lauroyl, tridecanoyl, myristoyl, pentadecanoyl, palmitoyl, l0,l2-dimethyltetradecanoyl, heptadecanoyl, stearoyl, nonadecanoyl, icosanoyl, etc.];

lower alkenoyl [e.g. acryloyl, methacryloyl, crotonoyl, 3-pentenoyl, 5-hexenoyl, etc.] which may have one or more (preferably l to 3) suitable substituent(s) such as aforesaid aryl which may have one or more (preferably l to 3) suitable substituent(s) like higher alkoxy as explained below, or the like, or the like;

higher alkenoyl [e.g. 4-heptenoyl, 3-octenoyl, 3,6-decadienoyl, 3,7,ll-trimethyl-2,6,l0-dodecatrienoyl, 4,l0-heptadecadienoyl, etc.];

lower alkoxycarbonyl [e.g. methoxycarbonyl, ethoxycarbonyl, propoxycarbonyl, butoxycarbonyl, t-butoxycarbonyl, pentyloxycarbonyl, hexyloxycarbonyl, etc.];

higher alkoxycarbonyl [e.g. heptyloxycarbonyl, octyloxycarbonyl, 2-ethylhexyloxycarbonyl, nonyloxycarbonyl, decyloxycarbonyl, 3,7-dimethyloctyloxycarbonyl, undecyloxycarbonyl, dodecyloxycarbonyl, tridecyloxycarbonyl, tetradecyloxycarbonyl, pentadecyloxycarbonyl, 3-methyl-l0-ethyldodecyloxycarbonyl, hexadecyloxycarbonyl, heptadecyloxycarbonyl, octadecyloxycarbonyl, nonadecyloxycarbonyl, icosyloxycarbonyl, etc.];

aryloxycarbonyl [e.g. phenoxycarbonyl, naphthyloxycarbonyl, etc.];

arylglyoxyloyl [e.g. phenylglyoxyloyl, naphthylglyoxyloyl, etc.];

ar(lower)alkoxycarbonyl which may have one or more suitable substituent(s) such as phenyl(lower)-alkoxycarbonyl which may have nitro or lower alkoxy [e.g. benzyloxycarbonyl, phenethyloxycarbonyl, p-nitrobenzyloxycarbonyl, p-methoxybenzyloxycarbonyl, etc.];

lower alkylsulfonyl [e.g. methylsulfonyl, ethylsulfonyl, propylsulfonyl, isopropylsulfonyl, pentylsulfonyl, butylsulfonyl, etc.];

arylsulfonyl [e.g. phenylsulfonyl, naphthylsulfonyl, etc.] which may have one or more (preferably l to 3) suitable substituent(s) such as lower alkyl as explained below, higher alkoxy as explained below, or the like;

ar(lower)alkylsulfonyl such as phenyl(lower)alkylsulfonyl [e.g. benzylsulfonyl, phenethylsulfonyl, benzhydrylsulfonyl, etc.], or the like;

aroyl [e.g. benzoyl, naphthoyl, anthrylcarbonyl, etc.] which may have one or more (preferably l to 5) suitable substituent(s) such as aforesaid halogen; lower alkyl (e.g. methyl, ethyl, propyl, butyl, t-butyl, pentyl, hexyl, etc.); aforesaid higher alkyl; lower alkoxy (e.g. methoxy, ethoxy, propoxy, butoxy, t-butoxy, pentyloxy, hexyloxy, etc.) which may have one or more (preferably l to l0) suitable substituent(s) like aforesaid lower alkoxy, aforesaid halogen, aforesaid aryl, or the like; higher alkoxy (e.g. heptyloxy, octyloxy, 2-ethylhexyloxy, nonyloxy, decyloxy, 3,7-dimethyloctyloxy, undecyloxy, dodecyloxy, tridecyloxy, tetradecyloxy, pentadecyloxy, 3-methyl-10-ethyldodecyloxy, hexadecyloxy, heptadecyloxy, octadecyloxy, nonadecyloxy, icosyloxy, etc.) which may have one or more (preferably l to l7) suitable substituent(s) like aforesaid halogen; higher alkenyloxy (e.g. 3-heptenyloxy, 7-octenyloxy, 2,6-octadienyloxy, 5-nonenyloxy, l-decenyloxy, 3,7-dimethyl-6-octenyloxy, 3,7-dimethyl-2,6-octadienyloxy, 8-undecenyloxy, 3,6,8-dodecatrienyloxy, 5-tridecenyloxy, 7-tetradecenyloxy, l,8-pentadecadienyloxy, l5-hexadecenyloxy, ll-heptadecenyloxy, 7-octadecenyloxy, l0-nonadecenyloxy, l8-icosenyloxy, etc.); carboxy; aforesaid aryl which may have one or more (preferably l to 3) suitable substituent(s) like aforesaid higher alkoxy; aryloxy (e.g. phenoxy, naphthyloxy, anthryloxy, etc.) which may have one or more (preferably l to 3) suitable substituent-(s) like aforesaid lower alkoxy, or aforesaid higher alkoxy; or the like; or the like.

In said "acyl group", the preferred one may be lower alkanoyl; halo(lower)alkanoyl;

ar(lower)alkanoyl which may have one or more (preferably l to 3) hydroxy, lower alkoxy, higher alkoxy, aryl, amino, protected amino, di(lower)alkylamino, lower alkoxyimino or ar(lower)alkoxyimino which may have one or more (preferably l to 3) higher alkoxy;

heterocyclicthio(lower)alkanoyl which may have one or more (preferably l to 3) higher alkyl;

heterocyclic(lower)alkanoyl which may have one or more (preferably l to 3) lower alkoxyimino, higher alkyl, amino or protected amino;

ar(lower)alkoxyimino(lower)alkanoyl which may have one or more (preferably l to 3) higher alkoxy;

higher alkanoyl;

ar(lower)alkenoyl which may have one or more (preferably l to 3) higher alkoxy;

higher alkenoyl; lower alkoxycarbonyl; higher alkoxycarbonyl; aryloxycarbonyl;

arylsulfonyl which may have one or more (preferably l to 3) lower alkyl or higher alkoxy;

aroyl which may have one or more (preferably l to 5) halogen, lower alkyl, higher alkyl, carboxy, lower alkoxy which may have one or more (preferably l to l0) halogen, lower alkoxy(lower)alkoxy, ar(lower)alkoxy, higher alkoxy which may have one or more (preferably l to l7) halogen, higher alkenyloxy, aryl which may have one or more (preferably l to 3) higher alkoxy or aryloxy which may have one or more (preferably l to 3) lower alkoxy or higher alkoxy;

in which the more preferred one may be lower alkanoyl; halo(lower)alkanoyl;

phenyl(lower)alkanoyl or naphthyl(lower)alkanoyl, each of which may have l to 3 hydroxy, lower alkoxy, higher alkoxy, phenyl, amino, lower alkoxycarbonylamino, di(lower)alkylamino, lower alkoxyimino, or phenyl-(lower)alkoxyimino which may have l to 3 higher alkoxy;

pyridylthio(lower)alkanoyl which may have l to 3 higher alkyl;

imidazolyl(lower)alkanoyl or thiazolyl(lower)alkanoyl, each of which may have l to 3 lower alkoxyimino, higher alkyl, amino or lower alkoxycarbonylamino;

phenyl(lower)alkoxyimino(lower)alkanoyl which may have l to 3 higher alkoxy;

higher alkanoyl;

phenyl(lower)alkenoyl which may have l to 3 higher alkoxy;

higher alkenoyl; lower alkoxycarbonyl, higher alkoxycarbonyl; phenoxycarbonyl;

phenylsulfonyl or naphthylsulfonyl, each of which may have l to 3 lower alkyl or higher alkoxy;

benzoyl, naphthoyl or anthrylcarbonyl, each of which may have l to 5 halogen, lower alkyl, higher alkyl, carboxy, lower alkoxy which may have 6 to l0 halogen, lower alkoxy(lower)alkoxy, phenyl(lower)alkoxy, higher alkoxy which may have l2 to l7 halogen, higher alkenyloxy, phenyl which may have l to 3 higher alkoxy, phenoxy which may have l to 3 lower alkoxy or higher alkoxy;

the much more preferred one may be ($C_1$-$C_4$)alkanoyl; halo($C_1$-$C_4$)alkanoyl;

phenyl($C_1$-$C_4$)alkanoyl which may have l to 3 hydroxy, ($C_1$-$C_4$)alkoxy, ($C_7$-$C_{16}$)alkoxy, phenyl, amino, ($C_1$-$C_4$)alkoxycarbonylamino, di($C_1$-$C_4$)alkylamino, ($C_1$-$C_4$)alkoxyimino or phenyl($C_1$-$C_4$)alkoxyimino which may have ($C_7$-$C_{16}$)alkoxy;

naphthyl($C_1$-$C_4$)alkanoyl which may have l to 3 ($C_1$-$C_4$)alkoxycarbonylamino;

l-($C_7$-$C_{16}$)alkylpyridiniothio($C_1$-$C_4$)alkanoyl;

imidazolyl($C_1$-$C_4$)alkanoyl which may have 1 to 3 ($C_7$-$C_{16}$)alkyl or ($C_1$-$C_4$)alkoxycarbonylamino;

thiazolyl($C_1$-$C_4$)alkanoyl which may have 1 to 3 ($C_1$-$C_4$)alkoxyimino or amino;

phenyl($C_1$-$C_4$)alkoxyimino($C_1$-$C_4$)alkanoyl which may have l to 3 ($C_7$-$C_{16}$)alkoxy;

($C_7$-$C_{17}$)alkyl;

phenyl($C_1$-$C_4$)alkenoyl which may have l to 3 ($C_7$-$C_{16}$)alkoxy;

($C_7$-$C_{18}$)alkenoyl; ($C_3$-$C_6$)alkoxycarbonyl; $C_7$-$C_{16}$)alkoxycarbonyl; phenoxycarbonyl;

phenylsulfonyl which may have ($C_1$-$C_4$)alkyl or ($C_7$-$C_{16}$)alkoxy;

naphthylsulfonyl which may have ($C_7$-$C_{16}$)alkoxy;

benzoyl which may have l to 5 halogen, ($C_3$-$C_6$)alkyl, ($C_7$-$C_{16}$)alkyl, carboxy, ($C_1$-$C_6$)alkoxy which may have 6 to l0 halogen, ($C_1$-$C_4$)alkoxy($C_1$-$C_4$)alkoxy, phenyl($C_3$-$C_6$)alkoxy, ($C_7$-$C_{16}$)alkoxy which may have 12 to 17 halogen, phenyl which may have l to 3 ($C_7$-$C_{16}$)alkoxy or phenoxy which may have l to 3 ($C_3$-$C_6$)-alkoxy or ($C_7$-$C_{16}$)alkoxy

naphthoyl which may have l to 3 ($C_3$-$C_6$)alkoxy, ($C_7$-$C_{16}$)alkoxy or ($C_7$-$C_{16}$)alkenyloxy;

anthrylcarbonyl;

and the most preferred one may be acetyl,
2-bromoacetyl, 2-(4-biphenylyl)acetyl,
2-(4-octyloxyphenyl)acetyl, 3-(4-octyloxyphenyl)propionyl,
2-amino-2-(4-octyloxyphenyl)acetyl, 2-(t-butoxycarbonylamino)-2-(4-octyloxyphenyl)acetyl,
2-amino-3-(4-octyloxyphenyl)propionyl,
2-(t-butoxycarbonylamino)-3-(4-octyloxyphenyl)propionyl,
2-dimethylamino-3-(4-octyloxyphenyl)propionyl,
2-(t-butoxycarbonylamino)-2-(2-naphthyl)acetyl,
2-methoxy-2-(4-octyloxyphenyl)acetyl,
2-methoxyimino-2-(4-octyloxyphenyl)acetyl,
2-(4-octyloxybenzyloxyimino)-2-(4-hydroxyphenyl)acetyl,
2-(4-octyloxybenzyloxyimino)-2-phenylacetyl,
2-(4-octyloxybenzyloxyimino)acetyl,
2-(l-octyl-4-pyridinio)thioacetyl,
2-methoxyimino-2-(2-aminothiazol-4-yl)acetyl,
2-(t-butoxycarbonylamino)-3-(l-octyl-4-imidazolyl)propionyl, 3-(4-octyloxyphenyl)acryloyl,
3,7,ll-trimethyl-2,6,l0-dodecatrienoyl, t-butoxycarbonyl, octyloxycarbonyl, phenoxycarbonyl, p-tolylsulfonyl,
4-octyloxyphenylsulfonyl, 6-octyloxy-2-naphthylsulfonyl,
4-(t-butyl)benzoyl, 4-octylbenzoyl,
2,3,5,6-tetrafluoro-4-(2,2,3,3,4,4,5,5-octafluoropentyloxy)benzoyl, 4-(2-butoxyethoxy)benzoyl,
4-(4-phenylbutoxy)benzoyl, 4-octyloxybenzoyl,
2-carboxy-4-octyloxybenzoyl, 3-methoxy-4-octyloxybenzoyl,
4-(2,2,3,3,4,4,5,5,6,6,7,7,8,8,8-pentadecafluorooctyloxy)-2,3,5,6-tetrafluorobenzoyl, 4-(4-octyloxyphenyl)-benzoyl,
4-(4-octyloxyphenoxy)benzoyl, 6-butoxy-2-naphthoyl,
6-hexyloxy-2-naphthoyl, 6-octyloxy-2-naphthoyl,
6-(2-ethylhexyloxy)-2-naphthoyl, 6-decyloxy-2-naphthoyl,
6-(3,7-dimethyloctyloxy)-2-naphthoyl, 6-dodecyloxy-2-naphthoyl, 6-(3,7-dimethyl-6-octenyloxy)-2-naphthoyl,
6-(3,7-dimethyl-2,6-octadienyloxy)-2-naphthoyl,
2-anthrylcarbonyl , 4-(4-heptyloxyphenyl)benzoyl and
4-(4-hexyloxyphenoxy)benzoyl.

Suitable "ar(lower)alkanoyl" moiety in "ar(lower)alkanoyl which has higher alkoxy and protected amino" and "ar(lower)alkanoyl which has higher alkoxy and amino" can be referred to the ones as exemplified before for "acyl group" and suitable examples of the substituent(s) "higher alkoxy" and "protected amino" can be referred to the ones as exemplified before for "acyl group".

Suitable "halo(lower)alkanoyl" can be referred to the ones as exemplified before for "acyl group".

Suitable "pyridylthio(lower)alkanoyl" in "pyridylthio(lower)alkanoyl which may have higher alkyl" can be referred to the ones as exemplified before for "acyl group", and suitable examples of the substituent "higher alkyl" can be exemplified before for "acyl group".

Suitable "acyloxy" may include hydroxysulfonyloxy, phosphonooxy, and the like.

In the object compound [I] thus defined, the following compound [Ik] is especially preferable.

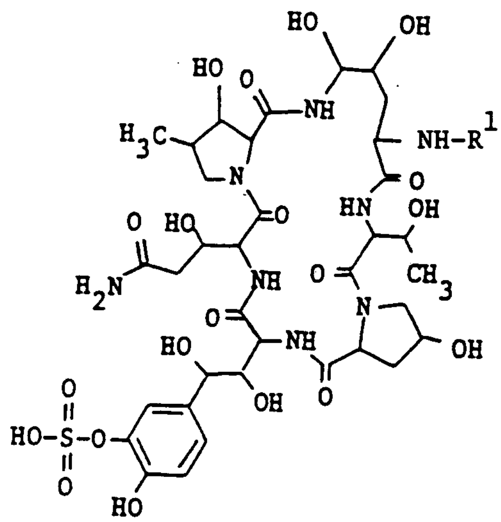

[Ik]

wherein $R^1$ is hydrogen or acyl group.

Suitable "acylating agent" for the acylation reaction is Process 4 may be an acid compound corresponding to the acyl group to be introduced or its reactive derivative at the carboxy group or a salt thereof and suitable example of said acylating agent is represented by the formula :

$R^1_a$ - OH [V]

wherein $R^1_a$ is as defined above,
or its reactive derivative at the carboxy group or a salt thereof.

In the compound [V], the following compounds are novel.

(1)

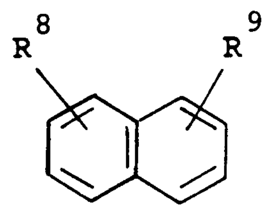

[V-1]

or its reactive derivative
at the carboxy group
or a salt thereof

(2)

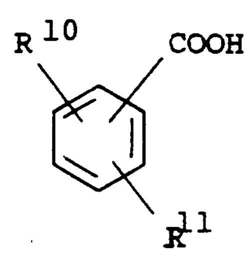

[V-2]

or its reactive derivative
at the carboxy group
or a salt thereof

wherein

$R^8$ is lower alkoxy, higher alkoxy or higher alkenyloxy,
$R^9$ is -COOH or $-SO_3H$,
$R^{10}$ is I to 4 halogen,
$R^{11}$ is lower alkoxy which has one or more halogen, higher alkoxy which has one or more halogen.

The compounds [V-l] and [V-2] can be prepared by the following processes.

Process A

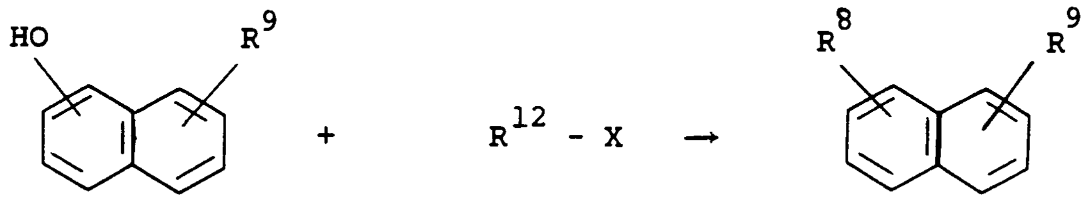

[VI] or a salt thereof — [VII] or a salt thereof — [V-1] or a salt thereof

Process B

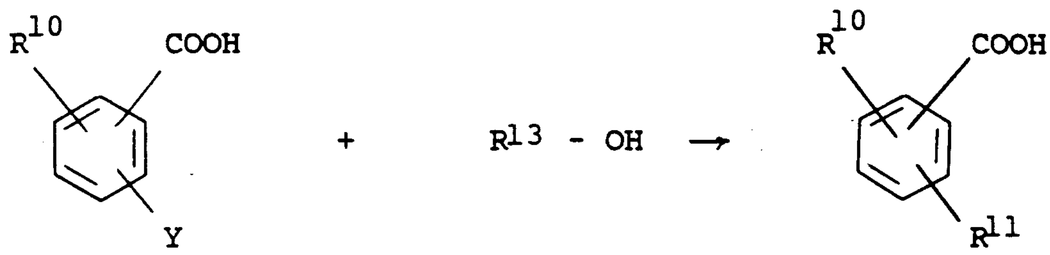

[VIII] or a salt thereof — [IX] or a salt thereof — [V-2] or a salt thereof

wherein

| | |
|---|---|
| $R^8$, $R^9$, $R^{10}$ and $R^{11}$ are | each as defined above, |
| $R^{12}$ is | lower alkyl, higher alkyl or higher alkenyl, |
| $R^{13}$ is | lower alkyl which has one or more halogen or higher alkyl which has one or more halogen, and |
| X and Y are | each a leaving group. |

In the above definitions, suitable "lower alkoxy", "higher alkoxy", "higher alkenyloxy", "halogen", "lower alkyl" and "higher alkyl" can be referred to the ones as exemplified before.

Suitable "higher alkenyl" may include 3-heptenyl, 7-octenyl, 2,6-octadienyl, 5-nonenyl, l-decenyl, 3,7-dimethyl-6-octenyl, 3,7-dimethyl-2,6-octadienyl, 8-undecenyl, 3,6,8-dodecatrienyl, 5-tridecenyl, 7-tetradecenyl, l,8-pentadecadienyl, l5-hexadecenyl, ll-heptadecenyl, 7-octadecenyl, l0-nonadecenyl, l8-icosenyl and the like, in which the preferred one may be $(C_7-C_{16})$alkenyl.

As for $R^{11}$, "lower alkoxy" has one or more (preferably l to 10, more preferably 6 to l0) halogen, and "higher alkoxy" has one or more (preferably l to l7, more preferably l2 to l7) halogen.

As for $R^{13}$, "lower alkyl" has one or more (preferably l to l0, more preferably 6 to l0) halogen, and "higher alkyl" has one or more (preferably l to l7, more preferably l2 to l7) halogen.

As for $R^8$, preferred "lower alkoxy" may be $(C_4-C_6)$alkoxy.

Suitable "a leaving group" may include aforesaid halogen, lower alkanoyloxy (e.g. acetoxy, etc.), sulfonyloxy (e.g. mesyloxy, tosyloxy, etc.), and the like.

Regarding suitable salts and the reactive derivatives at the carboxy group of the compounds [V-l] and [V-2], they can be referred to the ones as exemplified below for the compound [V].

The reactions in Processes A and B can be carried out according to the methods disclosed later in Preparations of the present specification or the similar manners thereto.

In the compound [V], there are other novel compounds than compounds [V-l] and [V-2], and they can be prepared, for example, by the methods disclosed later in Preparations.

Suitable "pyridinethione" in Process 6 may include l,2-dihydropyridine-2-thione, l,4-dihydropyridine-4-thione, and the like, and said "pyridinethione" may have aforesaid "higher alkyl".

The processes for preparing the object compound [I] or a salt thereof of the present invention are explained in detail in the following.

Process 1

The object compound [Ia] or a salt thereof can be prepared by the fermentation process.

The fermentation process is explained in detail in the following.

The compound [Ia] or a salt thereof of this invention can be produced by fermentation of the compound [Ia] or a salt thereof-producing strain belonging to the genus *Coleophoma* such as *Coleophoma* sp. F-11899 in a nutrient medium.

(i) Microorganism :

Particulars of the microorganism used for producing the compound [Ia] or a salt thereof is explained in the following.

The strain F-11899 was originally isolated from a soil sample collected at Iwaki-shi, Fukushima-ken, Japan. This organism grew rather restrictedly on various culture media, and formed dark grey to brownish grey colonies. Anamorph (conidiomata) produced on a steam-sterilized leaf segment affixed on a Miura's LCA plate[1)]or a corn meal agar plate by inoculating the isolate, while neither teleomorph nor anamorph formed on the agar media. Its morphological, cultural and physiological characteristics are as follows.

Cultural characteristics on various agar media are summarized in Table 1. Cultures on potato dextrose agar grew rather rapidly, attaining 3.5-4.0 cm in diameter after two weeks at 25°C. This colony surface was plane, felty, somewhat wrinkly and brownish grey. The colony center was pale grey to brownish grey, and covered with aerial hyphae. The reverse color was dark grey. Colonies on malt extract agar grew more restrictedly, attaining 2.5-3.0 cm in diameter under the same conditions. The surface was plane, thin to felty and olive brown. The colony center was yellowish grey, and covered with aerial hyphae. The reverse was brownish grey.

The morphological characteristics were determined on basis of the cultures on a sterilized leaf affixed to a Miura's LCA plate. Conidiomata formed on the leaf segment alone. They were pycnidial, superficial, separate, discoid to ampulliform, flattened at the base, unilocular, thin-walled, black, 90-l60(-200) $\mu$m in diameter and 40-70 $\mu$m high. Ostiole was often single, circular, central, papillate, l0-30 $\mu$m in diameter and l0-20 $\mu$m high. Conidiophores formed from the lower layer of inner pycnidial walls. They were hyaline, simple or sparingly branched, septate and smooth. Conidiogenous cells were enteroblastic, phialidic, determinate, ampulliform to obpyriform, hyaline, smooth, 5-8 x 4-6 $\mu$m, with a collarette. The collarettes were campanulate to cylindrical, and l4-l8 x 3-5 $\mu$m. Conidia were hyaline, cylindrical, thin-walled, aseptate, smooth and l4-l6(-l8) x 2-3 $\mu$m.

The vegetative hyphae were septate, brown, smooth and branched. The hyphal cells were cylindrical and 2-7 $\mu$m thick. The chlamydospores were absent.

The strain F-11899 had a temperature range for growth of 0 to 3l°C and an optimum temperature of 23 to 27°C on potato dextrose agar.

The above characteristics indicate that the strain F-11899 belongs to the order Coelomycetes[2), 3), 4)].

1) Miura, K. and M. Y. Kudo: An agar-medium for aquatic Hyphomycetes., Trans. Ycolo. Soc. Japan, ll:ll6-ll8, l970.

Thus, we named the strain "Coelomycetes strain F-11899".

Table 1 Cultural characteristics of the strain F-11899

| Medium | Cultural characteristics |
|---|---|
| Malt extract agar (Blakeslee 1915) | G: Rather restrictedly, 2.5-3.0 cm<br>S: Circular, plane, thin to felty, olive brown (4F5), arising aerial hyphae at the center (yellowish grey (4B2))<br>R: Brownish grey (4F2) |
| Potato dextrose agar (Difco 0013) | G: Rather rapidly, 3.5-4.0 cm<br>S: Circular, plane, felty, somewhat wrinkly, brownish grey (4F2), arising aerial hyphae at the center (pale grey (4B1) to brownish grey (4F2))<br>R: Dark grey (4F1) |

2) Arx, J. A. von: The Genera of Fungi - Sporulating in Pure Culture (3rd ed.), 3l5 p., J. Cramer, Vaduz, l974.

3) Sutton, B. C.: The Coelomycetes - Fungi Imperfecti with Pycnidia, Acervuli and Stromata., 696 p., Commonwealth Mycological Institute, Kew, l980.

4) Hawksworth, D. L., B. C. Sutton and G. C. Ainsworth: Dictionary of the Fungi (7th ed.), 445 p., Commonwealth Mycological Institute, Kew., l983.

| Medium | Cultural characteristics |
|---|---|
| Czapeck's solution agar (Raper and Thom 1949) | G: Very restrictedly, 1.0-1.5 cm<br>S: Irregular, thin, scanty, immersed, subhyaline to white<br>R: Subhyaline to white |
| Sabouraud dextrose agar (Difco 0109) | G: Restrictedly, 2.0-2.5 cm<br>S: Circular, plane, thin, white, sectoring, light brown (6D5) at the colony center<br>R: Pale yellow (4A3) |
| Oatmeal agar (Difco 0552) | G: Fairly rapidly, 4.0-4.5 cm<br>S: Circular, plane, felty to cottony, dark grey (4F1) to brownish grey (4F2)<br>R: Brownish grey (4D2) |
| Emerson Yp Ss agar (Difco 0739) | G: Restrictedly, 2.0-2.5 cm<br>S: Circular to irregular, plane, felty, dark grey (4F1) to brownish grey (4F2)<br>R: Medium grey (4E1) to dark grey (4F1) |
| Corn meal agar (Difco 0386) | G: Rather restrictedly, 2.5-3.0 cm<br>S: Circular, plane, thin to felty, dark grey (2F1) to olive (2F3)<br>R: Dark grey (2F1) to olive (2F3) |
| MY20 agar | G: Restrictedly, 1.5-2.0 cm<br>S: Circular to irregular, thin, sectoring, yellowish white (4A2)<br>R: Pale yellow (4A3) to orange white (5A2) |

Abbreviations : G: growth, measuring colony size in diameter
S: colony surface
R: reverse

These characteristics were observed after 14 days of incubation at 25 °C. The color descriptions were base on the Methuen Handbook of Colour[5].

A culture of Coelomycetes strain F-11899 thus named has been deposited with the Fermentation Research Institute Agency of Industrial Science and Technology (l-3, Higashi I chome, Tsukuba-shi, IBARAKI 305 JAPAN) on October 26, l989 under the number of FERM BP-2635.

After that, however, we have further studied the classification of the strain F-11899, and have found that the strain F-11899 resembled Coleophoma empetri (Rostrup) Petrak 1929 [2), 3), 4)]belonging to the order Coelomycetes, but differed in some pycnidial characteristics: globose or flattened at the base, immersed, and not papillate.

Considering these characteristics, we classified this strain in more detail and renamed it as "Coleophoma sp. F-11899".

In this connection, we have already taken step to amend the name, "Coelomycetes strain F-11899" to Coleophoma sp. F-11899 with the Fermentation Research Institute Agency of Industrial Science and Technology on September 21, l990.

(ii) Production of the compound [Ia] or a salt thereof

The compound [Ia] or a salt thereof of this invention is produced when the compound [Ia] or a salt thereof-producing strain belonging to the genus Coleophoma is grown in a nutrient medium containing sources of assimilable carbon and nitrogen under aerobic conditions (e.g. shaking culture, submerged culture, etc.).

The preferred sources of carbon in the nutrient medium are carbohydrates such as glucose, sucrose, starch, fructose or glycerin, or the like.

The preferred sources of nitrogen are yeast extract, peptone, gluten meal, cotton seed flour, soybean meal, corn steep liquor, dried yeast, wheat germ, etc., as well as inorganic and organic nitrogen compounds such as ammonium salts (e.g. ammonium nitrate, ammonium sulfate, ammonium phosphate, etc.), urea or amino acid, or the like.

The carbon and nitrogen sources, though advantageously employed in combination, need not to be used in their pure form because less pure materials, which contain traces of growth factors and considerable quantities of mineral nutrients, are also suitable for use.

When desired, there may be added to the medium mineral salts such as sodium or calcium carbonate, sodium or potassium phosphate, sodium or potassium chloride, sodium or potassium iodide, magnesium salts, copper salts, zinc salts, or cobalt salts, or the like.

If necessary, especially when the culture medium foams seriously a defoaming agent, such as liquid paraffin, fatty oil, plant oil, mineral oil or silicone, or the like may be added.

2) Arx, J. A. von: The Genera of Fungi - Sporulating in Pure Culture (3rd ed.), 3l5 p., J. Cramer, Vaduz, l974.

3) Sutton, B. C.: The Coelomycetes - Fungi Imperfecti with Pycnidia, Acervuli and Stromata., 696 p., Commonwealth Mycological Institute, Kew, l980.

4) Hawksworth, D. L., B. C. Sutton and G. C. Ainsworth: Dictionary of the Fungi (7th ed.), 445 p., Commonwealth Mycological Institute, Kew., l983.

5) Kornerup, A. and Wanscher, J. H.: Methuen Handbook of Colour (3rd ed.), 252 p., Methuen, London, l983.

As in the case of the preferred methods used for the production of other biologically active substances in massive amounts, submerged aerobic cultural conditions are preferred for the production of the compound [Ia] or a salt thereof in massive amounts.

For the production in small amounts, a shaking or surface culture in a flask or bottle is employed.

Further, when the growth is carried out in large tanks, it is preferable to use the vegetative form of the organism for inoculation in the production tanks in order to avoid growth lag in the process of production of the compound [Ia] or a salt thereof. Accordingly, it is desirable first to produce a vegetative inoculum of the organism by inoculating a relatively small quantity of culture medium with spores or mycelia of the organism and culturing said inoculated medium, and then to transfer the cultured vegetative inoculum to large tanks. The medium, in which the vegetative inoculum is produced, is substantially the same as or different from the medium utilized for the production of the compound [Ia] or a salt thereof.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The fermentation is usually conducted at a temperature between about 10 ° C and 40 ° C, preferably 20 ° C to 30 ° C, for a period of about 50 hours to l50 hours, which may be varied according to fermentation conditions and scales.

When the fermentation is completed, the culture broth is then subjected far recovery of the compound [Ia] or a salt thereof to various procedures conventionally used for recovery and purification of biological active substances, for instance, solvent extraction with an appropriate solvent or a mixture of some solvents, chromatography or recrystallization from an appropriate solvent or a mixture of some solvents, or the like.

According to this invention, in general, the compound [Ia] or a salt thereof is found both in the cultured mycelia and cultured broth. Accordingly, then the compound [Ia] or a salt thereof is removed from the whole broth by means of extraction using an appropriate organic solvent such as acetone or ethyl acetate, or a mixture of these solvents, or the like.

The extract is treated by a conventional manner to provide the compound [Ia] or a salt thereof, for example, the extract is concentrated by evaporation or distillation to a smaller amount and the resulting residue containing active material, i.e. the compound [Ia] or a salt thereof is purified by conventional purification procedures, for example, chromatography or recrystallization from an appropriate solvent or a mixture of some solvents.

When the object compound is isolated as a salt of the compound [$I_a$], it can be converted to the free compound [$I_a$] or another salt of the compound [$I_a$] according to a conventional manner.

Process 2

The compound [Ib] or a salt thereof can be prepared by subjecting the compound [Ia] or a salt thereof to elimination reaction of sulfo group.

Suitable salt of the compound [Ib] can be referred to the acid addition salt as exemplified for the compound [I].

This elimination reaction is carried out in accordance with a conventional method in this field of the art such as reaction with an enzyme or the like.

The reaction with an enzyme can be carried out by reacting the compound [Ia] or a salt thereof with an enzyme suitable for the elimination reaction of sulfo group.

Suitable example of said enzyme may include sulfatase such as sulfatase Type IV produced by Aerobacter aerogenes, or the like.

This elimination reaction is usually carried out in a solvent such as phosphate buffer, Tris-HCL buffer or any other solvent which does not adversely influence the reaction.

The reaction temperature is not critical and the reaction can be carried out at room temperature or under warming.

Process 3

The object compound [Id] or a salt thereof can be prepared by subjecting a compound [Ic] or a salt thereof to elimination reaction of N-acyl group.

This reaction is carried out in accordance with a conventional method such as hydrolysis, reduction, reaction with an enzyme or the like.

The hydrolysis is preferably carried out in the presence of a base or an acid including Lewis acid. Suitable base may include an inorganic base and an organic base such as an alkali metal [e.g. sodium, potassium, etc.], an alkaline earth metal [e.g. magnesium, calcium, etc.], the hydroxide or carbonate or bicarbonate thereof, trialkylamine [e.g. trimethylamine, triethylamine, etc.], picoline, l,5-diazabicyclo[4.3.0]-non-5-ene, l,4-diazabicyclo[2.2.2]octane, l,8-diazabicyclo[5.4.0]-undec-7-ene, or the like.

Suitable acid may include an organic acid [e.g. formic acid, acetic acid, propionic acid, trichloroacetic acid, trifluoroacetic acid, etc.] and an inorganic acid [e.g. hydrochloric acid, hydrobromic acid, sulfuric acid, hydrogen chloride, hydrogen bromide, etc.]. The elimination using Lewis acid such as trihaloacetic acid [e.g. trichloroacetic acid, trifluoroacetic acid, etc.] or the like is preferably carried out in the presence of cation trapping agents [e.g. anisole, phenol, etc.].

The reaction is usually carried out in a solvent such as water, an alcohol [e.g. methanol, ethanol, etc.], methylene chloride, tetrahydrofuran, a mixture thereof or any other solvent which does not adversely influence the reaction. A liquid base or acid can be also used as the solvent. The reaction temperature is not critical and the reaction is usually carried out under cooling to warming.

The reduction method applicable for the elimination reaction may include chemical reduction and catalytic reduction.

Suitable reducing agents to be used in chemical reduction are a combination of metal [e.g. tin, zinc, iron, etc.] or metallic compound [e.g. chromium chloride, chromium acetate, etc.] and an organic or inorganic acid [e.g. formic acid, acetic acid, propionic acid, trifluoroacetic acid, p-toluenesulfonic acid, hydrochloric acid, hydrobromic acid, etc.].

Suitable catalysts to be used in catalytic reduction are conventional ones such as platinum catalysts [e.g. platinum plate, spongy platinum, platinum black, colloidal platinum, platinum oxide, platinum wire, etc.], palladium catalysts [e.g. spongy palladium, palladium black, palladium oxide, palladium on carbon, colloidal palladium, palladium on barium sulfate, palladium on barium carbonate, etc.], nickel catalysts [e.g. reduced nickel, nickel oxide, Raney nickel, etc.], cobalt catalysts [e.g. reduced cobalt, Raney cobalt, etc.], iron catalysts [e.g. reduced iron, Raney iron, etc.], copier catalysts [e.g. reduced copper, Raney copper, Ullman copper, etc.] and the like.

The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, etc., or a mixture thereof.

The reaction temperature of this reduction is not critical and the reaction is usually carried out under cooling to warming.

The reaction with an enzyme can be carried out by reacting the compound [Ic] or a salt thereof with an enzyme suitable for the elimination reaction of N-acyl group.

Suitable example of said enzyme may include the one produced by certain microorganisms of the Actinoplanaceae, for example, Actinoplanes utahensis IFO-l3244, Actinoplanes utahensis ATCC l230l, Actinoplanes missourienses NRRL l2053, or the like; and the like.

This elimination reaction is usually carried out in a solvent such as phosphate buffer, Tris-HCl buffer or any other solvent which does not adversely influence the reaction

The reaction temperature is not critical and the reaction can be carried out at room temperature or under warming.

Process 4

The object compound [Ie] or a salt thereof can be prepared by subjecting the compound [Id] or a salt thereof to acylation reaction.

The acylation reaction of this process can be carried out by reacting the compound [Id] or a salt thereof with aforesaid "acylating agent", for example, the compound [V] or its reactive derivative at the carboxy group or a salt thereof.

Suitable reactive derivative at the carboxy group of the compound [V] may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid diphenylphosphoric acid, dibenzylphosphoric acid, halogenated phosphoric acid, etc.], dialkylphosphorous acid, sulfurous acid, thiosulfuric acid, sulfuric acid, sulfonic acid [e.g. methanesulfonic acid, etc.], aliphatic carboxylic acid [e.g. acetic acid,

propionic acid, butyric acid, isobutyric acid, pivaric acid, pentanoic acid, isopentanoic acid, 2-ethylbutyric acid, trichloroacetic acid, etc.]; or aromatic carboxylic acid [e.g. benzoic acid, etc.]; a symmetrical acid anhydride; an activated amide with imidazole, 4-substituted imidazole, dimethylpyrazole, triazole, tetrazole or l-hydroxy-lH-benzotriazole; or an activated ester [e.g. cyanomethyl ester, methoxymethyl ester, dimethyliminomethyl $[(CH_3)_2\overset{+}{N}=CH-]$ ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.], or an ester with a N-hydroxy compound [e.g. N,N-dimethylhydroxylamine, l-hydroxy-2-(lH)-pyridone, N-hydroxysuccinimide, N-hydroxyphthalimide, l-hydroxy-lH-benzotriazole, etc.], and the like. These reactive derivatives can optionally be selected from them according to the kind of the compound [V] to be used.

Suitable salts of the compound [V] and its reactive derivative can be referred to the ones as exemplified for the compound [I].

The reaction is usually carried out in a conventional solvent such as water, alcohol [e.g. methanol, ethanol, etc.], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound [V] is used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)-carbodiimide; N,N'-diethylcarbodiimide, N,N'-diisopropylcarbodiimide; N-ethyl-N'-(3-dimethylaminopropyl)-carbodiimide, N,N'-carbonylbis-(2-methylimidazole); pentamethyleneketene-N-cyclohexylimine; diphenylketene-N-cyclohexylimine; ethoxyacetylene; l-alkoxy-l-chloroethylene; trialkyl phosphite; ethyl polyphosphate; isopropyl polyphosphate; phosphorus oxychloride (phosphoryl chloride); phosphorus trichloride; thionyl chloride; oxalyl chloride; lower alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl)isoxazolium hydroxide intramolecular salt; l-(p-chlorobenzenesulfonyloxy)-6-chloro-lH-benzotriazole; so-called Vilsmeier reagent prepared by the reaction of N,N-dimethylformamide with thionyl chloride, phosgene, trichloromethyl chloroformate, phosphorus oxychloride, methanesulfonyl chloride, etc.; or the like.

The reaction may also be carried out in the presence of an inorganic or organic base such as an alkali metal carbonate, alkali metal bicarbonate, tri(lower)alkylamine, pyridine, di(lower)alkylaminopyridine (e.g. 4-dimethylaminopyridine, etc.), N-(lower)alkylmorpholine, N,N-di(lower)alkylbenzylamine, or the like.

The reaction temperature is not critical, and the reaction is usually carried out under cooling to warming.

Process 5

The object compound [Ig] or a salt thereof can be prepared by subjecting a compound [If] or a salt thereof to elimination reaction of amino protective group.

Suitable salts of the compounds [If] and [Ig] can be referred to the ones as exemplified for the compound [I].

This elimination reaction can be carried out in accordance with a conventional method as explained above for Process 3.

Process 6

The object compound [Ii] or a salt thereof can be prepared by reacting a compound [Ih] or a salt thereof with a compound [ II ] or a salt thereof.

Suitable salt of the compound [Ii] can be referred to the ones as exemplified for the compound [I].

Suitable salt of the compound [ II ] can be referred to acid addition salts as exemplified for the compound [I].

The present reaction may be carried out in a solvent such as water, phosphate buffer, acetone, chloroform, acetonitrile, nitrobenzene, methylene chloride, ethylene chloride, formamide, N,N-dimethylformamide, methanol, ethanol, diethyl ether, tetrahydrofuran, dimethyl sulfoxide, or any other organic solvent which does not adversely affect the reaction, preferably in ones having strong polarities. Among the solvents, hydrophilic solvents may be used in a mixture with water. When the compound [ II ] is in liquid, it can also be used as a solvent.

The reaction is preferably conducted in the presence of a base, for example, inorganic base such as alkali metal hydroxide, alkali metal carbonate, alkali metal bicarbonate, organic base such as trialkylamine, and the like.

The reaction temperature is not critical, and the reaction is usually carried out under cooling, at room temperature, under warming or under heating.

The present reaction is preferably carried out in the presence of alkali metal halide [e.g. sodium iodide, potassium iodide, etc.], alkali metal thiocyanate [e.g. sodium thiocyanate, potassium thiocyanate, etc.] or the like.

Process 7

The object compound [Ij] or a salt thereof can be prepared by subjecting a compound [III]or a salt thereof to acylation reaction.

Suitable salts of the compounds [Ij] and [III]can be referred to the ones as exemplified for the compound [I].

Suitable "acylating agent" in this Process 7 may be an acid compound corresponding to the acyl group to be introduced, for example, phosphoric acid and its derivative (e.g. phosphoryl chloride, diphenylphosphorochloridate, etc.), sulfuric acid and its derivative [e.g. sulfur trioxide-pyridine, sulfur trioxide-tri(lower)alkylamine (e.g. trimethylamine, triethylamine, etc.), chlorosulfonic acid, etc.], or the like.

This reaction can be carried out in a conventional manner.

The following Preparations and Examples are given for the purpose of illustrating the present invention in more detail.

Preparation 1

To methanol (50 ml) was added thionyl chloride (8.73 ml) at -5°C and the mixture was stirred for l0 minutes and then D-2-(p-hydroxyphenyl)glycine (5 g) was added thereto under ice-cooling. The mixture was stirred for 12 hours at room temperature. The reaction mixture was evaporated under reduced pressure to give D-2-(p-hydroxyphenyl)-glycine methyl ester hydrochloride (6.3 g).

IR (Nujol) : 3380, l720, l580, l250 $cm^{-1}$

NMR ($DMSO-d_6$, δ) : 3.70 (3H, s), 5.ll (lH, s), 6.83 (2H, d, J=8.6Hz), 7.28 (2H, d, J=8.6Hz), 8.91 (2H, s), 9.93 (lH, s)

Preparation 2

To a solution of D-2-(p-hydroxyphenyl)glycine methyl ester hydrochloride (6.3 g) and triethylamine (8.7l ml) in tetrahydrofuran (l00 ml) was added di-t-butyl dicarbonate (6.82 g). The mixture was stirred for 2 hours at room temperature. The reaction mixture was added to diethyl ether (1 ℓ) and an insoluble material was filtered off, and the filtrate was evaporated under reduced pressure to give N-(t-butoxycarbonyl)-D-2-(p-hydroxyphenyl)glycine methyl ester (6.83 g).

IR (Nujol) : 3420, 3350, l720, l660 $cm^{-1}$

NMR ($DMSO-d_6$, δ) : l.38 (9H, s), 3.59 (3H, s), 5.05 (lH, d, J=7.9Hz), 6.70 (2H, d, J=8.5Hz), 7.l6 (2H, d, J=8.5Hz), 7.60 (lH, d, J=7.9Hz), 9.48 (lH, s)

Preparation 3

To a suspension of N-(t-butoxycarbonyl)-D-2-(p-hydroxyphenyl)glycine methyl ester (6.8 g) and potassium bicarbonate (l.84 g) in N,N-dimethylformamide (34 ml) was added octyl bromide (4.l76 ml). The mixture was stirred for 6 hours at 60°C. The reaction mixture was added to a mixture of water and ethyl acetate. The organic layer was separated and dried over magnesium sulfate. The magnesium sulfate was filtered off, and the filtrate was evaporated under reduced pressure to give N-(t-butoxycarbonyl)-D-2-(p-octyloxyphenyl)glycine methyl ester (6.96 g).

IR (Nujol) : l7l0, l490, l240, ll60 $cm^{-1}$

NMR ($DMSO-d_6$, δ) : 0.859 (3H, t, J=6.2Hz), l.l7-l.33 (l0H, m), l.38 (9H, s), l.60-l.80 (2H, m), 3.59 (3H, s), 3.93 (2H, t, J=6.3Hz), 5.ll (lH, d, J=7.9Hz), 6.87 (2H, d, J=8.7Hz), 7.27 (2H, d, J=8.7Hz), 7.68 (lH, d, J=7.9Hz)

Preparation 4

To 4N aqueous solution of sodium hydroxide (8 77 ml) was added N-(t-butoxycarbonyl)-D-2-(p-octyloxyphenyl)-glycine methyl ester (6.9 g) and stirred for l.5 hours at room temperature. The reaction mixture was added to a mixture of water and ethyl acetate and lN hydrochloric acid was added thereto to adjust thee mixture to pH 3. The organic layer was separated and dried over magnesium sulfate. The magnesium sulfate was filtered off, and the filtrate was evaporated under reduced pressure to give N-(t-butoxycarbonyl)-D-2-(p-octyloxyphenyl)glycine (3.9 g).

NMR (DMSO-$d_6$, $\delta$): 0.860 (3H, t, J=6.8Hz), l.l7-l.33 (l0H, m), l.38 (9H, s), l.60-l.80 (2H, m), 3.93 (2H, t, J=6.4Hz), 5.l0 (lH, d, J=8.2Hz), 6.87 (2H, d, J=8.7Hz), 7.28 (2H, d, J=8.7HZ), 7.46 (lH, d, J=8.2Hz)

Preparation 5

To a solution of N-(t-butoxycarbonyl)-D-2-(p-octyloxyphenyl)glycine (l g) in acetonitrile (l0 ml) and pyridine (0.2l3 ml) in acetonitrile (l0 ml) was added N,N'-disuccinimidyl carbonate (0.675 g). The mixture was stirred for l2 hours at room temperature. The reaction mixture was added to a mixture of water and ethyl acetate. The organic layer was separated and dried over magnesium sulfate. The magnesium sulfate was filtered off, and the filtrate was evaporated under reduced pressure to give N-(t-butoxycarbonyl)-D-2-(p-octyloxyphenyl)glycine succinimido ester (0.92 g).

IR (Nujol) : 3350, l8l0, l730, l680 $cm^{-1}$

NMR (DMSO-$d_6$, $\delta$) : 0.862 (3H, t, J=6.7Hz), l.l7-l.33 (l0H, m), l.40 (9H, s), l.60-l.80 (2H, m), 2.77 (4H, s), 3.97 (2H, t, J=6.5Hz), 5.54 (lH, d, J=8.lHz), 6.9l (2H, d, J=8.7Hz), 7.39 (2H, d, J=8.7Hz), 8.05 (lH, d, J=8.lHz)

Preparation 6

N-(t-Butoxycarbonyl)-L-tyrosine methyl ester was obtained according to a similar manner to that of Preparation 2.

IR (Nujol) : 3430, 3360, l730, l670, ll70 $cm^{-1}$

NMR (DMSO-$d_6$, $\delta$) : l.33 (9H, s), 2.90 (2H, m), 3.59 (3H, s), 4.05 (lH, m), 6.65 (2H, d, J=8.4Hz), 7.00 (2H, d, J=8.4Hz), 7.2l (lH, d, J=8.0Hz), 9.22 (lH, s)

Preparation 7

$O^4$-Octyl-N-(t-butoxycarbonyl)-L-tyrosine methyl ester was obtained according to a similar manner to that of Preparation 3.

IR (Nujol) : 3350, l735, l685, l250, ll70 $cm^{-1}$

NMR (DMSO-$d_6$, $\delta$) : 0.859 (3H, t, J=6.7Hz), l.20-l.30 (l0H, m), l.68 (2H, quintet, J=7.3Hz), 2.82 (2H, m), 3.60 (3H, s), 3.9l (2H, t, J=7.3Hz), 4.08 (lH, m), 6.8l (2H, d, J=8.6Hz), 7.l2 (2H, d, J=8.6Hz), 7.25 (lH, d, J=8.0Hz)

Preparation 8

$O^4$-Octyl-N-(t-butoxycarbonyl)-L-tyrosine was obtained according to a similar manner to that of Preparation 4.

IR (Nujol) : 3400-2900 (br), l700, l240, ll60 $cm^{-1}$

NMR (DMSO-$d_6$, $\delta$) : 0.859 (3H, t, J=6.8Hz), l.20-l.30 (l0H, m), l.32 (9H, s), l.68 (2H, quintet, J=7.0Hz), 2.67-2.95 (lH, m), 3.90 (2H, t, J=7.0Hz), 4.0l (lH, m), 6.8l (2H, d, J=8.6Hz), 7.02 (lH, d, J=8.3Hz), 7.l3 (2H, d, J=8.6Hz)

Preparation 9

$O^4$-Octyl-N-(t-butoxycarbonyl)-L-tyrosine succinimido ester was obtained according to a similar manner to that of Preparation 5.

IR (Nujol) : 3350, l780, l720, l690 $cm^{-1}$

NMR ($DMSO-d_6$, $\delta$) : 0.860 (3H, t, J=6.7Hz), l.20-l.30 (l0H, m), l.32 (9H, s), l.68 (2H, quintet, J=7.0Hz), 2.82 (4H, s), 2.80-3.20 (lH, m), 3.92 (2H, t, J=7.0Hz), 4.44 (lH, m), 6.8l (2H, d, J=8.5Hz), 7.22 (2H, d, J=8.5Hz), 7.60 (lH, d, J=8.3Hz)

Preparation l0

(1) A seed medium (160 ml) consisting of sucrose 4%, cotton seed flour 2%, dried yeast l%, peptone l%, $KH_2PO_4$ 0.2%, $CaCO_3$ 0.2% and Tween 80 (made by NAKARAI CHEMICALS LTD.) 0.l% was poured into each of two 500 ml Erlenmeyer flasks and sterilized at l2l°C for 30 minutes. A loopful of slant culture of Coleophoma sp. F-11899 was inoculated to each of the medium and cultured under shaking condition at 25°C for 4 days.

A production medium (20 liters)consisting of Pine Dex #3 (made by Matsutani Chemical Ltd.) 3%, glucose l%, wheat germ l%, cotton seed flour 0.5%, $KH_2PO_4$ 2%, $Na_2HPO_4 \cdot 12H_2O$ l.5%, $ZnSO_4 \cdot 7H_2O$ 0.00l% and Adekanol (defoaming agent, made by Asahi Denka Co., Ltd.) 0.05% was poured into a 30 liter-jar fermentor and sterilized at 121°C for 30 minutes.

The resultant seed culture broth (320 ml) was inoculated to the production medium and cultured at 25°C for 4 days, agitated at 200 rpm and aerated at 20 liters per minute. To the cultured broth thus obtained (20 liters) was added an equal volume of acetone. After occasionally stirring at room temperature for a while, the broth was filtered. The filtrate was concentrated in vacuo to remove acetone. The aqueous filtrate (l0 liters) was washed with two equal volume of ethyl acetate and extracted with n-butanol (l0 liters) twice. The combined n-butanol layer was concentrated in vacuo and the residue was applied on a column (300 ml) of silica gel 60 (made by E. Merck) and eluted with a stepwise organic solvent mixture consisting of dichloromethane-methanol. The fractions having anti-Candida activity were eluted in the range of the solvent mixture (3:l through l:l). The active fractions were combined and concentrated in vacuo to dryness. The residue was dissolved in 50% aqueous methanol (l5 ml) and applied on a column (250 ml) of ODS YMC GEL (made by Yamamura Chemical Lab.). The column was washed with 50% aqueous methanol and eluted with 80% aqueous methanol. The eluate was concentrated and was further purified on a centrifugal partition chromatography (CPC) using a solvent system n-butanol:methanol:water (4:l:5) of upper stationary phase and lower mobile phase in a descending mode. The pooled fractions containing the object compound (major component) were concentrated in vacuo and applied on a column (35 ml) of silica gel 60. The column was developed with n-butanol:acetic acid:water (6:l:l). The active fractions were combined and concentrated in vacuo to dryness and dissolved in a small volume of 50% aqueous methanol. The solution was passed through a column (3.5 ml) of ODS YMC GEL. The column was washed with 50% aqueous methanol and eluted with methanol. The eluate was concentrated to dryness, dissolved in a small volume of water and adjusted to pH 7.0 with 0.0lN NaOH. The solution was freeze-dried to give a white powder of said compound in its sodium salt form (hereinafter referred to as FR901379 substance) (ll mg).

The fractions containing two minor components after CPC was concentrated in vacuo and purified on a preparative high performance liquid chromatography (HPLC), column of LiChrosorb RP-18 (Trademark, made by Merck 250 x $\phi$25 mm) using a mobile phase composed of 45% aqueous $CH_3CN$-0.5% $NH_4H_2PO_4$ at a flow rate of 9.9 ml/minute. The fraction containing one of the two components was diluted with an equal volume of water and passed through a column (l ml) of ODS YMC Gel. The column was washed with 40% aqueous MeOH and eluted with MeOH. The eluate was concentrated in vacuo to dryness, then dissolved in a small volume of water and freeze-dried to give said component in its ammonium salt form as a white powder (2.2 mg) (hereinafter referred to as FR901381 substance).

In a similar manner, the other minor component in its ammonium salt form was obtained as a white powder (l.2 mg) (hereinafter referred to as FR901382 substance).

The FR901379 substance as obtained has the following physico-chemical properties.

Appearance :
white powder
Nature :
neutral substance
Melting point :
215-221°C (dec.)
Specific rotation :
$[\alpha]_D^{23}$ -20.3 (C: 0.5, $H_2O$)
Molecular formula :

$C_{51}H_{81}N_8O_{21}SNa$

| Elemental Analysis : | | | | |
|---|---|---|---|---|
| Calcd : for $C_{51}H_{81}N_8SO_{21}Na$ | C 51.17, | H 6.77, | N 9.36, | S 2.68 (%) |
| Found : | C 49.61, | H 7.58, | N 7.65, | S 2.14 (%) |

Molecular weight :
HRFAB-MS 1219.5078
(Calcd for $C_{51}H_{82}N_8SO_{21}$ + 2Na - H: 1219.5032)
Solubility :
soluble : methanol, water
slightly soluble : ethyl acetate, acetone
insoluble : chloroform, n-hexane
Color reaction :
positive : iodine vapor reaction, cerium sulfate reaction, ferric chloride reaction, Ninhydrin reaction
negative : Dragendorff reaction, Ehrlich reaction
Thin layer chromatography (TLC) :

| Stationary phase | Developing solvent | Rf value |
|---|---|---|
| silica gel* | n-butanol:acetic acid: water (3:1:1) | 0.36 |
| | ethyl acetate:isopropyl alcohol:water (5:3:1) | 0.31 |

* Silica Gel 60 (made by E. Merck)

Ultraviolet absorption spectrum :

$$\lambda_{max}^{methanol} \ (E_{1cm}^{1\%}):$$

207(169), 276(13.5), 225(sh), 283(sh) nm

$$\lambda_{max}^{methanol+0.01N-NaOH} \ (E_{1cm}^{1\%}):$$

209 (232), 244(59.5), 284(13.5), 294(sh) nm
Infrared absorption spectrum :

$$\nu_{max}^{KBr}:$$

3350, 2920, 2840, 1660, 1625, 1530, 1510, 1435, 1270, 1240, 1070, 1045, 800, 755, 710 $cm^{-1}$
$^{1}H$ Nuclear magnetic resonance spectrum :
($CD_3OD$, 400MHz)

δ : 7.30 (1H, d, J = 2Hz), 7.03 (1H, dd, J = 8 and 2Hz), 6.85 (1H, d, J = 8Hz), 5.23 (1H, d, J = 3Hz), 5.06 (1H, d, J = 4Hz), 4.93 (1H, d, J = 3Hz), 4.59-4.51 (3H, m), 4.47-4.35 (5H, m), 4.29 (1H, dd, J = 6 and 2Hz), 4.17 (1H, m), 4.07 (1H, m), 3.95-3.89 (2H, m), 3.76 (1H, broad d, J = 11Hz), 3.36 (1H, m), 2.75 (1H, dd, J = 16 and 4Hz), 2.50 (1H, m), 2.47 (1H, dd, J = 16 and 9Hz), 2.38 (1H, m), 2.21 (2H, m), 2.03-1.93 (3H, m), 1.57 (2H, m), 1.45-1.20 (24H, m), 1.19 (3H, d, J = 6Hz), 1.08 (3H, d, J = 6Hz), 0.90 (3H, t, J = 7Hz)

From the analysis of the above physical and chemical properties, and the result of the further investigation of identification of chemical structure, the chemical structure of the FR901379 substance has been identified and assigned as follows.

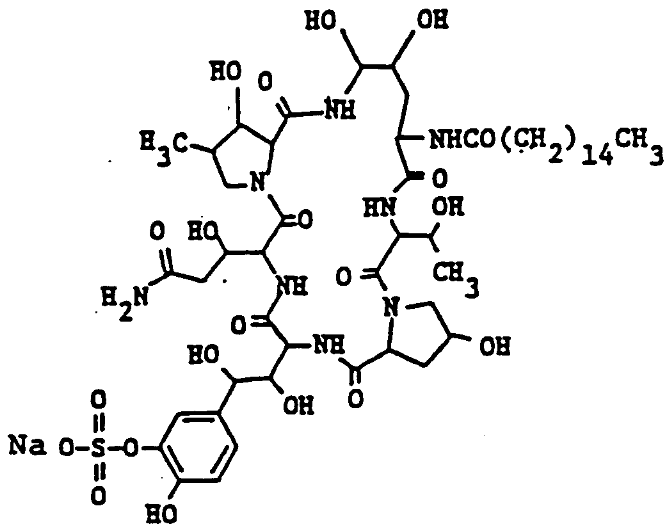

The FR901381 substance as obtained has the following physico-chemical properties.

Appearance :
white powder
Nature :
neutral substance
Melting point :
218-223 °C (dec.)
Specific rotation :
$[\alpha]_D^{23}$ -10.5 ° (C: 0.5, MeOH)
Molecular formula :

$C_{51}H_{81}N_8O_{20}S \cdot NH_4$

Molecular weight
HRFAB-MS 1203.5100
(Calcd for $C_{51}H_{82}N_8O_{20}S$ + 2Na - H : 1203.5083)
Solubility :
soluble : methanol, ethanol
slightly soluble : water, acetone
insoluble : chloroform, n-hexane
Color reaction :
positive : iodine vapor reaction, cerium sulfate reaction
negative : Dragendorff reaction, Ehrlich reaction
Thin layer chromatography (TLC) :

| Stationary phase | Developing solvent | Rf value |
|---|---|---|
| silica gel* | n-butanol:acetic acid: water (3:1:1)<br>ethyl acetate:isopropyl alcohol:water (5:3:l) | 0.34<br>0.67 |

* Silica Gel 60 (made by E. Merck)

Ultraviolet absorption spectrum :

$$\lambda_{max}^{methanol} (E_{1cm}^{1\%}) :$$

206(196), 278(4), 243(sh), 284(sh) nm

$$\lambda_{max}^{methanol+0.01N\text{-}NaOH} (E_{1cm}^{1\%}) :$$

208(252), 290(5), 241(sh) nm
Infrared absorption spectrum :

$$\nu_{max}^{KBr} :$$

3300, 2900, 2840, 1680, 1660, 1640, 1620, 1510, 1460, 1430, 1330, 1240, 1040, 960 $cm^{-1}$
$^{1}H$ Nuclear magnetic resonance spectrum :
($CD_3OD$ 400 MHz)
δ : 7.18 (1H, d, J=2Hz), 6.90 (1H, dd, J=2 and 8.5Hz), 6.81 (1H, d, J=8.5Hz), 5.29 (1H, d, J=3Hz), 5.08 (1H, d, J=3.5Hz), 4.98 (1H, d, J=3Hz), 4.63 (1H, dd, J=7 and 11Hz), 4.58-4.51 (3H, m), 4.46-4.38 (3H, m), 4.37 (1H, d, J=2Hz), 4.16 (1H, dd, J=2 and 5Hz), 4.07 (1H, dd, J=7.5 and 9.5Hz), 4.02-3.94 (2H, m), 3.78 (1H, br d, J=11Hz), 3.38 (1H, t, J=9.5Hz), 2.69 (1H, dd, J=4.5 and 15Hz), 2.63-2.50 (3H, m), 2.46 (1H, m), 2.43 (1H, dd, J=9 and 15Hz), 2.21 (2H, t, J=7.5Hz), 2.07-1.95 (3H, m), 1.58 (2H, m), 1.29 (24H, m), 1.16 (3H, d, J=6.5Hz), 1.07 (32, d, J=7Hz), 0.89 (3H, t, J=6.5Hz)
$^{13}C$ Nuclear magnetic resonance spectrum :
($CD_3OD$, 100MHz)
δ : 176.7 (s), 175.9 (s), 174.4 (s), 174.0 (s), 172.8 (s), 172.5 (s), 172.5 (s), 169.4 (s), 149.1 (s), 141.1 (s), 131.1 (s), 128.0 (d), 125.3 (d), 118.3 (d), 75.9 (d), 74.0 (d), 73.9 (d), 71.3 (d), 70.7 (d), 70.5 (d), 70.2 (d), 68.2 (d), 62.4 (d), 58.6 (d), 58.4 (d), 57.2 (t), 55.5 (d), 52.9 (t), 51.4 (d), 40.8 (t), 39.9 (t), 39.1 (d), 39.0 (t), 36.7 (t), 35.0 (t), 33.1 (t), 30.8 (t x 5), 30.7 (t), 30.7 (t), 30.5 (t), 30.4 (t), 30.3 (t), 27.0 (t), 23.7 (t), 19.5 (g), 14.4 (g), 11.1 (q)

From the analysis of the above physical and chemical properties, and the result of the further investigation for identification of chemical structure, the chemical structure of the FR901381 substance has been identified and assigned as follows.

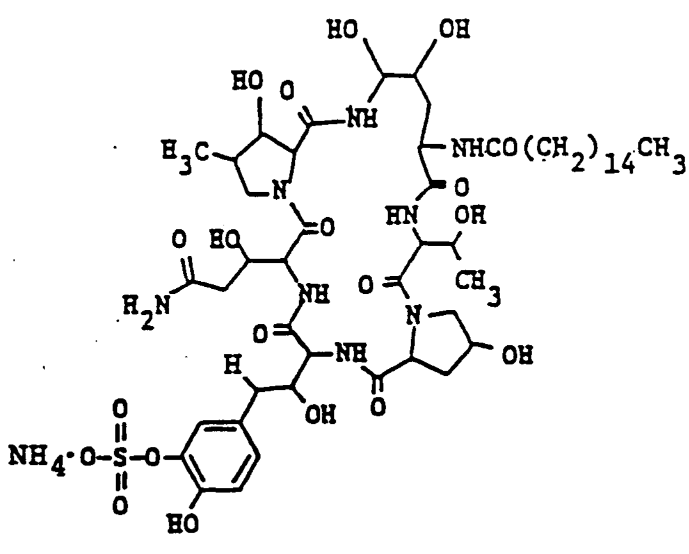

The FR901382 substance as obtained has the following physico-chemical properties.
Appearance :
white powder
Nature :
neutral substance
Melting point :
208-2l7 ° C (dec.)
Specific rotation :
$[\alpha]_D^{23}$ -9.4 ° (C: 0.5, MeOH)
Molecular formula :

$C_{51}H_{81}N_8O_{19}S \cdot NH_4$

Molecular weight :
HRFAB-MS 1187.5139
(Calcd. for $C_{51}H_{82}N_8O_{19}S$ + 2Na - H 1187.5134)
Solubility :
soluble : methanol, ethanol
slightly soluble : water, acetone
insoluble : chloroform, n-hexane
Color reaction :
positive : iodine vapor reaction, cerium sulfate reaction
negative : Dragendorff reaction, Ehrlich reaction
Thin layer chromatography (TLC) :

| Stationary phase | Developing solvent | Rf value |
|---|---|---|
| silica gel* | n-butanol:acetic acid: water (3:l:l) | 0.43 |
| | ethyl acetate:isopropyl alcohol:water (5:3:l) | 0.9 |

* Silica Gel 60 (made by E. Merck)

Ultraviolet absorption spectrum :

$$\lambda_{max}^{methanol} \ (E_{1cm}^{1\%}) :$$

205(l80), 276(13), 224(sh), 283(sh) nm

$$\lambda_{max}^{methanol+0.01N-NaOH} \ (E_{1cm}^{1\%}) :$$

208(262), 28l(12), 24l(sh), 295(sh) nm
Infrared absorption spectrum :

$$\nu_{max}^{KBr} :$$

3350, 2900, 2840, l680, l660, l640, l620, l5l0, l430, l330, l245, l080, l040, 960 $cm^{-1}$
$^1H$ Nuclear magnetic resonance spectrum :
($CD_3OD$, 400MHz)
δ : 7.l8 (lH, d, J=2Hz), 6.90 (lH, dd, J=2 and 8.5Hz), 6.80 (lH, d, J=8.5Hz), 5.37 (lH, dd, J=3 and llHz), 5.08 (lH, d, J=3.5Hz), 5.00 (lH, d, J=3Hz), 4.6l (lH, dd, J=7 and llHz), 4.59 (lH, d, J=2Hz), 4.58-4.52 (2H, m), 4.46-4.35 (3H, m), 4.29 (lH, d, J=2Hz), 4.l2 (lH, dd, J=2 and 4.5Hz), 4.07 (lH,

dd, J=8 and 9.5Hz), 4.0l (lH, dd, J=3 and llHz), 3.77 (lH, br d, J=llHz), 3.37 (lH, t, J=9.5Hz), 2.69 (lH, dd, J=4.5 and l5.5Hz), 2.63-2.50 (3H, m), 2.45 (lH, m), 2.43 (lH, dd, J=9 and l5.5Hz), 2.24 (2H, m), 2.09-l.95 (3H, m), l.76-l.66 (2H, m), l.59 (2H, m), l.29 (24H, m), l.l5 (3H, d, J=6.5Hz), l.06 (3H, d, J=7Hz), 0.89 (3H, t, J=7Hz)

$^{13}C$ Nuclear magnetic resonance spectrum :
($CD_3OD$, l00MHz)
δ : l76.7 (s), l76.0 (s), l75.l (s), l74.0 (s), l72.8 (s), l72.6 (s), l72.5 (s), l69.l (s), l49.l (s), l4l.l (s), l3l.l (s), l28.l (d), l25.3 (d), ll8.2 (d), 76.l (d), 74.0 (d), 7l.8 (d), 7l.3 (d), 70.5 (d), 70.3 (d), 68.3 (d), 62.5 (d), 58.5 (d), 58.2 (d), 57.2 (t), 55.4 (d), 52.9 (t), 52.l (d), 40.8 (t), 39.8(t), 39.l (d), 38.9 (t), 36.8 (t), 33.l (t), 30.9 (t), 30.8 (t x 5), 30.7 (t), 30.7 (t), 30.5 (t), 30.4 (t), 30.3 (t), 27.3 (t), 26.9 (t), 23.7 (t), l9.4 (q), l4.4 (q), ll.l (q)

From the analysis of the above physical and chemical properties, and the result of the further investigation for identification of chemical structure, the chemical structure of the FR90l382 substance has been identified and assigned as follows.

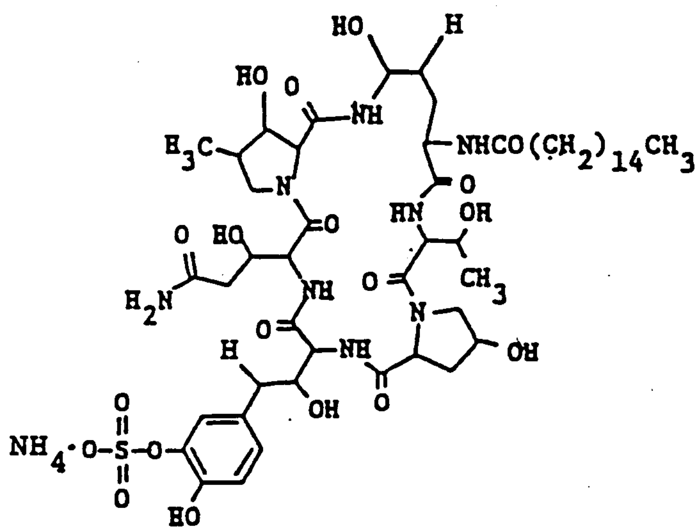

Preparation l0-l

To a solution of FR90l379 substance (60 mg) in 50 mM Tris-HCl buffer (pH 7.l, 30 ml) was added sulfatase (200 U) Type VI from Aerobacter aerogenes (SIGMA.No.S-1629). After incubating at 37°C for 30 hours, desulfonated FR90l379 substance (hereinafter referred to as FRl33302 substance) formed was extracted with a equal volume of n-butanol and washed once with water. The extract was concentrated in vacuo and applied on a column of LiChroprep RP-l8 (40-63 μm) pre-packed size B (made by Merck) equilibrated with 47% aqueous acetonitrile containing 0.5% $NH_4H_2PO_4$ and developed with the same solution. The fraction containing FRl33302 substance was diluted with the equal volume of water and directly passed through a column of ODS YMC GEL (made by Yamamura Chemical Lab.). The column was washed with water and eluted with methanol. The eluate was evaporated in vacuo to remove methanol and freeze-dried to give a white powder of FRl33302 substance (26 mg).

The FRl33302 substance as obtained has the following physico-chemical properties.

Appearance :
white powder
Nature :
neutral substance
Melting point :
2l8-222°C (dec.)
Specific rotation :
$[\alpha]_D^{22}$ -30° (C: l.0, MeOH)
Molecular formula :

$C_{51}H_{82}N_8O_{18}$

Molecular weight
HRFAB-MS 1117.5659
(Calcd. for $C_{51}H_{82}N_8O_{18}$ + Na 1117.5645)
Solubility :
soluble : methanol, ethanol
slightly soluble : water, ethyl acetate
insoluble : chloroform, n-hexane
Color reaction :
positive : iodine vapor reaction, cerium sulfate reaction
negative : Dragendorff reaction, Molish reaction
Thin layer chromatography (TLC) :

| Stationary phase | Developing solvent | Rf value |
|---|---|---|
| silica gel* | n-butanol:acetic acid: water (6:1:1) | 0.35 |

* Silica Gel 60 (made by E. Merck)

Ultraviolet absorption spectrum :

$$\lambda_{max}^{methanol} \; (E_{1cm}^{1\%}) :$$

207(353), 282(25), 232(sh) nm

$$\lambda_{max}^{methanol+0.01N-NaOH} \; (E_{1cm}^{1\%}) :$$

208(462), 246(54.5), 293(31.2)nm
Infrared absorption spectrum :

$$\nu_{max}^{KBr} :$$

3350, 2925, 2855, 1660, 1630, 1530, 1445, 1285, 1250, 1065 $cm^{-1}$
$^{1}H$ Nuclear magnetic resonance spectrum :
($CD_3OD$, 400MHz)
δ : 6.79 (1H, d, J=2Hz), 6.71 (1H, d, J=8Hz), 6.61 (1H, dd, J=8 and 2Hz), 5.25 (1H, d, J=2.5Hz), 5.06 (1H, d, J=4Hz), 4.96 (1H, d, J=3Hz), 4.60-4.20 (9H, m), 4.15 (1H, m), 4.08 (1H, m), 3.99 (1H, m), 3.91 (1H, m), 3.77 (1H, m), 3.34 (1H, m), 2.80 (1H, dd, J=15 and 3Hz), 2.54-2.40 (3H, m), 2.20 (2H, t, J=7Hz), 2.05-1.96 (3H, m), 1.56 (2H, m), 1.35-1.20 (24H, m), 1.15 (3H, d, J=6Hz), 1.02 (3H, d, J=7Hz), 0.89 (3H, t, J=7Hz)
$^{13}C$ Nuclear magnetic resonance spectrum :
($CD_3OD$, 100MHz)
δ : 177.2 (s), 175.8 (s), 174.5 (s), 173.4 (s), 172.7 (s), 172.6 (s), 172.5 (s), 169.1 (s), 146.4 (s), 146.3 (s), 133.7 (s), 120.1 (d), 116.2 (d), 115.3 (d), 76.9 (d), 75.9 (d), 75.8 (d), 74.0 (d), 71.3 (d), 70.6 (d), 70.6 (d), 70.1 (d), 68.2 (d), 62.5 (d), 58.4 (d), 57.1 (t), 56.4 (d), 55.6 (d), 53.0 (t), 51.5 (d), 39.5 (t), 39.0 (d), 38.5 (t), 36.7 (t), 34.8 (t), 33.1 (t), 30.8 (t x 5), 30.7 (t), 30.6 (t), 30.5 (t), 30.4 (t), 30.3 (t), 26.9 (t), 23.7 (t), 19.7 (q), 14.4 (q), 11.1 (q)

The chemical structure of the FR133302 substance is as follows.

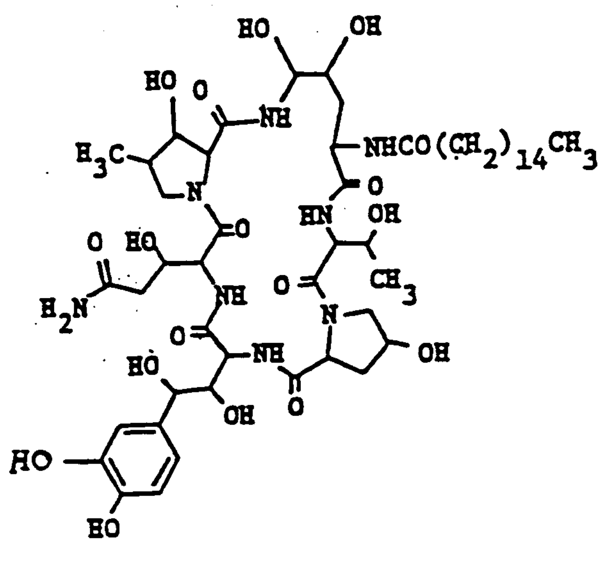

Example 1

N-acyl group of FR901379 substance was eliminated by the reaction with an enzyme. In the following, this elimination process is explained in detail.

(1) Fermentation of Actinoplanes utahensis

The enzyme which is useful for eliminating N-acyl group of FR90l379 substance is produced by certain microorganisms of the Actinoplanaceae, preferably the microorganism Actinoplanes utahensis IFO-l3244.

A stock culture of Actinoplanes utahensis IFO-l3244 is prepared and maintained on agar slant. A loopful of the slant culture was inoculated into a seed medium consisted of starch l%, sucrose l%, glucose l%, cotton seed flour l%, peptone 0.5%, soy bean meal 0.5% and $CaCO_3$ 0.l%. The inoculated vegetative medium was incubated in a 225-ml wide mouth Erlenmeyer flask at 30 ° C for about 72 hours on a rotary shaker.

This incubated vegetative medium was used directly to inoculate into a production medium consisted of sucrose 2%, peanut powder l%, $K_2HPO_4$ 0.l2% $KH_2PO_4$ 0.05% and $MgSO_4$ $7H_2O$ 0.025%. The inoculated production medium was allowed to ferment in a 30-liter jar fermentor at a temperature of 30 ° C for about 80 hours. The fermentation medium was stirred with conventional agitators at 250 rpm and aerated at 20 liters per minute. The vegetative mycelium was collected from the fermented broth by filtration and once washed with water. The washed mycelium was directly used to eliminate N-acyl group of FR90l379 substance as an enzyme source.

(2) Elimination Condition

FR90l379 substance was dissolved in 0.25 M phosphate buffer (pH 6.5) at a concentration of 0.9 mg/ml. To a 36-liter of the solution was added a 2 kg wet weight of washed mycelium of Actinoplanes utahensis IFO-l3244. The elimination reaction was carried out at 37 ° C under for 23 hours. Reduction of FR90l379 substance and increase of the deacylated FR90l379 substance(hereinafter referred to as FRl33303 substance) were measured using a HPLC equipped with a reverse phase column. From a 30 g of FR90l379 substance, a 22.2 g of FRl33303 substance was formed in the reaction mixture.

(3) Isolation of FRl33303 Substance

The reaction mixture described above was filtered with a filter aid. The mycelial cake was discarded. The filtrate thus obtained was passed through a column of activated carbon (2 L). The column was washed with 6 L of water and eluted with l2 L of 50% aqueous acetone. The eluate was evaporated in vacuo to remove acetone and then passed through a column (4 L) of YMC GEL ODS-AM l20-S50 (Yamamura Chemical Labs). The column was washed with water and eluted with 2% aqueous acetonitrile containing 50

mM $NaH_2PO_4$. Elution was monitored by analytical HPLC, using a column of LiChrospher l00 RP-l8 (Cica-MERCK) and a solvent system of 3% aqueous acetonitrile containing 0.5% $NH_4H_2PO_4$ at a flow rate of l ml/min, detecting the FRl33303 substance with a UV monitor at 2l0 nm. The fractions containing the FRl33303 substance were combined and passed through a column of activated carbon (400 ml). The column was washed with water and eluted with 50% aqueous acetone. The eluate was concentrated in vacuo to remove acetone and lyophilized to give l6.4 g of FRl33303 substance as a white powder.

FRl33303 substance has following physico-chemical properties :

Appearance :
white powder

Melting point :
l50-l60 ° C (dec.)

Specific rotation :
$[\alpha]_D^{24}$ -3l.l7 ° (C: l.0, $H_2O$)

Molecular formula :

$C_{35}H_{51}N_8SO_{20}Na$

| Elemental Analysis : | | | | |
|---|---|---|---|---|
| Calcd : for $C_{35}H_{51}N_8SO_{20}Na$<br>Found : | C 43.84,<br>C 4l.l4, | H 5.36,<br>H 5.74, | N ll.69,<br>N l0.88, | S 3.34 (%)<br>S 3.l0 (%) |

Solubility :
- soluble : water
- slightly soluble : methanol
- insoluble : n-hexane

Color reaction :
- positive : iodine vapor reaction, cerium sulfate reaction, Ninhydrin reaction
- negative : Molish reaction

Thin layer chromatography (TLC) :

| Stationary phase | Developing solvent | Rf value |
|---|---|---|
| silica gel* | n-butanol:acetic acid water (3:l:2) | 0.l5 |

* Silica Gel 60 (made by E. Merck)

Ultraviolet absorption spectrum :

$$\lambda_{max}^{H_2O} \qquad (E_{1\,cm}^{1\%}) :$$

20l(340), 273(18), 224(sh), 28l(sh) nm

$$\lambda_{max}^{H_2O+0.01N-NaOH} \qquad (E_{1\,cm}^{1\%}) :$$

207(4l4), 243(l22), 292 (34)

Infrared absorption spectrum :

$\nu_{max}^{KBr}$ :

3350, 2920, I660, I625, I5I5, I440, I270, I080, I045, 800, 755, 7I5 $cm^{-1}$

$^{1}H$ Nuclear magnetic resonance spectrum :

($D_2O$, 400MHz)

δ : 7.3I (IH, d, J = 2Hz), 7.I2 (IH, dd, J = 2Hz and 8Hz), 7.06 (IH, d, J = 8Hz), 5.40 (IH, d, J = 3Hz), 5.04 (IH, d, J = 3.5Hz), 4.94 (IH, d, J = 6Hz), 4.73-4.55 (3H, m), 4.5I-4.38 (4H, m), 4.3I-4.23 (3H, m), 4.II-4.06 (2H, m), 3.94-3.89 (2H, m), 3.4I (IH, m), 2.60-2.34 (5H, m), 2.I4 (IH, m), 2.03 (IH, m), I.28 (3H, d, J = 6Hz), I.0I (3H, d, J = 6.5Hz)

$^{13}C$ Nuclear magnetic resonance spectrum :

($D_2O$, I00MHz)

δ : I78.3 (s), I75.9 (s), I74.3 (s), I74.2 (s), I74.0 (s), I7I.8 (s), I7I.3 (s), I50.9 (s), I4I.5 (s), I34.4 (s), I28.2 (d), I24.5 (d), I20.3 (d), 78.I (d), 77.0 (d), 76.9 (d), 76.6 (d), 72.9 (d), 72.8 (d), 7I.2 (d), 69.3 (d), 69.2 (d), 63.7 (d), 60.I (d), 58.3 (t), 58.0 (d), 56.9 (d), 55.3 (d), 54.7 (t), 4I.8 (t), 39.7 (d), 39.5 (t), 33.5 (t), 2I.4 (q), I3.3 (q)

The chemical structure of FRI33303 substance has been identified and assigned as follows.

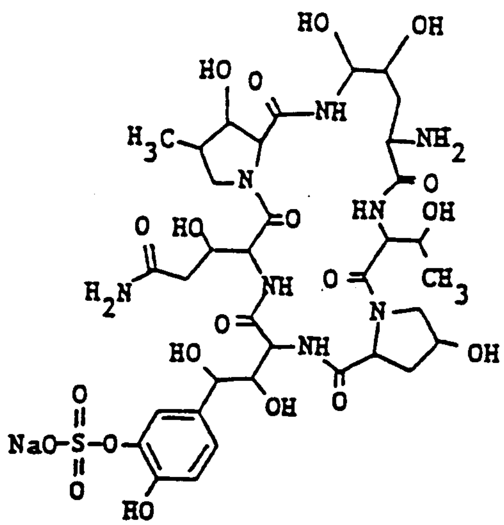

## Example 2

(1) A solution of 4-hydroxybenzoic acid (I9.2 g) in 10% NaOH (I20 ml) was dropwise added to 480 ml of dimethyl sulfoxide over 30 minutes during which the temperature in reaction mixture was controlled between 30 and 40° C. After adding, the solution was cooled to 17-20° C. I-Bromooctane (28.95 g) was dropwise added to the solution over 30 minutes and the reaction mixture was vigorously stirred for 4 hours at room temperature. The reaction mixture was poured into ice water (I200 ml) and acidified with 40 ml of conc. hydrochloric acid. After vigorously stirring for another I hour, the resulting solid was removed by filtration and dissolved in 60 ml of acetonitrile. The solution was refluxed over 30 minutes and was allowed to stand overnight at room temperature to yield 4-octyloxybenzoic acid (I3.8 g) as a crystal (MP 96° C, Anal Calcd. for $C_{15}H_{22}O_3$ : C 7I.97, H 8.86, Found : C 7I.30, H 8.89).

To a solution of 4-octyloxybenzoic acid (I3.8 g) in diethyl ether (552 ml) were added 2,4,5-trichlorophenol (I0.87 g) and N,N'-dicyclohexylcarbodiimide (II.37 g). The solution was stirred under a nitrogen atmosphere for 18 hours at room temperature. The precipitate was removed by filtration and the filtrate was concentrated in vacuo. The residue was dissolved in petroleum ether and was allowed to stand on ice-water. The resulting crystals (I5.2 g) were filtered and dissolved in warm n-hexane (I50 ml). After standing overnight at room temperature, the resulting crystal was removed by filtration. The filtrate was concentrated to an oil which was purified by a column chromatography over silica gel using a mixture of ethyl acetate and n-hexane to give 2,4,5-trichlorophenyl 4-octyloxybenzoate (7.58 g)(MP 53° C, Anal Calcd. for $C_{21}H_{23}O_3Cl_3$ : Cl 24.75, Found : Cl 24.05).

(2) To a solution of FR133303 substance (2.04 g) in N,N-dimethylformamide (60 ml) were added 2,4,5-trichlorophenyl 4-octyloxybenzoate (2.04 g) and 4-dimethylaminopyridine (0.283 g). The solution was stirred under a nitrogen atmosphere at room temperature for l5 hours. 4-Dimethylaminopyridine (0.20 g) was added to the solution and mixture was stirred for another 24 hours. The reaction mixture was poured into water (600 ml) and the pH was adjusted to 6.0. The mixture was washed twice with an equal volume of ethyl acetate and concentrated to 30 ml. The concentrate was applied on a column (l50 ml) of DEAE-Toyopearl (Cl type, manufactured by Tosoh). The column was washed with 50% aqueous methanol and developed with 50% aqueous methanol containing lM sodium chloride aqueous solution. The elution of product was assessed by the same HPLC system as described in Example 1(3) except that the concentration of acetonitrile in solvent was 40%. The fractions containing the object compound were pooled and evaporated in vacuo to remove methanol. The solution was absorbed on a column (l L) of YMC GEL ODS-AM 120-S50 in order to remove salt. The column was washed with water and eluted with 30% aqueous acetonitrile. The eluate was evaporated in vacuo to remove acetonitrile and lyophylized to give the object compound (hereinafter referred to as FRl3l535 substance) (l.4 g) as a white powder.

FRl3l535 substance has following physico-chemical properties :

Appearance :

white powder

Melting point :

l70-l89 ° C (dec.)

Specific rotation :

$[\alpha]_D^{20}$ -l4.4 ° (C: l0, $H_2O$)

Molecular formula :

$C_{50}H_{71}N_8SO_{22}Na$

| Elemental Analysis : | | | | | |
|---|---|---|---|---|---|
| Calcd : for $C_{50}H_{71}N_8SO_{22}Na \cdot 6H_2O$ | C 46.22, | H 6.44, | N 8.62, | S 2.46, | Na l.77 (%) |
| Found : | C 46.80, | H 6.l3, | N 8.78, | S l.96, | Na l.8l (%) |

Solubility :

soluble : methanol, water

slightly soluble : acetone

insoluble : n-hexane

Color reaction :

positive : iodine vapor reaction, cerium sulfate reaction

Thin layer chromatography (TLC) :

| Stationary phase | Developing solvent | Rf value |
|---|---|---|
| silica gel* | n-butanol:acetic acid: water (6:l:l) | 0.2l |

* Silica Gel 60 (made by E. Merck)

Infrared absorption spectrum :

$$\nu_{max}^{KBr} :$$

3330, 2900, 2850, l620, l500, l430, l270, l250, ll70, lll0, l080, l040, 960, 940, 880, 840, 800, 750, 7l0 $cm^{-1}$

$^1H$ Nuclear magnetic resonance spectrum :

($CD_3OD$, 200MHz)

δ : 7.78 (2H, d, J = 8Hz), 7.3l (lH d, J = 2Hz), 7.03 (lH, dd, J = 2Hz and 8Hz), 6.96 (2H, d, J = 8Hz), 6.87

(IH, d, J=8Hz), 5.33 (IH d, J=3Hz), 5.08 (IH, d, J=4Hz), 4.99 (IH, d, J=3Hz), 4.80-3.20 (I7H, m), 2.83 (IH, m), 2.65-2.30 (4H, m), 2.22-I.90 (2H, m), I.79 (2H, m), I.56-I.25 (I0H, m), I.I9 (3H, d, J=6Hz), I.06 (3H, d, J=6.5Hz), 0.90 (3H, t, J=6.5Hz)

The chemical structure of FRI3I535 substance has been identified and assigned as follows.

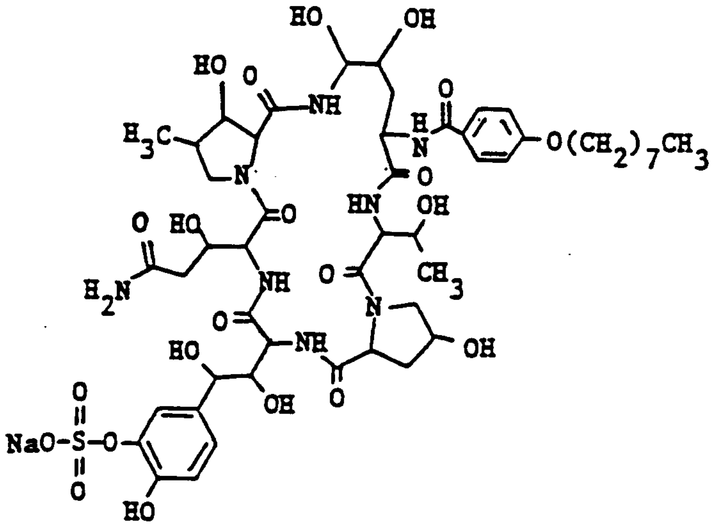

In the following, the structures of the compounds of Examples 3 to 11 are shown.

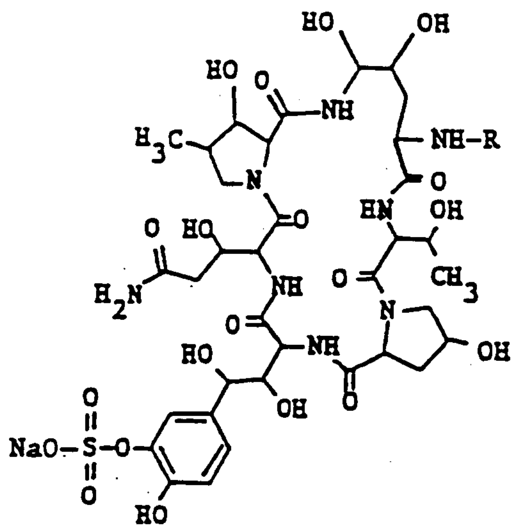

| Example No. | Compound No. | R |
|---|---|---|
| 3 | FR138260 | 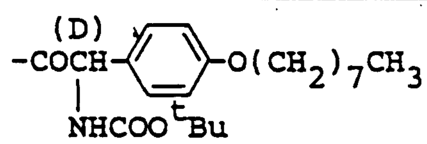 |
| 4 | FR138727 | 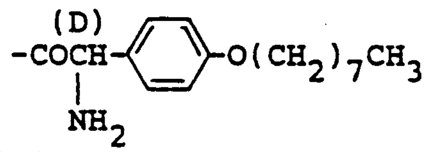 |
| 5 | FR138364 | 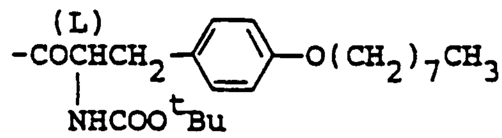 |
| 6 | FR138261 | $-COO^{t}Bu$ |
| 7 | FR138363 | $-COCH_3$ |
| 8 | FR138728 | $-COCH_2Br$ |
| 9 | FR138538 | 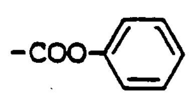 |
| 10 | FR138539 | 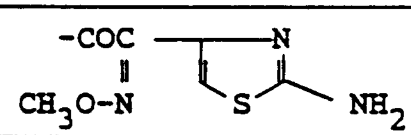 |
| 11 | FR138365 | 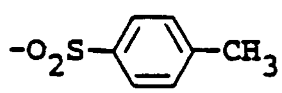 |

Example 3

To a solution of FR133303 substance (l g) and N-(t-butoxycarbonyl)-D-2-(p-octyloxyphenyl)glycine succinimido ester (0.596 g) in N,N-dimethylformamide (3 ml) was added 4-dimethylaminopyridine (0.l65g). The mixture was stirred for 12 hours at room temperature. The reaction mixture was added to water (30 ml) and then adjusted to pH 6. The aqueous solution was washed with ethyl acetate, and subjected to ion exchange chromatography on DEAE-Toyopearl ($C\ell^{\ominus}$)(60 ml) and eluted with 50% methanol in IM aqueous solution of sodium chloride. The fractions containing the object compound were combined and evaporated under reduced pressure to remove methanol. The aqueous solution was adjusted to pH 4.5 with IN

hydrochloric acid and subjected to column chromatography on Diaion HP-20 (Trademark, Manufactured by Mitsubishi Chemical Industries) (l30 ml) and eluted with 80% aqueous methanol. The fractions containing the object compound were combined and evaporated under reduced pressure to remove methanol. The residue was lyophilized to give object acylated compound (hereinafter referred to as FR138260 substance) (0.77 g).

IR (Nujol) : 3300, l660, l500, l240, l045, 800, 720 $cm^{-1}$

NMR ($CD_3OD$, δ) : 0.92 (3H, t, J = 6.8Hz), l.05 (3H, d, J = 6.8Hz), l.l7-l.33 (l3H, m), l.43 (9H, s), l.6-l.8 (2H, m), l.9-2.l (3H, m), 2.50 (3H, m), 2.75 (lH, dd, J = l6Hz and 4Hz), 3.35 (lH, m), 3.7-3.8 (lH, m), 3.93 (2H, t, J = 6.2Hz), 3.9-4.2 (5H, m), 4.3-4.5 (5H, m), 4.5-4.7 (3H, m), 4.97 (lH, d, J = 3Hz), 5.05 (lH, d, J = 4Hz), 5.ll (lH, s), 5.30 (lH, d, J = 3Hz), 6.85 (lH, d, J = 8.3Hz), 6.86 (2H, d, J = 8.6Hz), 7.02 (lH, d, J = 8.3Hz), 7.26 (2H, d, J = 8.6Hz), 7.3l (lH, s)

FAB-MS : e/z = l343 (M + Na)

Example 4

FR138260 substance obtained in Example 3 (0.25 g) was added to trifluoroacetic acid (l.25 ml) and stirred for l0 minutes. The reaction mixture was added to water (30 ml) and then adjusted to pH 4.5 with saturated aqueous solution of sodium bicarbonate. The aqueous solution was subjected to column chromatography on Diaion HP-20 (l00 ml) and eluted with 80% aqueous methanol. The fractions containing the object compound were combined and evaporated under reduced pressure to remove methanol. The residue was lyophilized to give the object compound (hereinafter referred to as FRl38727 substance) (l5 mg).

NMR ($CD_3OD$, δ) : 0.90 (3H, t, J = 6.8Hz), l.05 (3H, d, J = 6.8Hz), l.l7-l.33 (l3H, m), l.6-l.8 (2H, m), l.9-2.l (3H, m), 2.50 (lH, m), 2.75 (lH, dd, J = l6Hz and 4Hz), 3.40 (lH, m), 3.7-3.8 (lH, m), 3.98 (2H, t, J = 6.2Hz), 3.9-4.2 (5H, m), 4.3-4.5 (5H, m), 4.5-4.7 (3H, m), 4.97 (lH, d, J = 3Hz), 5.06 (lH, s), 5.20 (lH, d, J = 3Hz), 5.40 (lH, d, J = 3Hz), 6.85 (lH, d, J = 8.3Hz), 6.95 (2H, d, J = 8.5Hz), 7.02 (lH, d, J = 8.3Hz), 7.30 (lH, d, J = 8.5Hz), 7.44 (lH, s)

FAB-MS : e/z = l259 (M + K)

Example 5

FRl38364 substance was obtained by reacting FRl33303 substance with $O^4$-octyl-N-(t-butoxycarbonyl)-L-tyrosine succinimido ester according to a similar manner to that of Example 3.

IR (Nujol) : 3300, l660, l620, l240, l050 $cm^{-1}$

NMR ($CD_3OD$, δ) : 0.904 (3H, t, J = 6.8Hz), l.06 (3H, d, J = 6.8Hz), l.l7 (3H, d, J = 6.7Hz), l.20-l.30 (l0H, m), l.35 (9H, s), l.74 (2H, quintet, J = 6.5Hz), l.9-2.l (3H, m), 2.45 (3H, m), 2.76 (lH, dd, J = l6Hz and 4Hz), 3.0-3.l (2H, m), 3.37 (lH, m), 3.77 (lH, d, J = llHz), 3.92 (2H, t, J = 6.8Hz), 3.9-4.2 (7H, m), 4.3-4.5 (5H, m), 4.5-4.6 (3H, m), 4.94 (lH, d, J = 3Hz), 5.05 (lH, d, J = 3.8Hz), 5.3l (lH, d, J = 3Hz), 6.79 (2H, d, J = 8.5Hz), 6.85 (lH, d, J = 8.3Hz), 7.03 (lH, dd, J = 8.3Hz and 2Hz), 7.l2 (2H, d, J = 8.5Hz), 7.3l (lH, d, J = 2Hz)

FAB-MS : e/z = l357 (M + Na)

Example 6

A solution of FR133303 substance (0.5 g) in a mixture of water (5 ml) and tetrahydrofuran (5 ml) was adjusted to pH 7 with saturated aqueous solution of sodium bicarbonate and N,N-di-t-butylcarbonate (0.ll4 g) was added thereto at room temperature. The mixture was stirred for 5 hours at room temperature maintaining pH 7 with saturated aqueous solution of sodium bicarbonate. The reaction mixture was added to water and adjusted to pH6. The aqueous solution was washed with ethyl acetate, and subjected to ion exchange chromatography on DEAE-Toyopearl ($Cℓ^-$) (30 ml) and eluted with 50% methanol in lM aqueous solution of sodium chloride. The fractions containing the object compound were combined and evaporated under reduced pressure to remove methanol. The aqueous solution was adjusted to pH 4.5 with lN hydrochloric acid and subjected to column chromatography on Diaion HP-20 (l00 ml) and eluted with 80% aqueous methanol. The fractions containing the object compound were combined and evaporated under reduced pressure to remove methanol. The residue was lyophilized to give the object acylated compound

(hereinafter referred to as FRl3826l substance) (0.l45 g).

IR (Nujol) : 3300, l660, l620, l240, l050 $cm^{-1}$

NMR ($CD_3OD$, δ) : l.06 (3H, d, J = 6.8Hz), l.l8 (3H, d, J = 6.0Hz), l.40 (9H, s), l.9-2.l (3H, m), 2.44 (3H, m), 2.82 (lH, dd, J = l6Hz and 4Hz), 3.37 (lH, m), 3.75 (lH, d, J = llHz), 3.89-4 (2H, m), 4.l0 (lH, m), 4.l5 (lH, m), 4.29 (lH, dd, J = 6Hz and 2Hz), 4.36-4.45 (5H, m), 4.5-4.6 (3H, m), 4.97 (lH, d, J = 3Hz), 5.06 (lH, dd, J = 8.2Hz and 4Hz), 5.33 (lH, d, J = 3Hz), 6.85 (lH, d, J = 8.3Hz), 7.03 (lH, dd, J = 8.3Hz and 2Hz), 7.30 (lH, d, J = 2Hz), 7.50 (lH, d, J = 8.2Hz)

FAB-MS : e/z = l08l (M + Na)

Example 7

FR138363 substance was obtained by reacting FR133303 substance with acetyl chloride according to a similar manner to that of Example 6.

IR (Nujol) : 3300, l620, l250, l040 $cm^{-1}$

NMR ($CD_3OD$, δ) : l.06 (3H, d, J = 6.8Hz), l.20 (3H, d, J = 6Hz), l.78-2.05 (3H, m), l.96 (3H, s), 2.2l-2.54 (3H, m), 2.95 (lH, m), 3.35-3.42 (lH, m), 3.58-4.42 (llH, m), 4.50-5.05 (5H, m), 5.23 (lH, m), 6.88 (lH, d, J = 8.3Hz), 7.05 (lH, dd, J = 8.3Hz and 2Hz), 7.35 (lH, d, J = 2Hz)

FAB-MS : l023 (M + Na)

Example 8

FR138728 substance was obtained by reacting FR133303 substance with 2-bromoacetyl chloride according to a similar manner to that of Example 6.

IR (Nujol) : 3300, l660, l620, l500, l220, l040 $cm^{-1}$

NMR ($CD_3OD$, δ) : l.06 (3H, d, J = 6.9Hz), l.l7 (3H, d, J = 6.lHz), l.9-2.l (3H, m), 2.50 (32, m), 2.80 (lH, dd, J = l6Hz and 4Hz), 3.37 (lH, m), 3.6-4.0 (5H, m), 4.09 (lH, m), 4.l6 (lH, m), 4.29 (lH, dd, J = 6Hz and 2Hz), 4.36-4.45 (5H, m), 4.5-4.7 (3H, m), 4.97 (lH, d, J = 3Hz), 5.04 (lH, dd, J = 8.6Hz and 4Hz), 5.25 (lH, d, J = 3.lHz), 6.85 (lH, d, J = 8.3Hz), 7.03 (lH, dd, J = 8.3Hz and 2.lHz), 7.3l (lH, d, J = 2Hz), 7.52 (lH, d, J = 8.6Hz)

FAB-MS : e/z = ll03 (M + Na)

Example 9

FR138538 substance was obtained by reacting FR133303 substance with benzoyl chloride according to a similar manner to that of Example 6.

IR (Nujol) : 3300, l640, l240 $cm^{-1}$

NMR ($CD_3OD$, δ) : l.05 (3H, d, J = 6.8Hz), l.l8 (3H, d, J = 6Hz), l.89-2.l2 (3H, m), 2.3l-2.53 (3H, m), 2.75 (lH, dd, J = l2Hz and 4Hz), 3.38 (lH, m), 3.76 (lH, d, J = llHz), 3.87-3.98 (lH, m), 4.02-4.l8 (2H, m), 4.22-4.32 (4H, m), 4.37-4.40 (3H, m), 4.49-4.62 (3H, m), 4.98 (lH, m), 5.02 (lH, m), 5.37 (lH, d, J = 3Hz), 6.85 (lH, d, J = 8.3Hz), 7.04 (lH, dd, J = 8.3Hz and 2Hz), 7.ll-7.50 (6H, m)

FAB-MS : e/z = ll0l (M + Na)

Example 10

FR138539 substance was obtained by reacting FR133303 substance with 2-(2-aminothiazol-4-yl)-2-methoxyiminoacetic acid according to a similar manner to that of Example 6.

IR (Nujol) : 3300, l650, l620, l520, l260, l040 $cm^{-1}$

NMR ($CD_3OD$, δ) : l.05 (3H, d, J = 6.8Hz), l.2l (3H, d, J = 5.9Hz), l.89-2.2l (3H, m), 2.29-2.6l (3H, m), 2.78-2.89 (lH, m), 3.32-3.42 (lH, m), 3.76-3.82 (lH, m), 3.9l-4.0l (2H, m), 3.95 (3H, s), 4.l3 (lH, m), 4.l6 (lH, m), 4.24-4.27 (lH, m), 4.32-4.43 (5H, m), 4.46-4.62 (3H, m), 4.97-4.99 (lH, m), 5.08 (lH, m), 5.4l (lH, m), 6.79 (lH, s), 6.86 (lH, d, J = 8.lHz), 7.04 (lH, dd, J = 8.lHz and 2Hz), 7.3l (lH, d, J = 2Hz), 7.5l (lH, d, J = 7Hz)

FAB-MS : e/z = ll43 ($M^+$)

Example 11

FR138365 substance was obtained by reacting FR133303 substance with tosyl chloride according to a similar manner to that of Example 6.

IR (Nujol) : 3300, l650, l620, l260, l060 $cm^{-1}$

NMR ($CD_3OD$, δ) : 0.75 (3H, d, J=6.8Hz), l.07 (3H, d, J=6.0Hz), l.6l-l.79 (lH, m), l.9l-2.05 (3H, m), 2.30-2.59 (3H, m), 3.36 (lH, m), 3.68 (lH, d, J=llHz), 3.8l-4.07 (4H, m), 4.22 (lH, m), 4.32-4.40 (5H, m), 4.42-4.60 (3H, m), 4.7 (lH, m), 5.0 (lH, m), 5.42 (lH, d, J=3Hz), 6.85 (lH, d, J=8.3Hz), 7.03 (lH, dd, J=8.3Hz and 2Hz), 7.29-7.33 (3H, m), 7.75 (lH, d, J=8.3Hz)

FAB-MS : e/z = ll35 (M + Na)

Preparation 11

To a solution of 6-hydroxy-2-naphthoic acid (1 g) in the mixture of 10 % sodium hydroxide aqueous solution (4.25 ml) and dimethylsulfoxide (17 ml) was added octyl bromide (0.918 ml). The mixture was stirred for 6 hours at 60 °C.

The reaction mixture was added to a mixture of water and ethyl acetate and adjusted to pH 3 with conc. hydrochloric acid. The organic layer was separated and dried over magnesium sulfate. The magnesium sulfate was filtered off, and the filtrate was evaporated under reduced pressure to give 6-octyloxy-2-naphthoic acid (0.91 g).

IR (Nujol) : 1670, 1620, 1210 $cm^{-1}$

NMR (DMSO-$d_6$,δ) : 0.86 (3H, t, J=6.7 Hz), 1.2 - 1.6 (10H, m), 1.78 (2H, m), 4.10 (2H, t, J=6.7 Hz), 7.19 (1H, dd, J=2.3 and 8.8 Hz), 7.36 (1H, d, J=2.3 Hz), 7.83 (1H, d, J=8.8 Hz), 7.97 (2H, d, J=8.8 Hz), 8.52 (1H, s)

Preparation 12

1-Ethyl-3-(3-dimethylaminopropyl)carbodiimide hydrochloride (0.703 g) was added to a solution of 6-octyloxy-2-naphthoic acid (0.85 g) and 1-hydroxy-1H-benzotriazole (0.382 g) in ethyl acetate (26 ml). The mixture was stirred for two hours at room temperature.

The reaction mixture was added to water and the separated organic layer was washed with water and sodium chloride aqueous solution. Then the organic layer was dried over magnesium sulfate. The magnesium sulfate was filtered off, and the filtrate was evaporated under reduced pressure to give 1-(6-octyloxy-2-naphthoyl)-1H-benzotriazole-3-oxide (0.74 g).

IR (Nujol) : 1770, 1740, 1620, 1190, 1020, 740 $cm^{-1}$

NMR ($CDCl_3$, δ) : 0.90 (3H, t, J=6.8 Hz), 1.2 - 1.6 (10H, m), 1.89 (2H, m), 4.14 (2H, t, J=6.8 Hz), 7.1 - 7.3 (2H, m), 7.4 - 7.6 (3H, m), 7.8 - 8.0 (2H, m), 8.1 - 8.2 (2H, m), 8.80 (1H, s)

In the following, the structure of the compound of Example 12 is shown.

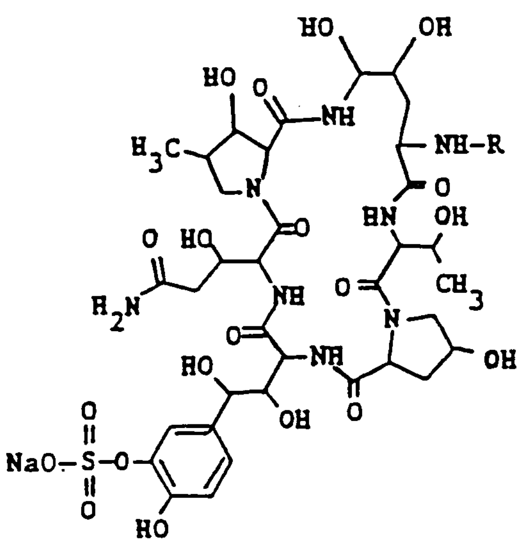

| Example No. | Compound No. | R |
|---|---|---|
| 12 | FR139687 | 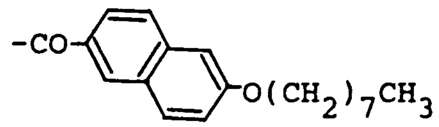 |

<u>Example 12</u>

To a solution of FR133303 substance (0.5 g) and 1-(6-octyloxy-2-naphthoyl)-1H-benzotriazole-3-oxide (0.271 g) in N,N-dimethylformamide (1.5 ml) was added 4-dimethylaminopyridine (0.0828 g). The mixture was stirred for 12 hours at room temperature.

The reaction mixture was added to water and adjusted to pH 6. The aqueous solution was washed with ethyl acetate, and subjected to ion exchange chromatography on DEAE-Toyopearl ($Cl^-$) (30 ml) and eluted with 50 % methanol in 1M sodium chloride solution . The fractions containing the object compound were combined and evaporated under reduced pressure to remove methanol. The aqueous solution was adjusted to pH 4.5 with 1N hydrochloric acid and subjected to column chromatography on Diaion HP-20 (65 ml) and eluted with 80 % aqueous methanol. The fractions containing the object compound were combined and evaporated under reduced pressure to remove methanol. The residue was lyophilized to give object acylated compound (hereinafter referred to as FR139687 substance) (0.214 g).

IR (Nujol) : 3300, 1620, 1500 $cm^{-1}$

NMR (DMSO-$d_6$ + $D_2O$, $\delta$) : 0.86 (3H, t, J=6.8 Hz), 0.97 (3K, d, J=6.8 Hz), 1.06 (3H, d, J=6.8 Hz), 1.2 - 1.5 (10H, m), 1.6 - 2.0 (5H, m), 2.2 - 2.5 (3H, m), 2.4 - 2.6 (1H, m), 3.18 (1H, m), 3.6 - 3.9 (1H, m), 4.0 - 4.6 (15H, m), 4.84 (1H, d, J=3 Hz), 4.90 (1H, d, J=3 Hz), 5.11 (1H, d, J=3 Hz), 6.76 (1H, d, J=8.3 Hz), 6.93 (1H, d, J=8.3 Hz), 7.13 (1H, s), 7.25 (1H, d, J=8.3 Hz), 7.39 (1H, s), 7.8 - 8.0 (3H, m), 8.44 (1H, s)

FAB-MS e/z=1264 (M+Na)

The following compounds (<u>Preparations 13 to 16</u>) were obtained according to a similar manner to that of <u>Preparation 5</u>.

<u>Preparation 13</u>

N-(t-Butoxycarbonyl)-L-2-(2-naphthyl)glycine succinimido ester

IR (Nujol) : 3350, 1800, 1770, 1730, 1680, 1500, 1200 $cm^{-1}$

<u>Preparation 14</u>

Succinimido 2-(4-biphenylyl)acetate

IR (Nujol) : 1800, 1770, 1720, 1200 $cm^{-1}$

NMR (DMSO-$d_6$, $\delta$) : 2.82 (4H, s), 4.17 (2H, s), 7.30-7.50 (5H, m), 7.45 (2H, d, J=8.1Hz), 7.67 (2H, d, J=8.1Hz)

<u>Preparation 15</u>

Succinimido 4-t-butylbenzoate

IR (Nujol) : 1760, 1730, 1200, 1070, 990 $cm^{-1}$

NMR (DMSO-$d_6$, $\delta$) : 1.33 (9H, s), 2.89 (4H, s), 7.68 (2H, d, J=8.5Hz), 8.03 (2H, d, J=8.5Hz)

Preparation 16

Succinimido 4-(4-phenylbutoxy)benzoate

IR (Nujol) : l730, l600, l240, ll70, l070 cm$^{-1}$

NMR (DMSO-$d_6$, δ) : l.75 (4H, m), 2.65 (2H, m), 4.l4 (2H, m), 7.l5 (2H, d, J=8.9Hz), 7.l3-7.35 (5H, m), 8.03 (2H, d, J=8.9Hz)

Preparation 17

To neat 3,7-dimethyloctanol (5 ml) was added phosphorus tribromide (l.0l ml). The mixture was stirred for 4 hours at 60 °C. The reaction mixture was added to a mixture of water and n-hexane. The organic layer was separated and dried over magnesium sulfate. The magnesium sulfate was filtered off, and the filtrate was evaporated under reduced pressure to give 3,7-dimethyloctyl bromide (4.40 g).

IR (Neat) : 2900, l450 cm$^{-1}$

NMR ($CDCl_3$, δ) : 0.87 (6H, d, J=6.6Hz), 0.89 (3H, d, J=6.4Hz), l.l-l.3 (6H, m), l.5-l.9 (4H, m), 3.3-3.5 (2H, m)

The following compounds (Preparations 18 to 23) were obtained according to a similar manner to that of Preparation 11.

Preparation 18

4-[4-(Octyloxy)phenoxy]benzoic acid

IR (Nujol) : l680, l600, l240, 840 cm$^{-1}$

NMR (DMSO-$d_6$, δ) : 0.87 (3H, t, J=6.7Hz), l.l-l.6 (l0H, m), l.7l (2H, m), 3.96 (2H, t, J=6.4Hz), 6.9-7.l (6H, m), 7.92 (2H, d, J=8.7Hz), l2.8 (lH, br s)

Preparation 19

6-(Butoxy)-2-naphthoic acid

IR (Nujol) : l660, l6l0, l205 cm$^{-1}$

NMR (DMSO-$d_6$, δ) : 0.96 (3H, t, J=7.29Hz), l.48 (2H, qt, J=7.29Hz and 7Hz), l.78 (2H, tt, J=7Hz and 6.45Hz), 4.l2 (2H, t, J=6.45Hz), 7.24 (lH, dd, J=9.0Hz and 2.3Hz), 7.40 (lH, d, J=2.3Hz), 7.86 (lH, d, J=8.7Hz), 7.94 (lH, d, J=8.7Hz), 8.0l (lH, d, J=9.0Hz), 8.52 (lH, s)

Preparation 20

6-Decyloxy-2-naphthoic acid

IR (Nujol) : l670, l620, l2l0 cm$^{-1}$

NMR (DMSO-$d_6$, δ) : 0.85 (3H, t, J=6.7Hz), l.2-l.6 (l4H, m), l.78 (2H, m), 4.ll (2H, t, J=6.4Hz), 7.23 (lH, dd, J=8.9Hz and 2.4Hz), 7.39 (lH, d, J=2.4Hz), 7.86 (lH, d, J=8.7Hz), 7.93 (lH, d, J=8.7Hz), 8.0l (lH, d, J=8.9Hz), 8.5 (lH, s)

Preparation 21

6-Hexyloxy-2-naphthoic acid

IR (Nujol) : l660, l620, l290, l2l0 cm$^{-1}$

NMR (DMSO-$d_6$, δ) : 0.89 (3H, t, J=6.8Hz), l.2-l.6 (6H, m), l.78 (2H, quint, J=6.5Hz), 4.ll (2H, t, J=6.5Hz), 7.23 (lH, dd, J=9.0Hz and 2.4Hz), 7.39 (lH, d, J=2.4Hz), 7.86 (lH, d, J=8.7Hz), 7.94 (lH, d, J=8.7Hz), 8.0l (lH, d, J=9.0Hz), 8.52 (lH, s)

Preparation 22

6-Dodecyloxy-2-naphthoic acid

IR (Nujol) : l670, l620, l2l0 cm$^{-1}$

NMR (DMSO-$d_6$, δ) : 0.85 (3H, t, J=6.7Hz), l.20-l.60 (l8H, m), l.78 (2H, m), 4.ll (2H, t, J=6.5Hz), 7.22 (lH, dd, J=9.0Hz and 2.4Hz), 7.39 (lH, d, J=2.4Hz), 7.85 (lH, d, J=8.7Hz), 7.93 (lH, d, J=8.7Hz), 8.00 (lH, d, J=9.0Hz), 8.5l (lH, s), l2.90 (lH, s)

Preparation 23

6-(3,7-Dimethyloctyloxy)-2-naphthoic acid

IR (Nujol) : l660, l6l0, l290, l2l0 $cm^{-1}$

NMR ($DMSO-d_6$, $\delta$) : 0.84 (6H, d, J = 6.6Hz), 0.94 (3H, d, J = 6.lHz), l.l-l.4 (6H, m), l.4-l.9 (4H, m), 4.15 (2H, t, J = 6.7Hz), 7.22 (lH, dd, J = 9.0Hz and 2.4Hz), 7.4l (lH, d, J = 2.4Hz), 7.86 (lH, d, J = 8.6Hz), 7.93 (lH, d, J = 8.6Hz), 8.0l (lH, d, J = 9.0Hz), 8.52 (lH, s)

The following compounds (Preparations 24 to 3l) were obtained according to a similar manner to that of Preparation 12.

Preparation 24

1-[4-(4-Octyloxy)phenoxy]benzoyl-1H-benzotriazole-3-oxide

IR (Nujol) : l770, l730, l600, l500, l230, 980 $cm^{-1}$

Preparation 25

l-(6-Butoxy-2-naphthoyl)-1H-benzotriazole-3-oxide

IR (Nujol) : l760, l6l0, l260, ll80, l020 $cm^{-1}$

Preparation 26

1-(6-Decyloxy-2-naphthoyl)-1H-benzotriazole-3-oxide

IR (Nujol) : l780, l620, ll90, l000 $cm^{-1}$

Preparation 27

1-(6-Hexyloxy-2-naphthoyl)-1H-benzotriazole-3-oxide

IR (Nujol) : l780, l6l0, ll90 $cm^{-1}$

NMR ($DMSO-d_6$, $\delta$) : 0.89 (3H, t, J = 6.7Hz), l.2-l.6 (6H, m), l.79 (2H, m), 4.l2 (2H, t, J = 6.5Hz), 7.24 (lH, dd, J = 9.0Hz and 2.4Hz), 7.39 (lH, d, J = 2.4Hz), 7.4l (lH, t, J = 8Hz), 7.54 (lH, t, J = 8Hz), 7.72 (lH, d, J = 8Hz), 7.88 (lH, d, J = 8.7Hz), 7.90 (lH, d, J = 8.7Hz), 7.97 (lH, d, J = 8Hz), 8.02 (lH, d, J = 9.0Hz), 8.5l (lH, s)

Preparation 28

l-(6-Dodecyloxy-2-naphthoyl)-1H-benzotriazole-3-oxide

IR (Nujol) : l770, l620, ll90, l030, 730 $cm^{-1}$

NMR ($DMSO-d_6$, $\delta$) : 0.85 (3H, t, J = 6.7Hz), l.2-l.3 (l8H, m), l.78 (2H, m), 4.ll (2H, t, J = 6.5Hz), 7.22 (lH, dd, J = 9.0Hz and 2.4Hz), 7.39 (lH, d, J = 2.4Hz), 7.40 (lH, t, J = 8Hz), 7.55 (lH, t, J = 8Hz), 7.73 (lH, d, J = 8Hz), 7.85 (lH, d, 3 = 8.7Hz), 7.93 (lH, d, J = 8.7Hz), 7.99 (lH, d, J = 8Hz), 8.00 (lH, d, J = 9.0Hz), 8.5l (lH, s)

Preparation 29

1-[6-(3,7-Dimethyloctyloxy)-2-naphthoyl]-1H-benzotriazole-3-oxide

IR (Nujol) : 1780, 1620, 1190 $cm^{-1}$

Preparation 30

1-[(2E,6E)-3,7,11-Trimethyl-2,6,10-dodecatrienoyl]-1H-benzotriazole-3-oxide

IR (Neat) : 2900, l780, l620, l420, l070 $cm^{-1}$

Preparation 31

3,7-Dimethyl-6-octenyl bromide was obtained according to a similar manner to that of Preparation 17.

IR (Neat) : 2900, l440, l380 $cm^{-1}$

NMR (DMSO-$d_6$, δ) : 0.86 (3H, d, J=6.3Hz), l.0-l.5 (2H, m), l.57 (3H, s), l.65 (3H, s), l.7-2.l (5H, m), 3.4-3.7 (2H, m), 5.08 (lH, m)

Preparation 32

To a suspension of sodium hydride (2.04 g) in N,N-dimethylformamide (50 ml) was added 4-hydroxypyridine (5 g) at room temperature. Octyl bromide (9.08 ml) was added thereto. The mixture was stirred for 2 hours at 50°C. The reaction mixture was added to a mixture of brine (l00 ml), trtrahydrofuran (100 ml) and ethyl acetate (100 ml). The organic layer was separated and dried over magnesium sulfate. The magnesium sulfate was filtered off, and the filtrate was evaporated under reduced pressure to give l-octyl-4-pyridone (l4.7 g).

NMR (DMSO-$d_6$, δ) : 0.86 (3H, t, J=6Hz), l.l-l.4 (l0H, m), l.4-l.8 (2H, m), 3.8l (2H, t, J=7Hz), 6.05 (2H, d, J=8Hz), 7.63 (2H, d, J=8Hz)

Preparation 33

To a solution of l-octyl-4-pyridone (l0.9 g) in pyridine (l00 ml) was added phosphorous pentasulfide (8.65 g) at room temperature. The mixture was stirred for 3 hours at 80°C. The reaction mixture was added to a mixture of water (200 ml) and methylene chloride (200 ml). The organic layer was separated and dried over magnesium sulfate. The magnesium sulfate was filtered off, and the filtrate was evaporated under reduced pressure to give l-octyl-l,4-dihydropyridine-4-thione (5.27 g).

IR (Neat) : 29l0, 2850, l620, l460, lll0 $cm^{-1}$

NMR (DMSO-$d_6$, δ) : 0.86 (3H, t, J=6Hz), l.l-l.4 (l0H, m), l.5-l.9 (2H, m), 3.95 (2H, t, J=7Hz), 7.l3 (2H, d, J=7Hz), 7.60 (2H, d, J=7Hz)

The following compounds (Preparations 34 to 36) were obtained according to a similar manner to that of Preparation 1.

Preparation 34

Methyl 2-(4-hydroxyphenyl)-2-methoxyacetate

IR (Nujol) : 3350, l740, l6l0, l600, l220, ll00 $cm^{-1}$

NMR (DMSO-$d_6$, δ) : 3.23 (3H, s), 3.60 (3H, s), 4.73 (lH, s), 6.72 (2H, d, J=8.9Hz), 7.l5 (2H, d, J=8.9Hz)

EI-MS (e/z) = l96 ($M^+$)

Preparation 35

D-Tyrosine methyl ester hydrochloride

IR (Nujol) : 3300, l740, l220 $cm^{-1}$

NMR (DMSO-$d_6$, δ) : 3.02 (2H, m), 3.67 (3H, s), 4.l6 (lH, t, J=6.7Hz), 6.72 (2H, d, J=8.4Hz), 7.0l (2H, d, J=8.4Hz), 8.58 (2H, s), 9.47 (lH, s)

Preparation 36

Methyl (4-hydroxyphenyl)glyoxylate

IR (Nujol) : 3380, l730, l700, l600, l580, l220 $cm^{-1}$

NMR (DMSO-$d_6$, δ) : 3.9l (3H, s), 6.94 (2H, d, J=8.8Hz), 7.83 (2H, d, J=8.8Hz), l0.9 (lH, s)

Preparation 37

N-(t-Butoxycarbonyl)-D-tyrosine methyl ester was obtained according to a similar manner to that of Preparation 2.

IR (Nujol) : 3360, 1700, 1680, 1290, 1270, 1250 $cm^{-1}$

NMR (DMSO-$d_6$, δ) : 1.33 (9H, s), 2.73 (2H, m), 3.59 (3H, s), 4.05 (1H, m), 6.65 (2H, d, J=8.4Hz), 7.00 (2H, d, J=8.4Hz), 7.23 (1H, d, J=7.9Hz), 9.23 (1H, s)

Preparation 38

To a solution of L-tyrosine methyl ester hydrochloride (1 g) in water (1.5 ml) was added sodium bicarbonate (0.363 g) under ice-cooling and stirred for 10 minutes, and then acetonitrile (7 ml), 37% formaldehyde aqueous solution (0.637 ml) and sodium cyanoborohydride (0.182 g) was added thereto at -5°C. The mixture was stirred for 2 hours at -5°C. The resultant insoluble material was filtered off, and the filtrate was extracted with ethyl acetate. The organic layer was separated and dried over magnesium sulfate. The magnesium sulfate was filtered off, and the filtrate was evaporated under reduced pressure to give N,N-dimethyl-L-tyrosine methyl ester (0.21 g).

IR (Nujol) : 1730, 1260, 1010 $cm^{-1}$

NMR (DMSO-$d_6$, δ) : 2.24 (6H, s), 2.72 (2H, m), 3.34 (1H, m), 3.53 (3H, s), 6.64 (2H, d, J=8.4Hz), 6.97 (2H, d, J=8.4Hz), 9.18 (1H, s)

The following compounds (Preparations 39 to 44) were obtained according to a similar manner to that of Preparation 3.

Preparation 39

Methyl 2-(4-octyloxyphenyl)acetate

IR (Neat) : 2910, 2850, 1730, 1240 $cm^{-1}$

NMR (DMSO-$d_6$, δ) : 0.86 (3H, t, J=6.3Hz), 1.2-1.5 (10H, m), 1.6-1.9 (2H, m), 3.58 (2H, s), 3.59 (3H, s), 3.92 (2H, t, J=6.4Hz), 6.85 (2H, d, J=8.7Hz), 7.15 (2H, d, J=8.7Hz)

Preparation 40

Ethyl 3-(4-octyloxyphenyl)propionate

IR (Neat) : 2920, 2850, 1730, 1240 $cm^{-1}$

NMR (DMSO-$d_6$, δ) : 0.86 (3H, t, J=6.7Hz), 1.15 (3H, t, J=7.1Hz), 1.2-1.5 (10H, m), 1.6-1.8 (2H, m), 2.55 (2H, t, J=7.2Hz), 2.77 (2H, t, J=7.2Hz), 3.90 (2H, t, J=6.4Hz), 4.03 (2H, q, J=7.1Hz), 6.81 (2H, d, J=8.6Hz), 7.11 (2H, d, J=8.6Hz)

Preparation 41

Methyl 2-(4-octyloxyphenyl)-2-methoxyacetate

IR (Neat) : 2910, 2850, 1740, 1600, 1240, 1100 $cm^{-1}$

NMR (DMSO-$d_6$, δ) : 0.86 (3H, t, J=6.8Hz), 1.2-1.5 (10H, m), 1.6-1.8 (2H, m), 3.26 (3H, s), 3.62 (3H, s), 3.94 (2H, t, J=6.4Hz), 4.83 (1H, s), 6.91 (2H, d, J=8.7Hz), 7.27 (2H, d, J=8.7Hz)

EI-MS (e/z) = 308 ($M^+$)

Preparation 42

$O^4$-Octyl-N-(t-butoxycarbonyl)-D-tyrosine methyl ester

IR (Nujol) : 3350, 1730, 1680, 1510, 1240, 1160 $cm^{-1}$

NMR (DMSO-$d_6$, δ) : 0.86 (3H, t, J=6.7Hz), 1.2-1.3 (10H, m), 1.68 (2H, m), 2.82 (2H, m), 3.60 (3H, s), 3.91 (2H, t, J=7.3Hz), 4.08 (1H, m), 6.81 (2H, d, J=8.6Hz), 7.12 (2H, d, J=8.6Hz), 7.25 (1H, d, J=8.0Hz)

Preparation 43

$O^4$-Octyl-N,N-dimethyl-L-tyrosine methyl ester

IR (Neat) : 2930, 2860, l730, l250 $cm^{-1}$

NMR ($DMSO-d_6$, $\delta$) : 0.86 (3H, t, J = 6.6Hz), l.26 (l0H, m), l.68 (2H, m), 2.80 (2H, m), 3.33 (6H, s), 3.37 (lH, m), 3.53 (3H, s), 3.89 (2H, t, J = 6.4Hz), 6.79 (2H, d, J = 8.6Hz), 7.08 (2H, d, J = 8.6Hz)

Preparation 44

Methyl (4-octyloxyphenyl)glyoxylate

IR (Neat) : 2930, 2850, l730, l670, l600, l260, l2l0, ll60 $cm^{-1}$

NMR ($DMSO-d_6$, $\delta$) : 0.86 (3H, t, J = 6.3Hz), l.2-l.5 (l0H, m), l.6-l.9 (2H, m), 3.93 (3H, s), 4.l0 (2H, t, J = 6.5Hz), 7.l2 (2H, d, J = 8.9Hz), 7.92 (2H, d, J = 8.9Hz)

The following compounds (Preparations 45 to 51) were obtained according to a similar manner to that of Preparation 4.

Preparation 45

4-(2-Butoxyethoxy)benzoic acid

IR (Nujol) : l670, l6l0, l260 $cm^{-1}$

NMR ($DMSO-d_6$, $\delta$) : 0.87 (3H, t, J = 7.2Hz), l.2-l.6 (4H, m), 3.45 (2H, t, J = 6.4Hz), 3.70 (2H, t, 3 = 4.4Hz), 4.l6 (2H, t, J = 4.4Hz), 7.02 (2H, d, J = 8.9Hz), 7.88 (2H, d, J = 8.9Hz), l2.63 (lH, s)

Preparation 46

2-(4-Octyloxyphenyl)acetic acid

IR (Nujol) : l680, l240, 820, 780 $cm^{-1}$

NMR ($DMSO-d_6$, $\delta$) : 0.86 (3H, t, J = 6.8Hz), l.l-l.5 (l0H, m), l.6-l.8 (2H, m), 3.47 (2H, s), 3.92 (2H, t, J = 6.4Hz), 6.84 (2H, d, J = 8.6Hz), 7.l4 (2H, d, J = 8.6Hz)

Preparation 47

3-(4-Octyloxyphenyl)propionic acid

IR (Nujol) : l680, l500, l200 $cm^{-1}$

NMR ($DMSO-d_6$, $\delta$) : 0.86 (3H, t, J = 6.3Hz), l.l-l.5 (l0H, m), l.6-l.8 (2H, m), 2.47 (2H, t, J = 7.2Hz), 2.74 (2H, t, J = 7.2Hz), 3.90 (2H, t, J = 6.4Hz), 6.8l (2H, d, J = 8.6Hz), 7.ll (2H, d, J = 8.6Hz), l2.l0 (lH, br s)

Preparation 48

2-(4-Octyloxyphenyl)-2-methoxyacetic acid

IR (Nujol) : l760, l720, l600, l500, l240, ll80, ll00, 830 $cm^{-1}$

NMR ($DMSO-d_6$, $\delta$) : 0.86 (3H, t, J = 6.7Hz), l.2-l.5 (l0H, m), 2.6-2.8 (2H, m), 3.26 (3H, s), 3.94 (2H, t, J = 6.4Hz), 4.67 (lH, s), 6.90 (2H, d, J = 8.6Hz), 7.27 (2H, d, J = 8.6Hz)

Preparation 49

$O^4$-Octyl-N-(t-butoxycarbonyl)-D-tyrosine

IR (Nujol) : 3400-2900, l700, l500, l240, ll60 $cm^{-1}$

NMR ($DMSO-d_6$, $\delta$) : 0.859 (3H, t, J = 6.8Hz), l.20-l.30 (l0H, m), l.32 (9H, s), l.68 (2H, m), 2.67-2.95 (lH, m), 3.90 (2H, t, J = 7Hz), 4.0l (lH, m), 6.8l (2H, d, J = 8.6Hz), 7.02 (lH, d, J = 8.3Hz), 7.l3 (2H, d, J = 8.6Hz)

Preparation 50

$O^4$-Octyl-N,N-dimethyl-L-tyrosine
IR (Neat) : 2940, 2860, 2600, 1620, 1240 $cm^{-1}$
NMR (DMSO-$d_6$, $\delta$) : 0.86 (3H, t, J = 6.6Hz), 1.26 (10H, m), 1.68 (2H, m), 2.67 (6H, s), 2.8-3.6 (3H, m), 3.91 (2H, t, J = 6.4Hz), 6.85 (2H, d, J = 8.5Hz), 7.16 (2H, d, J = 8.5Hz)

Preparation 51

$O^4$-Octyloxyphenyl)glyoxylic acid
IR (Neat) : 1730, 1670, 1600, 1260, 1160 $cm^{-1}$
NMR (DMSO-$d_6$, $\delta$) : 0.86 (3H, t, J = 6.8Hz), 1.2-1.5 (10H, m), 1.65-1.85 (2H, m), 4.09 (2H, t, J = 6.5Hz), 7.12 (2H, d, J = 8.9Hz), 7.89 (2H, d, J = 8.9Hz)

Preparation 52

$N^{\tau}$-Octyl-N-(t-butoxycarbonyl)-L-histidine was obtained from N-(t-butoxycarbonyl)-L-histidine methyl ester according to similar manners to those of Preparations 3 and 4.
NMR (DMSO-$d_6$, $\delta$) : 0.85 (3H, t, J = 6.3Hz), 1.23 (10H, m), 1.35 (9H, s), 2.83 (2H, m), 3.90 (2H, t, J = 7Hz), 4.0-4.2 (1H, m), 6.36 (1H, s), 7.02 (1H, d, J = 8Hz), 7.75 (1H, s)

The following compounds (Preparations 53 to 60) were obtained according to a similar manner to that of Preparation 11.

Preparation 53

4-Octyloxyphthalic acid
IR (Neat) : 2930, 2860, 2500, 1700, 1600, 1260 $cm^{-1}$
NMR (DMSO-$d_6$, $\delta$) : 0.86 (3H, t, J = 6.8Hz), 1.2-1.5 (10H, m), 1.5-1.8 (2H, m), 4.05 (2H, t, J = 6.2Hz), 7.03 (1H, d, J = 2.6Hz), 7.06 (1H, dd, J = 8.4Hz and 2.6Hz), 7.72 (1H, d, J = 8.4Hz)

Preparation 54

3-Methoxy-4-octyloxybenzoic acid
IR (Nujol) : 2600, 1680, 1600, 1270, 1230 $cm^{-1}$
NMR (DMSO-$d_6$, $\delta$) : 0.86 (3H, t, J = 6.8Hz), 1.2-1.5 (10H, m), 1.6-1.8 (2H, m), 3.80 (3H, s), 4.01 (2H, t, J = 6.5Hz), 7.03 (1H, d, J = 8.5Hz), 7.44 (1H, d, J = 1.9Hz), 7.54 (1H, dd, J = 8.5Hz and 1.9Hz)

Preparation 55

4-(4-Octyloxyphenyl)benzoic acid
IR (Nujol) : 1670, 1600, 830, 770 $cm^{-1}$
NMR (DMSO-$d_6$, $\delta$) : 0.87 (3H, t, J = 6.7Hz), 1.2-1.5 (10H, m), 1.6-1.8 (2H, m), 4.01 (2H, t, J = 6.4Hz), 7.04 (2H, d, J = 8.8Hz), 7.68 (2H, d, J = 8.8Hz), 7.75 (2H, d, J = 8.5Hz), 7.99 (2H, d, J = 8.5Hz)

Preparation 56

6-(2-Ethylhexyloxy)-2-naphthoic acid
IR (Nujol) : 1660, 1610, 1280, 1200 $cm^{-1}$
NMR (DMSO-$d_6$, $\delta$) : 0.88 (3H, t, J = 7.3Hz), 0.92 (3H, t, J = 7.3Hz), 1.2-1.6 (8H, m), 1.7-1.9 (1H, m), 4.01 (2H, d, J = 5.7Hz), 7.23 (1H, dd, J = 8.9 and 2.4Hz), 7.42 (1H, d, J = 2.4Hz), 7.86 (1H, d, J = 8.7Hz), 7.94 (1H, d, J = 8.7Hz), 8.01 (1H, d, J = 8.9Hz), 8.51 (1H, s), 12.9 (1H, s)

Preparation 57

6-(3,7-Dimethyl-6-octenyloxy)naphthoic acid

IR (Nujol) : l660, l6l0, l290, l200 $cm^{-1}$

NMR ($DMSo-d_6$, δ) : 0.95 (3H, d, J = 6.lHz), l.l-l.5 (2H, m), l.57 (3H, s), l.64 (3H, s), l.6-2.l (5H, m), 4.l5 (2H, t, J = 6.7Hz), 5.l0 (lH, t, 3 = 7.lHz), 7.22 (lH, dd, J = 8.9Hz and 2.3Hz), 7.42 - (lH, d, J = 2.3Hz), 7.86 (lH, d, J = 8.6Hz), 7.94 (lH, d, J = 8.6Hz), 8.0l (lH, d, J = 8.9Hz), 8.52 (lH, S), l2.89 (lH, S)

Preparation 58

6-(3,7-Dimethyl-2,6-octadienyloxy)naphthoic acid

IR (Nujol) : l660, l620, l2l0 $cm^{-1}$

NMR ($DMSO-d_6$, δ) : l.57 (3H, s), l.60 (3H, s), l.76 (3H, s), 2.07 (4H, m), 4.70 (2H, d, J = 6.5Hz), 5.07 (lH, m), 5.5l (lH, t, J = 6.5Hz), 7.24 (lH, dd, J = 8.9Hz and 2.4Hz), 7.4l (lH, d, J = 2.4Hz), 7.85 (lH, d, J = 8.7Hz), 7.94 (lH, d, J = 8.7Hz), 8.0l (lH, d, J = 8.9Hz), 8.52 (lH, s), l2.88 (lH, s)

Preparation 59

(2E)-3-(4-Octyloxyphenyl)acrylic acid

IR (Nujol) : l660, l600, l240 $cm^{-1}$

NMR ($DMSO-d_6$, δ) : 0.86 (3H, t, J = 6.7Hz), l.2-l.5 (l0H, m), l.6-l.8 (2H, m), 4.00 (2H, t, J = 6.4Hz), 6.36 (lH, d, J = l6Hz), 6.95 (2H, d, J = 8.7Hz), 7.54 (lH, d, J = l6Hz), 7.6l (2H, d, J = 8.7Hz), l2.20 (lH, br s)

Preparation 60

Sodium 6-octyloxy-2-naphthalene sulfonate

IR (Nujol) : l230, ll80, 860, 820 $cm^{-1}$

NMR ($DMSO-d_6$, δ) : 0.86 (3H, t, J = 6Hz), l.l-l.6 (l0H, m), 4.06 (2H, t, J = 5Hz), 7.08 (lH, d, J = 9Hz), 7.2l (lH, s), 7.79 (lH, d, J = 9Hz), 8.00 (lH, s)

Preparation 6l

To a solution of thionyl chloride (0.692 ml) and N,N-dimethylformamide (0.022 ml) was added sodium 6-octyloxy-2-naphthalenesulfonate (l g) under ice-cooling and stirred for l.5 hours at 95 °C. The reaction mixture was evaporated under reduced pressure to give 6-octyloxy-2-naphthylsulfonyl chloride (l g).

IR (Nujol) : l6l0, l260, ll60 $cm^{-1}$

NMR ($CDCl_3$, δ) : 0.90 (3H, t, J = 6.2Hz), l.2-l.7 (l0H, m), l.8-2.0 (2H, m), 4.l2 (2H, t, J = 6.5Hz), 7.20 (lH, d, J = 2.2Hz), 7.32 (lH, dd, J = 9.0Hz and 2.2Hz), 7.84-7.97 (3H, m), 8.49 (lH, s)

The following compounds (Preparations 62 to 7l) were obtained according to a similar manner to that of Preparation 12.

Preparation 62

l-(4-Octylbenzoyl)-lH-benzotriazole-3-oxide

IR (Neat) : 2930, 2850, l780, l6l0, l240, 990 $cm^{-1}$

Preparation 63

l-[4-(4-Octyloxyphenyl)benzoyl]-lH-benzotriazole-3-oxide

IR (Nujol) : 1770, 1600, 980 $cm^{-1}$

Preparation 64

1-[6-(2-Ethylhexyloxy)-2-naphthoyl]-1H-benzotriazole-3-oxide

IR (Nujol) : l770, l620, l270, ll80 $cm^{-1}$

NMR ($CDCl_3$, δ) : 0.93 (3H, t, J=7.lHz), 0.98 (3H, t, J=7.4Hz), l.3-l.7 (8H, m), l.7-2.0 (lH, m), 4.03 (2H, d, J=5.7Hz), 7.22 (lH, d, J=2.2Hz), 7.29 (lH, dd, J=8.9Hz, 2.2Hz), 7.4-7.7 (3H, m), 7.87 (lH, d, J=9.5Hz), 7.92 (lH, d, J=9.5Hz), 8.l-8.2 (2H, m), 8.80 (lH, s)

Preparation 65

1-[6-(3,7-Dimethyl-6-octenyloxy)-2-naphthoyl]-lH-benzotriazole-3-oxide

IR (Neat) : 2900, l770, l620, ll80 $cm^{-1}$

Preparation 66

1-[6-{(E)-3,7-Dimethyl-2,6-octadienyloxy}-2-naphthoyl]-lH-benzotriazole-3-oxide

IR (Nujol) : l770, l620, l270, ll80 $cm^{-1}$

Preparation 67

1-(2-Anthrylcarbonyl)-lH-benzotriazole-3-oxide

IR (Nujol) : l780, l200, 720, 740 $cm^{-1}$

Preparation 68

1-[2-(4-Octyloxyphenyl)acetyl]-lH-benzotriazole-3-oxide

IR (Nujol) : l730, l460, l420, l250, ll30 $cm^{-1}$

Preparation 69

1-[3-(4-Octyloxyphenyl)propionyl]-lH-benzotriazole-3-oxide

IR (Nujol) : l730, l420, l340, l240, 950 $cm^{-1}$

Preparation 70

1-[(E)-3-(4-Octyloxyphenyl)acryloyl]-lH-benzotriazole-3-oxide

IR (Nujol) : l770, l600, l260, l080 $cm^{-1}$

Preparation 7l

1-($O^4$-Octyl-N,N-dimethyl-L-tyrosyl)-lH-benzotriazole-3-oxide

IR (Neat) : 2930, 2850, l800, l6l0 $cm^{-1}$

Preparation 72

To a suspension of lithium aluminum hydride (4.05 g) in tetrahydrofuran (475 ml) was added dropwise a solution of 4-octyloxybenzaldehyde (25 g) in tetrahydrofuran (25 ml) at 55 ~ 60°C. The reaction mixture was stirred under reflux for l hour, and thereto was added sodium fluoride (35.84 g) and water (ll.52 ml) under ice-cooling. The mixture was stirred for 30 minutes, and filtrated. The filtrate was evaporated in vacuo to give 4-octyloxybenzyl alcohol (25.l g) as crystals.

IR (Nujol) : 3200, l605, l5l0 $cm^{-1}$

NMR (DMSO-$d_6$, δ) : 0.86 (3H, t, J=6.7Hz), l.26-l.38 (l0H, m), l.62-l.72 (2H, m), 3.92 (2H, t, J=6.5Hz), 4.40 (2H, d, J=5.7Hz), 5.03 (lH, t, J=5.7Hz), 6.85 (2H, d, J=8.6Hz), 7.20 (2H, d, J=8.6Hz)

Preparation 73

To a suspension of 4-octyloxybenzyl alcohol (25 g), N-hydroxyphthalimide (l7.l5 g) and triphenylphosphine (27.74 g) in tetrahydrofuran (250 ml) was added dropwise diethyl azodicarboxylate (l8.4 g) under ice-cooling. The reaction mixture was stirred at room temperature for 2 hours, and evaporated in vacuo. The residue was purified by chromatography on silica gel to give N-(4-octyloxybenzyloxy)phthalimide (33.45 g) as crystals.

IR (Nujol) : l780, l725, l605, l580, l505 $cm^{-1}$

NMR (DMSO-$d_6$, δ) : 0.86 (3H, m), l.26 (l0H, m), l.70 (2H, m), 3.95 (2H, t, J=6.5Hz), 5.08 (2H, s), 6.93 (2H, d, J=8.6Hz), 7.40 (2H, d, J=8.6Hz), 7.85 (4H, s)

Preparation 74

To a solution of N-(4-octyloxybenzoyloxy)phthalimide (4.l3 g) in tetrahydrofuran (l6 ml) was added hydrazine-hydrate (0.53 ml) at room temperature. After the mixture was stirred at the same temperature for l hour, the precipitate was filtered off. To the filtrate was added water (6 ml) and 4-hydroxyphenylglyoxylic acid (l.5 g) at room temperature. The mixture was maintained at pH 4~4.5 with agueous sodium bicarbonate solution for 2 hours, thereto was added ethyl acetate, and adjusted to pH 2 with lN hydrochloric acid. The separated organic layer was washed with brine, and dried over magnesium sulfate. The organic solvent was evaporated in vacuo to give 2-(4-hydroxyphenyl)-2-(4-octyloxybenzyloxyimino)acetic acid (3.4 g).

IR (Nujol) : 3400, l7l5, l605, l590, l505 $cm^{-1}$

NMR (DMSO-$d_6$, δ) : 0.86 (3H, m), l.25 (l0H, m), l.69 (2H, m), 3.94 (2H, t, J=6.4Hz), 5.07 (2H, s), 6.82 (2H, d, J=8.7Hz), 6.90 (2H, d, J=8.6Hz), 7.29 (2H, d, J=8.6Hz), 7.35 (2H, d, J=8.7Hz)

The following compounds (Preparations 75 and 76) were obtained according to a similar manner to that of Preparation 74.

Preparation 75

2-Phenyl-2-(4-octyloxybenzyloxyimino)acetic acid

IR (Nujol) : l720, l6l0, l585, l5l5 $cm^{-1}$

NMR (DMSO-$d_6$, δ) : 0.86 (3H, t, J=6.7Hz), l.26 (l0H, m), l.69 (2H, m), 3.94 (2H, t, J=6.5Hz), 5.l3 (2H, s), 6.9l (2H, d, J=8.6Hz), 7.22-7.49 (7H, m)

Preparation 76

2-(4-Octyloxybenzyloxyimino)acetic acid

IR (Nujol) : l700, l670, l600 $cm^{-1}$

NMR (DMSO-$d_6$, δ) : 0.86 (3H, t, J=6.2Hz), l.26 (l0H, m), l.70 (2H, m), 3.95 (2H, t, J=6.5Hz), 5.l3 (2H, s), 6.9l (2H, d, J=8.6Hz), 7.29 (2H, d, J=8.6Hz), 7.56 (lH, s)

Preparation 77

A solution of 4-octyloxyphenylglyoxylic acid (0.935 g) in a mixture of water (9 ml) and tetrahydrofuran (l8 ml) and adjusted to pH 3.5-4 with lN hydrochloric acid and methoxyamine hydrochloride (0.337 g) was added thereto at room temperature. The mixture was stirred for 2 hours at room temperature maintaining pH 3.5~4 with lN hydrochloric acid. The reaction mixture was added to ethyl acetate. The organic layer was separated and dried over magnesium sulfate. The magnesium sulfate was filtered off, and the filtrate was evaporated under reduced pressure to give 2-(4-octyloxyphenyl)-2-methoxyiminoacetic acid (0.57 g).

IR (Nujol) : l700, l600, l250, l030 $cm^{-1}$

NMR (DMSO-$d_6$, δ) : 0.86 (3H, t, J=6.3Hz), l.2-l.5 (l0H, m), l.6-l.8 (2H, m), 3.89 (3H, s), 3.99 (2H, t, J=6.4Hz), 7.00 (2H, d, J=8.9Hz), 7.45 (2H, d, J=8.9Hz), l4.05 (lH, s)

Preparation 78

To a mixture of 2,3,4,5,6-pentafluorobenzoic acid (l g) and 2,2,3,3,4,4,5,5-octafluoropentanol (l.l8 g) in N,N-dimethylformamide (5 ml) was added 62% sodium hydride (0.39 g) at room temperature. The mixture was stirred at the same temperature for l hour, and thereto was added a mixture of water and ethyl acetate.

The separated organic layer was washed with water and brine, dried over magnesium sulfate, and evaporated in vacuo. The residue was purified by chromatography on silica gel to give 4-(2,2,3,3,4,4,5,5-octafluoropentyloxy)-2,3,5,6-tetrafluorobenzoic acid (923.0 mg).

IR (Nujol) : l700, l580 $cm^{-1}$

NMR (DMSO-$d_6$, $\delta$) : 4.96 (2H, t, J=l4.2Hz), 7.l0 (lH, tt, J=5.6Hz and 50.2Hz)

Preparation 79

4-(2,2,3,3,4,4,5,5,6,6,7,7,8,8,8-Pentadecafluorooctyloxy)-2,3,5,6-tetrafluorobenzoic acid

IR (Nujol) : 3400, l640, l560 $cm^{-1}$

NMR (DMSO-$d_6$, $\delta$) : 4.95 (2H, t, J=l4.0Hz)

The following compounds (Preparations 80 to 90) were obtained according to a similar manner to that of Preparation 5.

Preparation 80

Succinimido 2-(4-hydroxyphenyl)-2-(4-octyloxybenzyloxyimino)acetate

IR (Nujol) : l800, l770, l700, l600 $cm^{-1}$

Preparation 8l

Succinimido 2-phenyl-2-(4-octyloxybenzyloxyimino)acetate

IR (Nujol) : l780, l730, l605 $cm^{-1}$

NMR (DMSO-$d_6$, $\delta$) : 0.86 (3H, m), l.26 (l0H, m), l.69 (2H, m), 2.90 (4H, m), 3.94 (2H, t, J=6.4Hz), 5.30 (2H, s), 6.9l (2H, d, J=8.6Hz), 7.25-7.56 (7H, m)

Preparation 82

Succinimido 2-(4-Octyloxybenzyloxyimino)acetate

IR (Nujol) : l760, l725, l600, l580 $cm^{-1}$

NMR (DMSO-$d_6$, $\delta$) : 0.86 (3H, t, J=6.7Hz), l.26 (l0H, m), l.70 (2H, m), 2.85 (4H, s), 3.96 (2H, m), 5.28 (2H, s), 6.9l (2H, d, J=8.6Hz), 7.33 (2H, d, J=8.6Hz), 8.l2 (lH, s)

Preparation 83

Succinimido 4-(2,2,3,3,4,4,5,5-octafluoropentyloxy)-2,3,5,6-tetraflurobenzoate

IR (Nujol) : 3500, l770, l740, l640 $cm^{-1}$

NMR (DMSO-$d_6$, $\delta$) : 2.90 (4H, s), 5.23 (2H, t, J=l3.8Hz), 7.ll (lH, tt, J=50.2Hz and 5.6Hz)

Preparation 84

Succinimido 4-(2,2,3,3,4,4,5,5,6,6,7,7,8,8,8-pentadecafluorooctyloxy)-2,3,5,6-tetrafluorobenzoate

IR (Nujol) : l735, l620, l600 $cm^{-1}$

NMR (DMSO-$d_6$, $\delta$) : 2.90 (4H, s), 5.l2 (2H, t, J=l3.8Hz)

Preparation 85

Succinimido 3-methoxy-4-octyloxybenzoate

IR (Nujol) : l760, l730, l600, l280, l200, 880 $cm^{-1}$

NMR (DMSO-$d_6$, $\delta$) : 0.86 (3H, t, J=6.7Hz), l.2-l.5 (l0H, m), l.6-l.9 (2H, m), 2.88 (4H, s), 3.84 (3H, s), 4.09 (2H, t, J=6.5Hz), 7.l9 (lH, d, J=8.6Hz), 7.49 (lH, d, J=2.0Hz), 7.73 (lH, dd, J=8.6 and 2.0Hz)

Preparation 86

Succinimido 4-(2-butoxyethoxy)benzoate

IR (Nujol) : 1730, 1600, 1250, 1060 $cm^{-1}$

NMR ($DMSO-d_6$, $\delta$) : 0.87 (3H, t, J=7.2Hz), 1.2-1.6 (4H, m), 2.89 (4H, s), 3.46 (2H, t, J=6.3Hz), 3.73 (2H, t, J=4.4Hz), 4.25 (2H, t, J=4.4Hz), 7.18 (2H, d, J=9.0Hz), 8.04 (2H, d, J=9.0Hz)

Preparation 87

Succinimido 2-(4-Octyloxyphenyl)-2-methoxyacetate

IR (Nujol) : 1810, 1740, 1610, 1250, 1210, 1100 $cm^{-1}$

NMR ($DMSO-d_6$, $\delta$) : 0.86 (3H, t, J=6.7Hz), 1.2-1.5 (10H, m), 1.6-1.8 (2H, m), 2.80 (4H, s), 3.35 (3H, s), 3.97 (2H, t, J=6.4Hz), 5.35 (1H, s), 6.96 (2H, d, J=8.7Hz), 7.38 (2H, d, J=8.7Hz)

Preparation 88

$O^4$-Octyl-N-(t-butoxycarbonyl)-D-tyrosine succinimido ester

IR (Nujol) : 3370, 1780, 1730, 1700, 1250, 1200 $cm^{-1}$

Preparation 89

Succinimido 2-(4-octyloxyphenyl)-2-methoxyiminoacetate

IR (Nujol) : 1800, 1780, 1730, 1600, 1250, 1180, 1130 $cm^{-1}$

NMR ($DMSO-d_6$, $\delta$) : 0.86 (3H, t, J=6.6Hz), 1.2-1.5 (10H, m), 1.6-1.8 (2H, m), 2.89 (4H, s), 4.01 (3H, s), 4.03 (2H, t, J=6.4Hz), 7.08 (2H, d, J=8.9Hz), 7.68 (2H, d, J=8.9Hz)

Preparation 90

$N^{\tau}$-Octyl-N-(t-butoxycarbonyl)-L-histidine succinimido ester

IR (Neat) : 1810, 1780, 1730, 1500, 1360, 1200, 1160 $cm^{-1}$

Preparation 91

4-Octyloxyphthalic anhydride was obtained from 4-octyloxyphthalic acid according to a similar manner to that of Preparation 5.

IR (Neat) : 2910, 2850, 1840, 1760, 1640, 1610, 1290, 1260 $cm^{-1}$

NMR ($DMSO-d_6$, $\delta$) : 0.86 (3H, t, J=6.8Hz), 1.2-1.5 (10H, m), 1.6-1.9 (2H, m), 4.19 (2H, t, J=6.5Hz), 7.47 (1H, dd, J=8.4Hz and 2.2Hz), 7.57 (1H, d, J=2.2Hz), 7.98 (1H, d, J=8.4Hz)

Preparation 92

N-Octyloxycarbonyloxysuccinimide was obtained according to a similar manner to that of Preparation 5.

IR (Neat) : 2960, 2850, 1780, 1740, 1260, 1230 $cm^{-1}$

NMR ($CDCl_3$, $\delta$) : 0.89 (3H, t, J=6.7Hz), 1.2-1.4 (10H, m), 1.6-1.8 (2H, m), 2.84 (4H, s), 4.32 (2H, t, J=6.7Hz)

Preparation 93

To a solution of octyl phenyl ether (1.53 g) in chloroform (6 ml) was added chlorosulfonic acid at 0 °C. The mixture was stirred at room temperature for 30 minutes, then the mixture was poured into a mixture of water and tetrahydrofuran.

The separated organic layer was washed with sodium chloride aqueous solution, dried over magnesium sulfate and then the solvent was evaporated in vacuo. The residue was subjected to a column chromatography on silica gel to give 4-octyloxyphenylsulfonyl chloride (1.25 g).

IR (Nujol) : 1600, 1580, 1500, 1380, 1180 $cm^{-1}$

NMR ($CDCl_3$, $\delta$) : 0.89 (3H, t, J = 6.6Hz), 1.20-1.50 (10H, m), 1.80 (2H, m), 4.06 (2H, t, J = 6.4Hz), 7.03 (2H, d, J = 9.0Hz), 7.96 (2H, d, J = 9.0Hz)

In the following, the structures of the compounds of Examples 13 to 53 are shown.

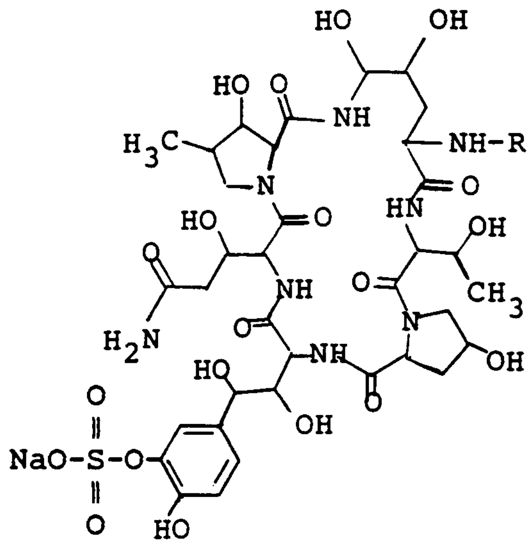

In the following formulae, $^tBu$ means t-butyl, and p-TsOH means p-toluenesulfonic acid.

| Example No. | Compound No. | R |
|---|---|---|
| 13 | FR139835 | $-COO(CH_2)_7CH_3$ |
| 14 | FR139537 | 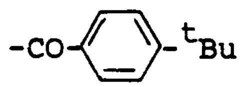 |
| 15 | FR141145 | 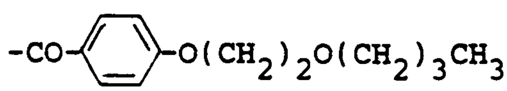 |
| 16 | FR139538 | 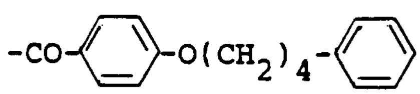 |

| Example No. | Compound No. | R |
|---|---|---|
| 17 | FR140215 | 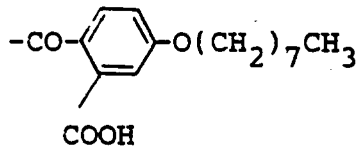 |
| 18 | FR140216 | 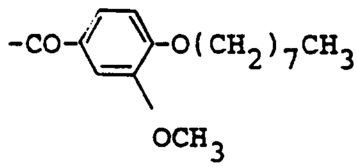 |
| 19 | FR140727 | 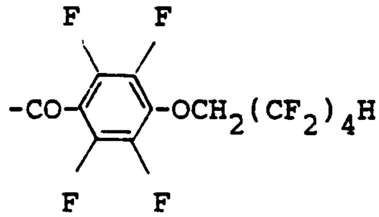 |
| 20 | FR143301 | 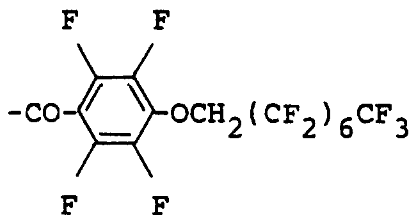 |
| 21 | FR140495 | 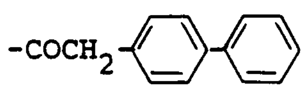 |
| 22 | FR139503 | 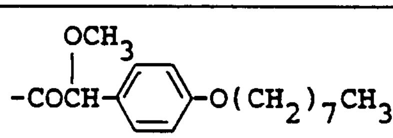 |
| 23 | FR139500 | 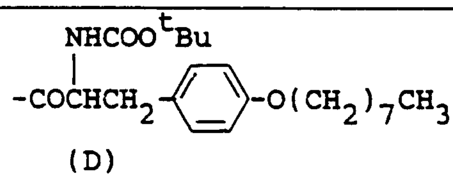 |
| 24 | FR139501 | 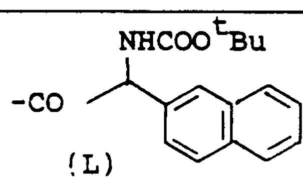 |

| Example No. | Compound No. | R |
|---|---|---|
| 25 | FR139502 | 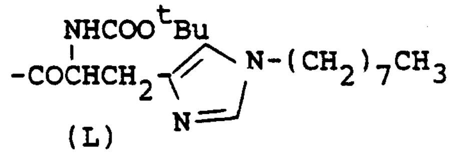 |
| 26 | FR138959 | 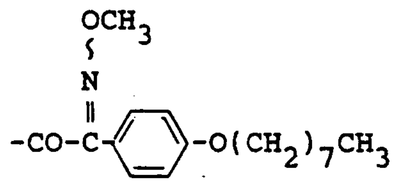 |
| 27 | FR140291 | 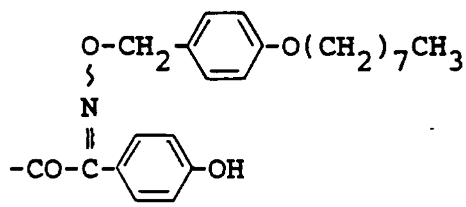 |
| 28 | FR141580 | 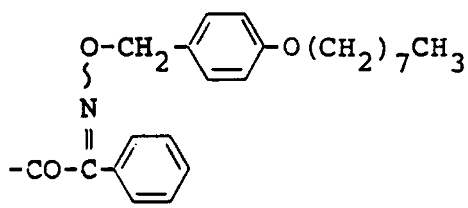 |
| 29 | FR141579 | 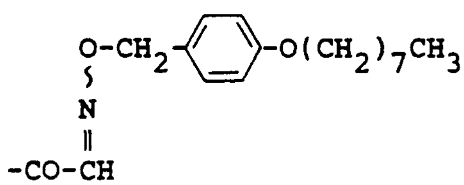 |
| 30 | FR141146 | 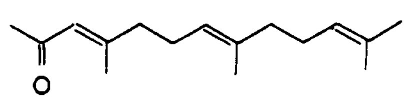 |
| 31 | FR140731 | 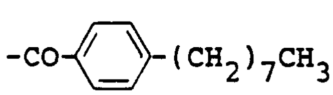 |
| 32 | FR140217 | 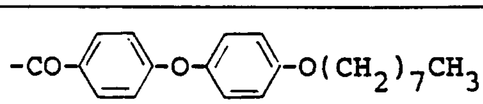 |

| Example No. | Compound No. | R |
|---|---|---|
| 33 | FR142472 | 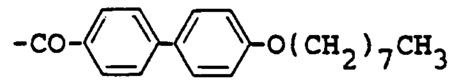 |
| 34 | FR140496 | 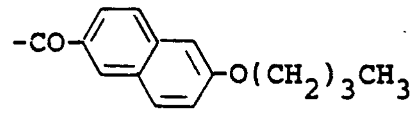 |
| 35 | FR140497 | 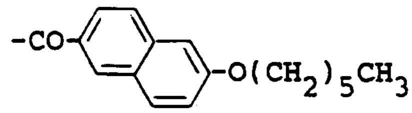 |
| 36 | FR143483 | 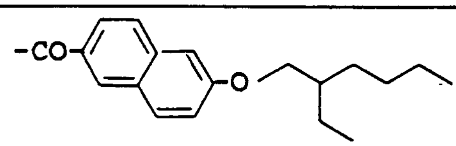 |
| 37 | FR140728 | 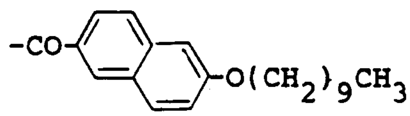 |
| 38 | FR142172 | 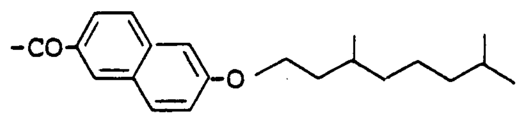 |
| 39 | FR143326 | 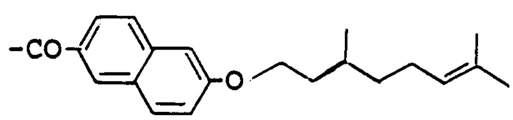 |
| 40 | FR142390 | 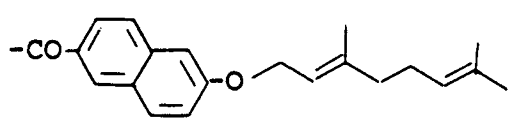 |
| 41 | FR140729 | 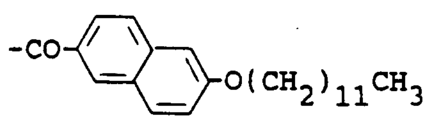 |
| 42 | FR140730 | 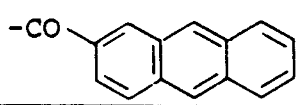 |

| Example No. | Compound No. | R |
|---|---|---|
| 43 | FR143020 | 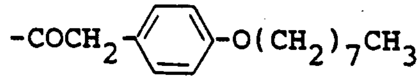 |
| 44 | FR143021 | 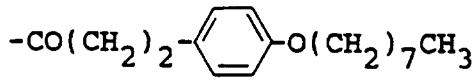 |
| 45 | FR141315 | 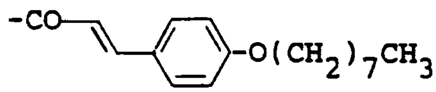 |
| 46 | FR140105 | 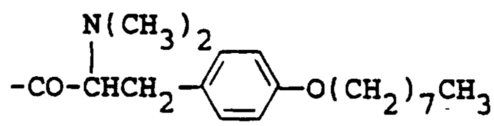 |
| 47 | FR141564 | 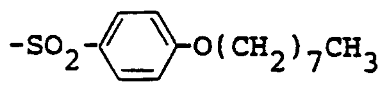 |
| 48 | FR143170 | 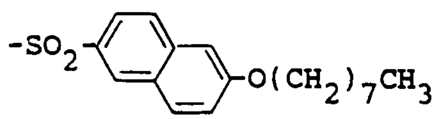 |
| 49 | FR138912 | 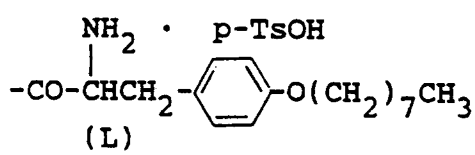 |
| 50 | FR138960 | 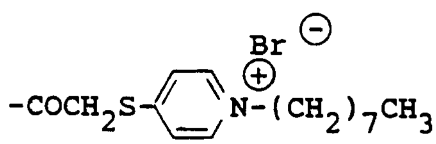 |
| 51 | FR138727 | 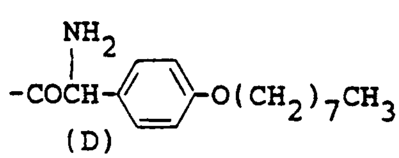 |

| Example No. | Compound No. | R |
|---|---|---|
| 52 | FR138912 | 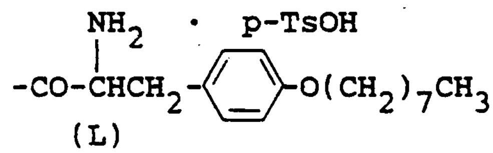 |
| 53 | FR138960 | 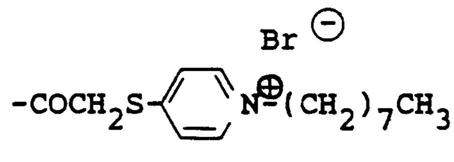 |

Example 13

FR139835 substance was obtained by reacting FR133303 substance with N- octyloxycarbonyloxysuccinimide according to a similar manner to that of Example 3.

IR (Nujol) : 3300, l620 $cm^{-1}$

FAB-MS e/z = 1137 (M + Na)

Example 14

FR139537 substance was obtained by reacting FR133303 substance with succinimido 4-t-butylbenzoate according to a similar manner to that of Example 3.

IR (Nujol) : 3300, 1620 $cm^{-1}$

NMR ($D_2O$, $\delta$) : l.05 (3H, d, J=6.9Hz), l.l5 (3H, d, J=5.9Hz), l.33 (9H, s), 2.0-2.3 (3H, m), 2.4-2.6 (3H, m), 2.7-2.9 (lH, m), 3.4-3.6 (lH, m), 3.8-4.9 (l2H, m), 5.07 (2H, m), 5.40 (lH, d, J=3Hz), 7.06 (lH, d, J=8.2Hz), 7.08 (lH, dd, J=8.2Hz and 2Hz), 7.27 (lH, d, J=2Hz), 7.60 (lH, d, J=8.6Hz), 7.75 (lH, d, J=8.6Hz)

Example 15

FR141145 substance was obtained by reacting FR133303 substance with succinimido 4-(2-butoxyethoxy)benzoate according to a similar manner to that of Example 3.

IR (Nujol) : 3300, l620 $cm^{-1}$

NMR (DMSO-$d_6$, + $D_2O$, $\delta$) : 0.88 (3H, t, J=7.3Hz), 0.96 (3H, d, J=6.7Hz), l.04 (3H, d, J=5.7Hz), l.2-l.6 (4H, m), l.7-2.0 (3H, m), 2.l-2.65 (4H, m), 3.l6 (lH, m), 3.7-4.5 (20H, m), 4.78 (lH, d, J=3Hz), 4.86 (lH, d, J=3.8Hz), 5.02 (lH, d, J=3Hz), 6.74 (lH, d, J=8.2Hz), 6.79 (lH, d, J=8.2Hz), 7.00 (2H, d, J=8.9Hz), 7.06 (lH, s), 7.87 (2H, d, J=8.9Hz)

FAB-MS e/z = 1201 (M + Na)

Example 16

FR139538 substance was obtained by reacting FR133303 substance with succinimido 4-(4-phenylbutoxy)benzoate according to a similar manner to that of Example 3.

IR (Nujol) : 3300, l620 $cm^{-1}$

FAB-MS e/z = l233 (M + Na)

Example 17

FR140215 substance was obtained by reacting FR133303 substance with 4-octyloxyphthalic anhydride according to a similar manner to that of Example 3.

IR (Nujol) : 3300, l620 $cm^{-1}$

FAB-MS e/z = 1257 (M + Na)

Example 18

FR1402l6 substance was obtained by reacting FR133303 substance with succinimido 3-methoxy-4-octyloxybenzoate according to a similar manner to that of Example 3.

IR (Nujol) : 3300, l620 $cm^{-1}$

FAB-MS e/z = l243 (M + Na)

Example 19

FR140727 substance was obtained by reacting FR133303 substance with succinimido 4-(2,2,3,3,4,4,5,5-octafluoropentyloxy)-2,3,5,6-tetrafluorobenzoate according to a similar manner to that of Example 3.

IR (Nujol) : 3300, l630 $cm^{-1}$

FAB-MS e/z : l387 (M + Na)

Example 20

FR143301 substance was obtained by reacting FR133303 substance with succinimido 4-(2,2,3,3,4,4,5,5,6,6,7,7,8,8,8-pentadecafluorooctyloxy)-2,3,5,6-tetrafluorobenzoate according to a similar manner to that of Example 3.

IR (Nujol) : 3300, l630 $cm^{-1}$

FAB-MS e/z = l534 ($M^+$)

Example 21

FR140495 substance was obtained by reacting FR133303 substance with succinimido 2-(4-biphenylyl)-acetate according to a similar manner to that of Example 3.

IR (Nujol) : 3300, l620 $cm^{-1}$

NMR ($CD_3OD$, δ) : l.0-l.l (6H, m), l.9-2.2 (3H, m), 2.3-2.6 (3H, m), 2.7-2.85 (lH, m), 3.35 (lH, m), 3.58 (2H, s), 3.65-4.7 (l3H, m), 4.93 (lH, d, J=3Hz), 5.04 (lH, d, J=3.8Hz), 5.25 (lH, d, J=3Hz), 6.85 (lH, d, J=8.3Hz), 7.0l (lH, dd, J=8.3Hz and 2Hz), 7.3-7.6 (l0H, m)

Example 22

FR139503 substance was obtained by reacting FR133303 substance with succinimido 2-(4-octyloxyphenyl)-2-methoxyacetate according to a similar manner to that of Example 3.

IR (Nujol) : 3330, l620 $cm^{-1}$

FAB-MS e/z = 1257 (M + Na)

Example 23

FR139500 substance was obtained by reacting FR133303 substance with $O^4$-octyl-N-(t-butoxycarbonyl)-D-tyrosine succinimido ester according to a similar manner to that of Example 3.

IR (Nujol) : 3300, l620 $cm^{-1}$

NMR ($CD_3OD$, δ) : 0.90 (3H, t, J=6.8Hz), l.06 (3H, d, J=6.8Hz), l.l7 (3H, d, J=6.7Hz), l.20-l.30 (l0H, m), l.35 (9H, s), l.74 (2H, m), l.9-2.l (3H, m), 2.45 (3H, m), 2.76 (lH, m), 3.0-3.l (lH, m), 3.37 (lH, m), 3.7-4.6 (l8H, m), 4.94 (lH, d, J=3Hz), 5.0l (lH, d, J=3.8Hz), 5.25 (lH, d, J=3Hz), 6.79 (2H, d, J=8.5Hz), 6.83 (lH, d, J=8.3Hz), 7.03 (lH, dd, J=8.3Hz and 2Hz), 7.l2 (2H, d, J=8.5Hz), 7.3l (lH, d, J=2Hz)

Example 24

FR139501 substance was obtained by reacting FR133303 substance with N-(t-butoxycarbonyl)-L-2-(2-naphthyl)glycine succinimido ester according to a similar manner to that of Example 3.
IR (Nujol) : 3300, l620 $cm^{-1}$

Example 25

FR139502 substance was obtained by reacting FR133303 substance with $N^{r}$-octyl-N-(t-butoxycarbonyl)-L-histidine succinimido ester according to a similar manner to that of Example 3.
IR (Nujol) : 3300, l620 $cm^{-1}$
FAB-MS e/z = l330 (M + Na)

Example 26

FR138959 substance was obtained by reacting FR133303 substance with succinimido 2-(4-octyloxyphenyl)-2-methoxyiminoacetate according to a similar manner to that of Example 3.
IR (Nujol) : 3300, l620 $cm^{-1}$
NMR ($CD_3OD$, $\delta$) : 0.9l (3H, t, J=6.6Hz), l.06 (3H, d, J=6.8Hz), l.25 (3H, d, J=6.3Hz), l.25-l.6 (l0H, m), l.65-l.9 (2H, m), l.9-2.2 (3H, m), 2.3-2.65 (3H, m), l.75-l.9 (lH, m), 3.3-3.5 (lH, m), 3.95 (3H, s), 3.7-4.75 (l6H, m), 5.03 (lH, d, J=3.0Hz), 5.ll (lH, d, J=3.7Hz), 5.46 (lH, d, J=2.7Hz), 6.86 (lH, d, J=8.2Hz), 6.89 (2H, d, J=8.9Hz), 7.0l (lH, dd, J=8.2Hz and 2Hz), 7.3l (lH, d, J=2Hz), 7.54 (2H, d, J=8.9Hz)
FAB-MS e/z = l270 (M + Na)

Example 27

FR14029l substance was obtained by reacting FR133303 substance with succinimido 2-(4-hydroxyphenyl)-2-(4-octyloxybenzyloxyimino)acetate according to a similar manner to that of Example 3.
IR (Nujol) : 3250, l650, l620 $cm^{-1}$
FAB-MS e/z = l363 (M + Na)

Example 28

FR141580 substance was obtained by reacting FR133303 substance with succinimido 2-phenyl-2-(4-octyloxybenzyloxyimino)acetate according to a similar manner to that of Example 3.
IR (Nujol) : 3300, l646 $cm^{-1}$
FAB-MS e/z = l346 (M + Na)

Example 29

FR141579 substance was obtained by reacting FR133303 substance with succinimido 2-(4-octyloxybenzyloxyimino)acetate according to a similar manner to that of Example 3.
IR (Nujol) : 3250, l650 $cm^{-1}$
FAB-MS e/z = l270 (M + Na)

Example 30

FR141146 substance was obtained by reacting FR133303 substance with 1-[(2E,6E)-3,7,11-trimethyl-2,6,10-dodecatrienoyl]-1H-benzotriazole-3-oxide according to a similar manner to that of Example 12.
IR (Nujol) : 3300, l620, l040 $cm^{-1}$
NMR ($CD_3OD$, $\delta$) : l.06 (3H, d, J=6.8Hz), l.l9 (3H, d, J=5.9Hz), l.60 (3H, s), l.62 (3H, s), l.66 (3H, s), l.9-2.2 (llH, m), 2.05 (3H, s), 2.3-2.6 (3H, m), 2.7-2.9 (lH, m), 3.35 (lH, m), 3.7-5.0 (14H, m), 5.08 (4H, m), 5.27 (lH, d, J=2.8Hz), 5.77 (lH, s), 6.86 (lH, d, J=8.3Hz), 7.04 (lH, dd, J=8.3Hz and l.9Hz), 7.32 (lH, d, J=l.9Hz)

Example 31

FR140731 substance was obtained by reacting FR133303 substance with 1-(4-octylbenzoyl)-1H-benzotriazole-3-oxide according to a similar manner to that of Example 12.

IR (Nujol) : 3300, 1620, 1040 $cm^{-1}$

NMR ($CD_3OD$, δ) : 0.86 (3H, t, J=6.8Hz), l.06 (3H, d, J=6.8Hz), l.2l (3H, d, J=5.8Hz), l.25-l.45 (l0H, m), l.55-l.75 (2H, m), l.9-2.25 (3H, m), 2.35-2.6 (3H, m), 2.65 (2H, t, J=7.5Hz), 2.8l (lH, m), 3.32 (lH, m), 3.7-4.8 (l4H, m), 4.98 (lH, d, J=3Hz), 5.09 (lH, d, J=3.9Hz), 5.3l (lH, d, J=3Hz), 6.86 (lH, d, J=8.3Hz), 7.03 (lH, dd, J=8.3Hz and 2Hz), 7.24 (2H, d, J=8.2Hz), 7.33 (lH, d, J=2Hz), 7.74 (2H, d, J=8.2Hz)

FAB-MS e/z = ll97 (M + Na)

Example 32

FR140217 substance was obtained by reacting FR133303 substance with 1-[4-(4-octyloxy)phenoxy]-benzoyl-1H-benzotriazole-3-oxide according to a similar manner to that of Example 12.

IR (Nujol) : 3300, l620 $cm^{-1}$

FAB-MS e/z = l305 (M + Na)

Example 33

FR142472 substance was obtained by reacting FR133303 substance with 1-[4-(4-octyloxyphenyl)-benzoyl]-1H-benzotriazole-3-oxide according to a similar manner to that of Example 12.

IR (Nujol) : 3300, l620 $cm^{-1}$

NMR ($CD_3OD$, δ) : 0.88 (3H, t, J=6.7Hz), l.06 (3H, d, J=6.8Hz), l.23 (3H, d, J=6.lHz), l.3-l.6 (l0H, m), l.8-l.9 (2H, m), l.9-2.3 (3H, m), 2.3-2.7 (3H, m), 2.9-3.0 (lH, m), 3.39 (lH, m), 3.7-4.7 (l6H, m), 4.99 (lH, d, J=3.0Hz), 5.l0 (lH, d, J=3.7Hz), 5.35 (lH, d, J=2.7Hz), 6.87 (lH, d, J=8.3Hz), 6.99 (2H, d, J=8.8Hz), 7.04 (lH, dd, J=8.3Hz and l.9Hz), 7.33 (lH, d, J=l.9Hz), 7.58 (2H, d, J=8.8Hz), 7.62 (2H, d, J=8.4Hz), 7.87 (2H, d, J=8.4Hz)

FAB-MS e/z = l289 (M + Na)

Example 34

FR140496 substance was obtained by reacting FR133303 substance with l-(6-butoxy-2-naphthoyl)-1H-benzotriazole-3-oxide according to a similar manner to that of Example 12.

IR (Nujol) : 3300, 1620 $cm^{-1}$

FAB-MS e/z = l207 (M + Na)

Example 35

FR140497 substance was obtained by reacting FR133303 substance with 1-(6-hexyloxy-2-naphthoyl)-1H-benzotriazole-3-oxide according to a similar manner to that of Example 12.

IR (Nujol) : 3300, l620 $cm^{-1}$

NMR (DMSO-$d_6$ + $D_2O$, δ) : 0.89 (3H, t, J=6.6Hz), 0.97 (3H, d, J=6.9Hz), l.08 (3H, d, J=5.9Hz), l.2-l.6 (6H, m), l.7-2.l (5H, m), 2.l-2.5 (3H, m), 2.5-2.7 (lH, m), 3.l9 (lH, m), 3.73 (2H, m), 3.8-4.5 (l2H, m), 4.80 (lH, d, J=3Hz), 4.88 (lH, d, J=3.8Hz), 5.08 (lH, d, J=3Hz), 6.74 (lH, d, J=8.2Hz), 6.80 (lH, dd, J=8.2Hz and 2Hz), 7.08 (lH, d, J=2Hz), 7.26 (lH, dd, J=8.9Hz and 2.4Hz), 7.39 (lH, d, J=2.4Hz), 7.85 (lH, d, J=8.7Hz), 7.89 (lH, d, J=8.7Hz), 7.93 (lH, d, J=8.9Hz), 8.44 (lH, s)

FAB-MS e/z = l236 (M + Na)

Example 36

FR143483 substance was obtained by reacting FR133303 substance with 1-[6-(2-ethylhexyloxy)-2-naphthoyl]-1H-benzotriazole-3-oxide according to a similar manner to that of Example 12.

IR (Nujol) : 3250, l620 $cm^{-1}$

NMR ($CD_3OD$, δ) : 0.93 (3H, t, J = 7.4Hz), 0.98 (3H, t, J = 7.4Hz), l.06 (3H, d, J = 6.8Hz), l.24 (3H, d, J = 6.0Hz), l.3-l.7 (8H, m), l.7-l.9 (lH, m), l.9-2.3 (3H, m), 2.3-2.7 (3H, m), 2.8-3.0 (lH, m), 3.39 (lH, m), 3.7-4.7 (l6H, m), 5.00 (lH, d, J = 4.4Hz), 5.ll (lH, d, J = 3.7Hz), 5.37 (lH, d, J = 2.6Hz), 6.87 (lH, d, J = 8.3Hz), 7.04 (lH, dd, J = 8.3Hz and 2Hz), 7.l7 (lH, dd, J = 8.9Hz and l.9Hz), 7.22 (lH, d, J = 2Hz), 7.33 (lH, d, J = l.9Hz), 7.7-7.9 (3H, m), 8.29 (lH, s)

FAB-MS e/z = 1263 (M + Na)

Example 37

FR140728 substance was obtained by reacting FR133303 substance with 1-(6-decyloxy-2-naphthoyl)-lH-benzotriazole-3-oxide according to a similar manner to that of Example 12.

IR (Nujol) : 3300, l620 $cm^{-1}$

NMR ($DMSO-d_6$ + $D_2O$, δ) : 0.86 (3H, t, J = 6.6Hz), 0.97 (3H, d, J = 6.7Hz), l.07 (3H, d, J = 5.9Hz), l.2-l.6 (l4H, m), l.7-2.l (5H, m), 2.l-2.5 (3H, m), 2.5-2.7 (lH, m), 3.l9 (lH, m), 3.45 (lH, m), 3.73 (2H, m), 3.9-4.5 (l2H, m), 4.79 (lH, d, J = 3Hz), 4.87 (lH, d, J = 3.8Hz), 5.07 (lH, d, J = 3Hz), 6.74 (lH, d, J = 8.2Hz), 6.79 (lH, dd, J = 8.lHz and 2Hz), 7.06 (lH, d, J = 2Hz), 7.23 (lH, dd, J = 8.9Hz and 2.4Hz), 7.38 (lH, d, J = 2.4Hz), 7.85 (lH, d, J = 8.7Hz), 7.89 (lH, d, J = 8.7Hz), 7.93 (lH, d, J = 8.9Hz), 8.45 (lH, s)

FAB-MS e/z = l29l (M + Na)

Example 38

FR142172 substance was obtained by reacting FR133303 substance with 1-[6-(3,7-dimethyloctyloxy)-2-naphthoyl]-1H-benzotriazole-3-oxide according to a similar manner to that of Example 12.

IR (Nujol) : 3300, l6l0 $cm^{-1}$

NMR ($DMSO-d_6$ + $D_2O$, δ) : 0.85 (6H, d, J = 6.6Hz), 0.95 (3H, d, J = 5.9Hz), 0.97 (3H, d, J = 6.7Hz), l.08 (3H, d, J = 5.9Hz), l.l-l.4 (6H, m), l.4-2.l (7H, m), 2.l-2.5 (3H, m), 2.5-2.7 (lH, m), 3.l9 (lH, m), 3.74 (2H, m), 3.9-4.6 (l2H, m), 4.8l (lH, d, J = 3Hz), 4.87 (lH, d, J = 3.8Hz), 5.07 (lH, d, J = 3Hz), 6.74 (lH, d, J = 8.2Hz), 6.83 (lH, dd, J = 8.lHz and 2Hz), 7.06 (lH, d, J = 2Hz), 7.23 (lH, dd, J = 8.9Hz and 2.4Hz), 7.40 (lH, d, J = 2.4Hz), 7.85 (lH, d, J = 8.7Hz), 7.89 (lH, d, J = 8.7Hz), 7.93 (lH, d, J = 8.9Hz), 8.45 (lH, s)

FAB-MS e/z = l29l (M + Na)

Example 39

FR143326 substance was obtained by reacting FR133303 substance with 1-[6-(3,7-dimethyl-6-octenyloxy)-2-naphthoyl]-lH-benzotriazole-3-oxide according to a similar manner to that of Example 12.

IR (Nujol) : 3300, l620, l260, l040 $cm^{-1}$

NMR ($CD_3OD$, δ) : l.00 (3H, d, J = 6.2Hz), l.06 (3H, d, J = 6.8Hz), l.25 (3H, d, J = 5.9Hz), l.2-l.6 (2H, m), l.6l (3H, s), l.67 (3H, s), l.63-2.3 (8H, m), 2.3-2.7 (3H, m), 2.8-3.0 (lH, m), 3.39 (lH, m), 3.7-4.8 (l6H, m), 5.00 (lH, d, J = 5.lHz), 5.08-5.2 (2H, m), 5.37 (lH, d, J = 2.5Hz), 6.87 (lH, d, J = 8.3Hz), 7.04 (lH, d, J = 8.3Hz), 7.l5 (lH, d, J = 8.9Hz), 7.2l (lH, s), 7.33 (lH, s), 7.7l (lH, d, J = 8.7Hz), 7.77-7.85 (2H, m), 8.28 (lH, s)

Example 40

FR142390 substance was obtained by reacting FR133303 substance with 1-[6-{(E)-3,7-dimethyl-2,6-octadienyloxy}-2-naphthoyl]-lH-benzotriazole-3-oxide according to a similar manner to that of Example 12.

IR (Nujol) : 3300, l620 $cm^{-1}$

NMR ($DMSO-d_6$ + $D_2O$, δ) : 0.97 (3H, d, J = 6.7Hz), l.07 (3H, d, J = 6.0Hz), l.57 (3H, s), l.6l (3H, s), l.76 (3H, s), l.8-2.5 (9H, m), 2.5-2.7 (lH, m), 3.l9 (lH, m), 3.45 (lH, m), 3.73 (2H, m), 3.9-4.6 (llH, m), 4.70 (2H, d, J = 6.5Hz), 4.80 (lH, d, J = 3Hz), 4.87 (lH, d, J = 3.8Hz), 5.07 (2H, m), 5.5l (lH, t, J = 6.5Hz), 6.74 (lH, d, J = 8.3Hz), 6.83 (lH, dd, J = 8.3Hz and 2Hz), 7.07 (lH, d, J = 2Hz),

7.24 (IH, dd, J=8.9Hz and 2.4Hz), 7.40 (IH, d, J=2.4Hz), 7.8-8.0 (3H, m), 8.45 (IH, s)

FAB-MS e/z = I287 (M + Na)

Example 41

FR140729 substance was obtained by reacting FR133303 substance with 1-(6-dodecyloxy-2-naphthoyl)-1H-benzotriazole-3-oxide according to a similar manner to that of Example 12.

IR (Nujol) : 3300, I6I0 $cm^{-1}$

NMR ($DMSO-d_6$ + $D_2O$, δ) : 0.85 (3H, t, J=6.6Hz), 0.97 (3H, d, J=6.7Hz), I.07 (3H, d, J=5.9Hz), I.2-I.6 (I8H, m), I.7-2.I (5H, m), 2.I-2.5 (3H, m), 2.5-2.7 (IH, m), 3.I9 (IH, m), 3.45 (IH, m), 3.73 (2H, m), 3.9-4.5 (I2H, m), 4.79 (IH, d, J=3Hz), 4.87 (IH, d, J=3.8Hz), 5.07 (IH, d, J=3Hz), 6.74 (IH, d, J=8.IHz), 6.78 (IH, dd, J=8.IHz and 2Hz), 7.06 (IH, d, J=2Hz), 7.23 (IH, dd, J=8.9Hz and 2.4Hz), 7.38 (IH, d, J=2.4Hz), 7.85 (IH, d, J=8.7Hz), 7.89 (IH, d, J=8.7Hz), 7.93 (IH, d, J=8.9Hz), 8.44 (IH, s)

FAB-MS e/z = I320 (M + Na)

Example 42

FR140730 substance was obtained by reacting FR133303 substance with 1-(2-anthrylcarbonyl)-IH-benzotriazole-3-oxide according to a similar manner to that of Example 12.

IR (Nujol) : 3300, 1620 $cm^{-1}$

FAB-MS e/z = 1185 (M + Na)

Example 43

FR143020 substance was obtained by reacting FR133303 substance with 1-[2-(4-octyloxyphenyl)-acetyl]-1H-benzotriazole-3-oxide according to a similar manner to that of Example 12.

IR (Nujol) : 3300, I620 $cm^{-1}$

NMR ($CD_3OD$, δ) : 0.87 (3H, t, J=6.8Hz), I.0-I.2 (6H, m), I.2-I.6 (I0H, m), I.6-I.85 (2H, m), I.85-2.I (3H, m), 2.3-2.6 (3H, m), 2.7-2.85 (IH, m), 3.32 (IH, m), 3.46 (2H, s), 3.7-4.7 (I6H, m), 5.04 (IH, d, J=3.7Hz), 5.23 (IH, d, J=2.7Hz), 6.75-6.9 (3H, m), 7.0I (IH, d, J=8.3Hz), 7.I5 (2H, d, J=8.5Hz), 7.30 (IH, s)

FAB-MS e/z = I227 (M + Na)

Example 44

FR143021 substance was obtained by reacting FR133303 substance with 1-[3-(4-octyloxyphenyl)-propionyl]-1H-benzotriazole-3-oxide according to a similar manner to that of Example 12.

IR (Nujol) : 3300, 1620 $cm^{-1}$

FAB-MS e/z = 1241 (M + Na)

Example 45

FR141315 substance was obtained by reacting FR133303 substance with 1-[(E)-3-(4-octyloxyphenyl)-acryloyl]-1H-benzotriazole-3-oxide according to a similar manner to that of Example 12.

IR (Nujol) : 3300, 1620 $cm^{-1}$

NMR ($DMSO-d_6$ + $D_2O$, δ) : 0.86 (3H, t, J=6.7Hz), 0.97 (3H, d, J=6.7Hz), I.04 (3H, d, J=5.4Hz), I.2-I.5 (I0H, m), I.6-2.0 (5H, m), 2.I-2.5 (3H, m), 2.5-2.6 (IH, m), 3.I7 (IH, m), 3.3-4.5 (I5H, m), 4.79 (IH, d, J=3Hz), 4.86 (IH, d, J=3.8Hz), 5.01 (IH, d, J=3Hz), 6.57 (IH, d, J=I5.8Hz), 6.74 (IH, d, J=8.2Hz), 6.82 (IH, d, J=8.2Hz), 6.97 (2H, d, J=8.8Hz), 7.09 (IH, s), 7.34 (IH, d, J=I5.8Hz), 7.52 (2H, d, J=8.8Hz)

FAB-MS e/z = I239 (M + Na)

Example 46

FR140105 substance was obtained by reacting FR133303 substance with l-($O^4$-octyl-N,N-dimethyl-L-tyrosyl)-lH-benzotriazole-3-oxide according to a similar manner to that of Example 12.

IR (Nujol) : 3300, l620 $cm^{-1}$

NMR ($CD_3OD$, δ) : 0.9l (3H, t, J = 6.8Hz), l.06 (3H, d, J = 6.8Hz), l.l2 (3H, d, J = 6.lHz), l.33 (l0H, m), l.74 (2H, m), l.98 (3H, m), 2.40 (6H, s), 2.3-2.6 (3H, m), 2.8 (2H, m), 2.9-3.l (lH, m), 3.3-3.5 (2H, m), 3.6-4.7 (l6H, m), 5.06 (lH, d, J = 3.8Hz), 5.33 (lH, d, J = 3Hz), 6.77 (2H, d, J = 8.6Hz), 6.86 (lH, d, J = 8.3Hz), 7.03 (lH, dd, J = 8.3Hz and 2Hz), 7.07 (2H, d, J = 8.6Hz), 7.3l (lH, d, J = 2Hz)

Example 47

FR141564 substance was obtained by reacting FR133303 substance with 4-octyloxyphenylsulfonyl chloride according to a similar manner to that of Example 6.

IR (Nujol) : 3300, l620 $cm^{-1}$

NMR ($DMSO-d_6$ + $D_2O$, δ) : 0.87 (3H, t, J = 6.7Hz), 0.97 (3H, d, J = 6.8Hz), l.04 (3H, d, J = 5.7Hz), l.l-l.5 (l0H, m), l.6-2.l (5H, m), 2.45 (3H, m), 2.5-2.7 (lH, m), 3.l9 (lH, m), 3.7-4.5 (l6H, m), 4.80 (lH, d, J = 3Hz), 4.88 (lH, d, J = 4Hz), 5.08 (lH, d, J = 3Hz), 6.74 (lH, d, J = 8.2Hz), 6.82 (lH, d, J = 8.2Hz), 6.84 (2H, d, J = 8.7Hz), 7.07 (lH, s), 7.5l (2H, d, J = 8.7Hz)

FAB-MS e/z = l249 (M + Na)

Example 48

FR143170 substance was obtained by reacting FR133303 substance with 6-octyloxy-2-naphthylsulfonyl chloride according to a similar manner to that of Example 6.

IR (Nujol) : 3300, l620 $cm^{-1}$

NMR ($CD_3OD$, δ) : 0.29 (3H, d, J = 6.0Hz), 0.9l (3H, t, J = 6.7Hz), l.07 (3H, d, J = 6.9Hz), l.25-l.6 (l0H, m), l.7-2.2 (5H, m), 2.2-2.6 (4H, m), 3.37 (lH, m), 3.55-4.65 (l7H, m), 4.97 (lH, m), 5.54 (lH, m), 6.84 (lH, d, J = 8.3Hz), 7.0l (lH, dd, J = 8.4Hz and 2Hz), 7.l5-7.3 (3H, m), 7.75-8.0 (3H, m), 8.35 (lH, s)

FAB-MS e/z = l299 (M + Na)

Example 49

To a solution of FR138364 substance obtained in Example 5 (0.24 g) in acetonitrile (5 ml) was added p-toluenesulfonic acid (0.l32 g) and stirred for 8 hours at room temperature. The reaction mixture was added to water and the aqueous layer was adjusted to pH 4.5 with saturated sodium bicarbonate aqueous solution. The aqueous solution was subjected to column chromatography on Diaion HP-20 and eluted with 80% aqueous methanol. The fractions containing the object compound were combined and evaporated under reduced pressure to remove methanol. The residue was lyophilized to give FR138912 substance (0.l5 g).

IR (Nujol) : 3300, l620 $cm^{-1}$

FAB-MS e/z = l272 (M + K)

Example 50

The mixture of FR138728 substance obtained in Example 8 (0.l5 g) and l-octyl-l,4-dihydropyridine-4-thione (0.03l g) in N,N-dimethylformamide was stirred for l.5 hours under ice-cooling. The reaction mixture was pulverized with diethyl ether (50 ml). The precipitate was filtrated and dried over phosphorus pentoxide under reduced pressure. The powder was added to water (300 ml) and adjusted to pH 4.5. The aqueous solution was subjected to column chromatography on Diaion HP-20 (50 ml) and eluted with 80% aqueous methanol. The fractions containing the object compound were combined and evaporated under reduced pressure to remove methanol. The residue was lyophilized to give FR138960 substance (0.l5 g).

IR (Nujol) : 3300, l620 $cm^{-1}$

FAB-MS e/z = l222 (Free M + Na)

The following compounds (Examples 51 to 53) were obtained according to a similar manner to that of Example 3.

Example 51

FR138727 substance

NMR ($CD_3OD$, $\delta$) : 0.90 (3H, t, J=6.8Hz), l.05 (3H, d, J=6.8Hz), l.l7-l.33 (l3H, m), l.6-l.8 (2H, m), l.9-2.l (3H, m), 2.50 (lH, m), 2.75 (lH, dd, J=l6Hz and 4Hz), 3.40 (lH, m), 3.7-3.8 (lH, m), 3.98 (2H, t, J=6.2Hz), 3.9-4.2 (5H, m), 4.3-4.5 (5H, m), 4.5-4.7 (3H, m), 4.97 (lH, d, J=3Hz), 5.06 (lH, s), 5.20 (lH, d, J=3Hz), 5.40 (lH, d, J=3Hz), 6.85 (lH, d, J=8.3Hz), 6.95 (2H, d,, J=8.5Hz), 7.02 (lH, d, J=8.3Hz), 7.30 (lH, d, J=8.5Hz), 7.44 (lH, s)

Example 52

FR138912 substance

IR (Nujol) : 3300, l620 $cm^{-1}$

Example 53

FR138960 substance

IR (Nujol) : 3300, l620 $cm^{-1}$

The following compounds (Preparations 94 and 95) were obtained according to a similar manner to that of Preparation 5.

Preparation 94

Succinimido 4-(4-heptyloxyphenyl)benzoate

IR (Nujol) : 1760, 1740, 1600 $cm^{-1}$

NMR ($CDCl_3$, $\delta$) : 0.87 (3H, t, J=6.8 Hz), 1.2-1.7 (8H, m), 1.7-1.9 (2H, m), 2.92 (4H, s), 4.01 (2H, t, J=6.5 Hz), 7.00 (2H, d, J=8.8 Hz), 7.58 (2H, d, J=8.8 Hz), 7.69 (2H, d, J=8.5 Hz), 8.17 (2H, d, J=8.5 Hz)

Preparation 95

Succinimido 4-(4-hexyloxyphenoxy)benzoate

IR (Nujol) : 1760, 1720, 1600 $cm^{-1}$

NMR ($CDCl_3$, $\delta$) : 0.92 (3H, t, J=6.8 Hz), 1.2-1.5 (6H, m), 1.7-1.9 (2H, m), 2.90 (4H, s), 3.96 (2H, t, J=6.5 Hz), 6.9-7.1 (6H, m), 8.07 (2H, d, J=9 Hz)

In the following, the structures of the compounds of Examples 54 and 55 are shown.

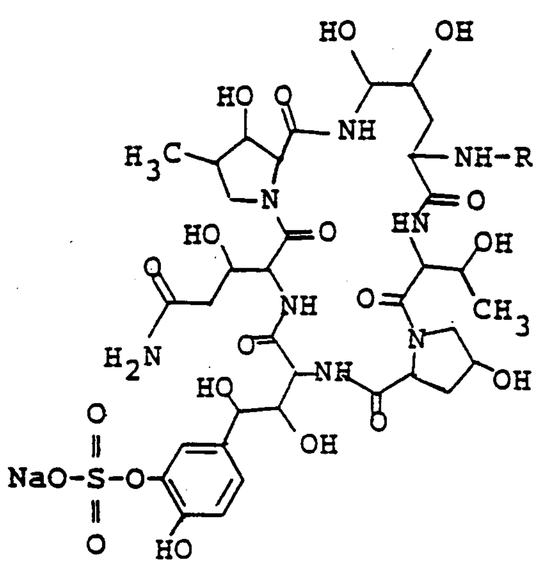

| Example No. | Compound No. | R |
|---|---|---|
| 54 | FR144274 | 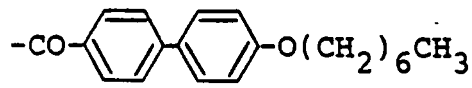 |
| 55 | FR144271 | 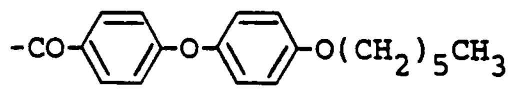 |

The following compounds (Examples 54 and 55) were obtained according to a similar manner to that of Example 3.

Example 54

FR144274

IR (Nujol) : 3300, 1620 $cm^{-1}$

| | | | | |
|---|---|---|---|---|
| Anal. Calcd. for $C_{55}H_{73}N_8SO_{22}Na\ 6H_2O$ | C : 48.53, | H : 6.29, | N : 8.23, | S : 2.35 |
| Found | C : 48.36, | H : 6.34, | N : 8.15, | S : 2.30 |

FAB-MS e/z 1275 (M + Na)

Example 55

FR144271

| | | | | |
|---|---|---|---|---|
| Anal. Calcd. for $C_{54}H_{71}N_8SO_{23}Na\ 6H_2O$ | C : 47.57, | H : 6.14, | N : 8.22, | S : 2.35 |
| Found | C : 47.58, | H : 6.05, | N : 8.18, | S : 2.27 |

FAB-MS e/z = 1277 (M + Na)

**Claims**

**1.** A pharmaceutical composition for the prevention and/or the treatment of Pneumocystis carinii infection which comprises, as an active ingredient, a polypeptide compound of the formula :

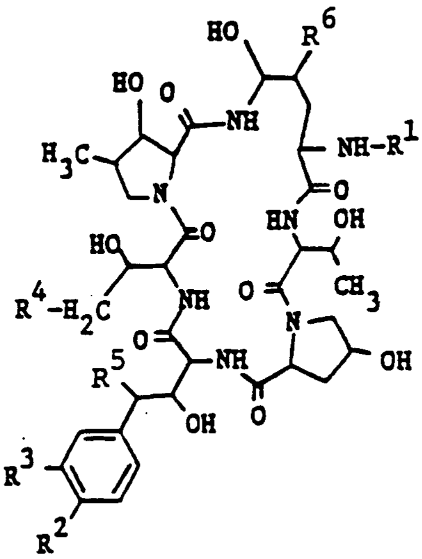

wherein
$R^1$ is hydrogen or acyl group,
$R^2$ is hydroxy or acyloxy,
$R^3$ is hydrogen, hydroxy or hydroxysulfonyloxy,
$R^4$ is hydrogen or carbamoyl, and
$R^5$ and $R^6$ are each hydrogen or hydroxy,
with proviso that
(i) $R^2$ is acyloxy, when $R^3$ is hydrogen, and
(ii) $R^5$ is hydrogen, when $R^6$ is hydrogen,

or a pharmaceutically acceptable salt thereof in admixture with a pharmaceutically acceptable carrier or excipient.

**2.** Use of a polypeptide compound of the formula :

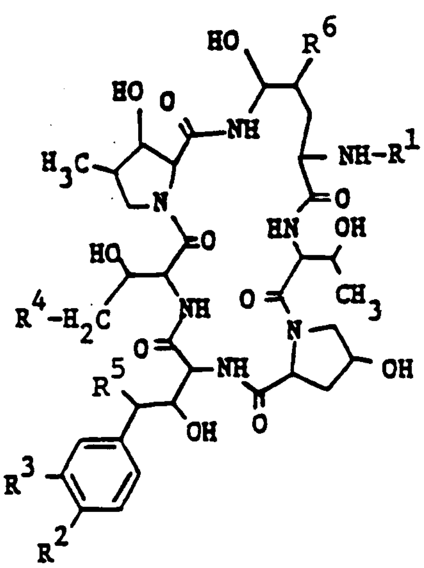

wherein
$R^1$ is hydrogen or acyl group,
$R^2$ is hydroxy or acyloxy,
$R^3$ is hydrogen, hydroxy or hydroxysulfonyloxy,

$R^4$ is hydrogen or carbamoyl, and
$R^5$ and $R^6$ are each hydrogen or hydroxy,
with proviso that
(i) $R^2$ is acyloxy, when $R^3$ is hydrogen, and
(ii) $R^5$ is hydrogen, when $R^6$ is hydrogen,

or a pharmaceutically acceptable salt thereof for the preparation of a medicament for the prevention and/or the treatment of Pneumocystis carinii infection.

**3.** A method for the prevention and/or the treatment of Pneumocystis carinii infection, which comprises administering a polypeptide compound of the formula :

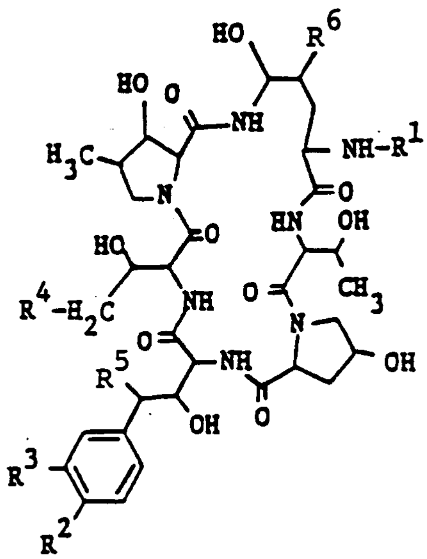

wherein
$R^1$ is hydrogen or acyl group,
$R^2$ is hydroxy or acyloxy,
$R^3$ is hydrogen, hydroxy or hydroxysulfonyloxy,
$R^4$ is hydrogen or carbamoyl, and
$R^5$ and $R^6$ are each hydrogen or hydroxy,
with proviso that
(i) $R^2$ is acyloxy, when $R^3$ is hydrogen, and
(ii) $R^5$ is hydrogen, when $R^6$ is hydrogen,

or a pharmaceutically acceptable salt thereof to a human being or an animal.